(19) Europäisches Patentamt
European Patent Office
Office européen des brevets

(11) EP 3 185 843 B2

(12) **NOUVEAU FASCICULE DE BREVET EUROPEEN**
Après la procédure d'opposition

(45) Date de publication et mention de la décision concernant l'opposition:
**02.07.2025 Bulletin 2025/27**

(45) Mention de la délivrance du brevet:
**11.08.2021 Bulletin 2021/32**

(21) Numéro de dépôt: **15771271.2**

(22) Date de dépôt: **26.08.2015**

(51) Classification Internationale des Brevets (IPC):
***A61K 8/25*** *(2006.01)* ***A61Q 1/00*** *(2006.01)*
***A61Q 17/04*** *(2006.01)* ***A61K 8/04*** *(2006.01)*

(52) Classification Coopérative des Brevets (CPC):
**A61Q 17/04; A61K 8/042; A61K 8/25; A61Q 1/00;**
A61K 2800/612; A61K 2800/651

(86) Numéro de dépôt international:
**PCT/IB2015/056471**

(87) Numéro de publication internationale:
**WO 2016/030839 (03.03.2016 Gazette 2016/09)**

(54) **COMPOSITION GEL/GEL COMPRENANT UN FILTRE UV**
GELZUSAMMENSETZUNG UND GEL MIT EINEM UV-FILTER
GEL COMPOSITION AND GEL COMPRISING A UV FILTER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.08.2014 FR 1458031**
**28.08.2014 FR 1458034**
**28.08.2014 FR 1458032**

(43) Date de publication de la demande:
**05.07.2017 Bulletin 2017/27**

(73) Titulaire: **L'OREAL**
**75008 Paris (FR)**

(72) Inventeurs:
- **ROUDOT, Angelina**
  **F-94270 Le Kremelin Bicetre (FR)**
- **FALIP, Anne**
  **F-94140 Alfortville (FR)**
- **CANDAU, Didier**
  **F-91570 Bievres (FR)**
- **VALVERDE, Elodie**
  **26400 Gigors-et-Lozeron (FR)**
- **FAGEON, Laure**
  **94152 Chevilly la Rue (FR)**

(74) Mandataire: **Ipsilon NNY**
**11 rue Saint-Georges**
**75009 Paris (FR)**

(56) Documents cités:
**EP-A1- 0 669 323**
**EP-A1- 1 380 288**
**EP-A2- 0 848 944**
**WO-A1-2010/043588**
**WO-A1-2013/087927**
**WO-A1-2014/128680**
**WO-A2-02/074255**
**DE-A1- 102008 052 521**
**DE-A1- 102009 009 004**
**FR-A1- 2 986 422**
**FR-A1- 2 986 424**
**US-A- 5 788 954**

- **AEROSIL: "Versatile and Effective", DEGUSSA, TECHNICAL INFORMATION,, no. TI 1251, 1 March 2003 (2003-03-01), pages 1 - 21, XP003026229**

EP 3 185 843 B2

## Description

La présente invention vise à proposer pour le domaine de la protection solaire et plus particulièrement pour le domaine des compositions de soin et/ou de maquillage des matières kératiniques, notamment la peau et/ou les lèvres, une nouvelle galénique tout particulièrement intéressante au regard de ses performances techniques notamment en termes de photoprotection et des ressentis sensoriels qu'elle procure à l'utilisateur lors de son application sur celles-ci et en particulier sur la peau.

Par « matières kératiniques », on entend notamment la peau, les lèvres, et également les fibres kératiniques telles que les cheveux, en particulier la peau et/ou les cheveux, et de préférence la peau.

Il est connu que les radiations de longueur d'onde comprise entre 280 nm et 400 nm permettent le bronzage de l'épiderme humain et que les radiations de longueur d'onde comprise entre 280 et 320 nm, connus sous le nom de rayons UV-B nuisent au développement du bronzage naturel. L'exposition est aussi susceptible d'induire une altération des propriétés biomécaniques de l'épiderme qui se traduit par l'apparition de rides conduisant un vieillissement prématuré de la peau.

Il est aussi connu que les rayons UV-A de longueur d'onde comprise entre 320 et 400 nm pénètrent plus profondément dans la peau que les rayons UV-B. Les rayons UV-A provoquent un brunissement immédiat et persistant de la peau. Une exposition quotidienne aux rayons UVA, même de courte durée, dans des conditions normales peut conduire à une dégradation des fibres collagène et de l'élastine qui se traduit par une modification du micro-relief de la peau, l'apparition de rides et une pigmentation irrégulière (tâches brunes, hétérogénéité du teint).

De nombreuses compositions photoprotectrices ont été proposées à ce jour pour remédier aux effets induits par les rayonnements UVA et/ou UVB. Elles contiennent généralement des filtres UV organiques liposolubles et hydrosolubles qui fonctionnent par absorption des UV selon leur propre nature chimique.

Il est connu que la formulation d'émulsions à forts taux de filtres, nécessaires pour atteindre de hauts niveaux d'efficacité filtrante, ne se prête pas à une élaboration rapide et aisée d'une gamme variée de compositions et de textures.

Ainsi, dans un système émulsionné donné, il s'avère souvent compliqué d'intégrer des filtres UV à haute teneur, sans en altérer la stabilité. Il faut alors ré-ajuster la formule et en particulier reformuler le système émulsionné.

Il est par ailleurs connu que les formulations filtrantes présentent des aspects sensoriels inconfortables voire désagréables masquant la fraîcheur et le confort des formules. Le point faible des émulsions filtrantes de haut indice de protection est souvent une sensation importante de gras et de collant, et donc un manque de légèreté des textures obtenues, mais aussi un aspect brillant sur la peau qui peut être rédhibitoire en particulier pour les compositions de maquillage. Il est ainsi difficile de concilier dans une même composition des performances techniques qui s'opposent, telles qu'un haut niveau de protection UV qui implique un fini gras et collant sur la peau et un sensoriel agréable, attribué entre autre à la sensation de fraîcheur, ainsi qu'un visuel lisse homogène.

Il demeure donc un besoin de disposer de compositions de protection solaire UV qui soient stables, efficaces en photo-protection et qui ne présentent pas les inconvénients présentés ci-dessus, en particulier qui procurent un résultat visuel immédiat sur la peau avec un sensoriel agréable notamment une sensation de fraîcheur et de légèreté à l'application.

Dans le domaine du maquillage, notamment pour les fonds de teint, il est en outre recherché des compositions permettant un dépôt homogène conférent un aspect visiblement plus lisse et qui soient performantes en termes de photoprotection.

De manière inattendue et avantageuse, les inventeurs ont montré qu'on pouvait répondre à ce besoin au moyen des compositions photo-protectrices selon la présente invention.

Il est ainsi décrit une composition, en particulier cosmétique de maquillage et/ou de soin des matières kératiniques, comprenant :

- au moins une phase aqueuse gélifiée par au moins un agent gélifiant hydrophile non amylacé,
- au moins une phase huileuse gélifiée par au moins un agent gélifiant lipophile non cellulosique différent des cires hydrocarbonées apolaires de point de fusion supérieur à 75,0 °C, de préférence supérieur à 80,0 °C, et des polyamides siliconés lesdites phases y formant un mélange macroscopiquement homogène ;

ladite composition comprenant en outre au moins un filtre UV.

Ainsi, selon un de ses aspects, la présente invention a pour objet une composition, en particulier cosmétique de maquillage et/ou de soin des matières kératiniques, différente d'une émulsion, comprenant :

- au moins une phase aqueuse gélifiée par au moins un agent gélifiant hydrophile non amylacé,
- au moins une phase huileuse gélifiée par au moins un agent gélifiant lipophile choisi parmi les élastomères d'organopolysiloxane, les polymères semi-cristallins, les esters de dextrine, les polyamides hydrocarbonés, les gélifiants particulaires choisis parmi les cires polaires, les cires apolaires hydrocarbonées de point de fusion inférieur

ou égal à 75,0 °C, les cires siliconées, les argiles modifiées, les silices, ainsi que leurs mélanges ;

lesdites phases y formant un mélange macroscopiquement homogène ;
ladite composition comprenant en outre au moins un filtre UV choisi parmi les filtres organiques hydrosolubles, les filtres organiques liposolubles et les filtres organiques insolubles.

Avantageusement, le ou les filtres UV sont présents en tout ou en partie, et de préférence uniquement, dans la phase aqueuse gélifiée ou sont présents en tout ou en partie, et de préférence uniquement, dans la phase huileuse gélifiée.
Contre toute attente, et comme il ressort des exemples figurant ci-après, les inventeurs ont constaté que la formulation d'un filtre UV dans une architecture gel-gel telle que définie ci-dessus permet l'obtention d'une composition permettant d'apporter de la fraîcheur et de la légèreté même lorsque ladite composition contient un taux important de filtres UV. En outre l'architecture gel-gel telle que définie ci-dessus permet de maintenir les propriétés de photo-protection et conduit à des compositions stables, notamment sans apparition de déphasage.
L'application de la composition sur la peau conduit à un dépôt lisse et homogène.
Des compositions, dites gel-gel, sont déjà proposées dans le domaine cosmétique. Ce type de formulations associe une phase aqueuse gélifiée à une phase huileuse gélifiée. Ainsi, des formulations gel/gel sont décrites dans Almeida et al., Pharmaceutical Development and Technology, 2008, 13:487, tableaux 1 et 2, page 488 ; WO 99/65455 ; PI 0405758-9 ; WO 99/62497 ; JP 2005-112834 et WO 2008/081175. Toutefois, à la connaissance des inventeurs, ce type de composition ne permet pas à l'heure actuelle de garantir toutes les propriétés essentielles attendues dans le domaine cosmétique, telles qu'une texture agréable lors de la préhension du produit, un dépôt au collant réduit, confortable et homogène notamment pour le maquillage, ou encore une stabilité de la formulation.
On connaît également dans la demande WO 2013/093869 des compositions comprenant au moins :

- une phase aqueuse gélifiée contenant au moins 3 % en poids par rapport à son poids total de particules d'au moins un carboxyalkyle en (C1-C4) d'amidon et dont la taille particulaire varie de 25 à 300 μm, et
- une phase huileuse gélifiée à l'aide d'au moins un agent texturant,

avec l'une desdites phases constituant la phase continue de ladite composition dans laquelle est dispersée uniformément l'autre phase.
Comme précisé ci-dessus, les inventeurs ont constaté que la mise en œuvre de filtres UV dans une composition multiphasiques selon l'invention permet de garantir la tenue des propriétés photoprotectrices et de la stabilité.
Ainsi, une composition selon l'invention manifeste une très bonne stabilité tant en termes de stabilité visuelle (pas de déphasage) que de propriétés photoprotectrices, tout en procurant un ressenti fraîcheur et légèreté à l'utilisateur à l'application.
Enfin, la composition s'avère facile à appliquer en surface de la matière kératinique visée et conduit à un dépôt lisse et homogène conformes aux exigences dans le domaine du maquillage.
Il est également décrit un procédé de préparation d'une composition, en particulier cosmétique de maquillage et/ou de soin des matières kératiniques, comprenant au moins une étape de mélange :

- d'une phase aqueuse gélifiée par au moins un agent gélifiant hydrophile non amylacé ; et
- d'au moins une phase huileuse gélifiée par au moins un agent gélifiant lipophile non cellulosique différent des cires hydrocarbonées apolaires de point de fusion supérieur à 75,0 °C, de préférence supérieur à 80,0 °C, et des polyamides siliconés ;

lesdites phases y formant un mélange macroscopiquement homogène ;
ladite composition comprenant en outre au moins un filtre UV.

Ainsi, l'invention a encore pour objet, selon un autre de ses aspects, un procédé de préparation d'une composition, en particulier cosmétique de maquillage et/ou de soin des matières kératiniques, différente d'une émulsion, comprenant au moins une étape de mélange :

- d'une phase aqueuse gélifiée par au moins un agent gélifiant hydrophile non amylacé ; et
- d'au moins une phase huileuse gélifiée par au moins un agent gélifiant lipophile choisi parmi les élastomères d'organopolysiloxane, les polymères semi-cristallins, les esters de dextrine, les polyamides hydrocarbonés, les gélifiants particulaires choisis parmi les cires polaires, les cires apolaires hydrocarbonées de point de fusion inférieur ou égal à 75,0 °C, les cires siliconées, les argiles modifiées, les silices, ainsi que leurs mélanges ;

lesdites phases y formant un mélange macroscopiquement homogène ;
ladite composition comprenant en outre au moins un filtre UV choisi parmi les filtres organiques hydrosolubles, les filtres organiques liposolubles et les filtres organiques insolubles.

Selon une variante de réalisation, ce procédé peut avantageusement comprendre une étape de mélange d'au moins deux, mieux au moins trois phases gélifiées voire plus.
Pour des raisons évidentes, le nombre de phases aqueuses gélifiées et de phases huileuses gélifiées à considérer pour former une composition selon l'invention peut varier pour chacun des deux types de phase au delà de deux.
Avantageusement, le mélange des phases peut être réalisé à température ambiante.
Toutefois, le procédé de l'invention peut comporter, si nécessaire, une étape de chauffage du mélange.
Selon une variante de réalisation, la formule finale peut être fabriquée sans suivre un ordre particulier d'introduction des différents constituants et dans certains cas une fabrication « tout en un » peut être réalisée.
Selon un mode de réalisation particulier, les phases gélifiées représentatives d'un même type d'architecture sont gélifiées par un gélifiant différent.
Des formules multiphasiques peuvent ainsi être développées.
L'invention a encore pour objet, selon un autre de ses aspects, un procédé cosmétique de maquillage et/ou de soin de matières kératiniques, en particulier de la peau du corps et/ou du visage, et/ou des fibres kératiniques, notamment les cheveux, comprenant au moins une étape consistant à appliquer sur ladite matière kératinique une composition selon l'invention
Il est également décrit un procédé cosmétique de maquillage et/ou de soin de matières kératiniques, en particulier de la peau du corps et/ou du visage, et/ou des fibres kératiniques, notamment les cheveux, comprenant au moins l'application sur lesdites matières kératiniques d'une composition macroscopiquement homogène obtenue par mélange extemporané, avant application ou au moment de l'application sur ladite matière kératinique, d'au moins une phase aqueuse gélifiée par au moins un agent gélifiant hydrophile non amylacé, et au moins une phase huileuse gélifiée par au moins un agent gélifiant lipophile non cellulosique différent des cires hydrocarbonées apolaires de point de fusion supérieur à 75,0 °C, de préférence supérieur à 80,0 °C, et des polyamides siliconés , et ladite composition comprenant en outre au moins au moins un filtre UV.
Ainsi, l'invention a encore pour objet, selon un autre de ses aspects, un procédé cosmétique de maquillage et/ou de soin de matières kératiniques, en particulier de la peau du corps et/ou du visage, et/ou des fibres kératiniques, notamment les cheveux, comprenant au moins l'application sur lesdites matières kératiniques d'une composition macroscopiquement homogène obtenue par mélange extemporané, avant application ou au moment de l'application sur ladite matière kératinique, d'au moins une phase aqueuse gélifiée par au moins un agent gélifiant hydrophile non amylacé, et au moins une phase huileuse gélifiée par au moins un agent gélifiant lipophile choisi parmi les élastomères d'organopolysiloxane, les polymères semi-cristallins, les esters de dextrine, les polyamides hydrocarbonés, les gélifiants particulaires choisis parmi les cires polaires, les cires apolaires hydrocarbonées de point de fusion inférieur ou égal à 75,0 °C, les cires siliconées, les argiles modifiées, les silices, ainsi que leurs mélanges, et ladite composition comprenant en outre au moins au moins un filtre UV choisi parmi les filtres organiques hydrosolubles, les filtres organiques liposolubles et les filtres organiques insolubles.
L'invention a encore pour objet, selon un autre de ses aspects, un procédé cosmétique pour limiter l'assombrissement de la peau, et/ou améliorer la couleur et/ou l'homogénéité du teint, comprenant l'application sur la surface de la matière kératinique d'une composition selon l'invention.
Elle concerne également un procédé cosmétique pour prévenir et/ou traiter les signes du vieillissement d'une matière kératinique comprenant l'application sur la surface de la matière kératinique d'une composition selon l'invention.

## Définitions

Par *« filtre UV »,* au sens de la présente invention, on entend tout composé organique (comprenant au moins des atomes de carbone et d'hydrogène) ou tout composé inorganique (ne comprenant pas d'atomes de carbone) capable de filtrer par absorption et/ou diffusion et/ou réflexion les radiations UV allant de 280nm à 400nm et qui ne présente pas une couvrance suffisante pour produire une couleur sur la surface d'une matière kératinique par application d'une composition le contenant.
Par *« cire »*, au sens de la présente invention, on entend d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.
Au sens de l'invention, la température de fusion ou le point de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu

sous la dénomination « *MDSC 2920* » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Par « *cire hydrocarbonée* », on entend une cire formée essentiellement, voire constituée, d'atomes de carbone et d'hydrogène, et éventuellement d'atomes d'oxygène, d'azote, et ne contenant pas d'atome de silicium ou de fluor.

Par « *cire apolaire* », au sens de la présente invention, on entend une cire dont le paramètre de solubilité à 25 °C tel que défini ci-après, $\delta_a$ est égal à 0 $(J/cm^3)^{½}$.

La définition et le calcul des paramètres de solubilité dans l'espace de solubilité tridimensionnel de Hansen sont décrits dans l'article de C. M. Hansen : « The three dimensionnal solubility parameters » J. Paint Technol. 39, 105 (1967).

Selon cet espace de Hansen :

- $\delta_D$ caractérise les forces de dispersion de London issues de la formation de dipôles induits lors des chocs moléculaires ;
- $\delta_p$ caractérise les forces d'interactions de Debye entre dipôles permanents ainsi que les forces d'interactions de Keesom entre dipôles induits et dipôles permanents ;
- $\delta_h$ caractérise les forces d'interactions spécifiques (type liaisons hydrogène, acide/base, donneur/accepteur, etc.) ;
- $\delta_a$ est déterminé par l'équation : $\delta_a = (\delta_p^2 + 8_h^2)^{½}$.

Les paramètres $\delta_p$, $\delta_h$, $\delta_D$ et $\delta_a$ sont exprimés en $(J/cm^3)^{½}$.

Les cires apolaires sont en particulier les cires hydrocarbonées constituées uniquement d'atomes de carbone et d'hydrogène et exempte d'hétéroatomes tel que N, O, Si et P.

Par « *polyamide* », on entend au sens de l'invention un composé ayant au moins 2 motifs de répétition amide, de préférence au moins 3 motifs de répétition amide et mieux encore 10 motifs de répétition amide.

Par « *polyamide siliconé* », on entend un polyamide comprenant une chaine polyorganosiloxanique formée essentiellement, voire constituée, d'atomes de carbone et d'hydrogène et d'atomes de silicium et notamment des groupes -SiO.

## Composition

Tout d'abord, il est important de noter qu'une composition selon l'invention est différente d'une émulsion.

Une émulsion est généralement constituée d'une phase liquide huileuse et d'une phase liquide aqueuse. Il s'agit d'une dispersion de gouttelettes de l'une des deux phases liquides dans l'autre. La taille des gouttelettes formant la phase dispersée de l'émulsion est typiquement de l'ordre du micromètre (0,1 à 100 µm). De plus, une émulsion requiert la présence d'un tensio-actif ou d'un émulsifiant pour assurer sa stabilité.

A l'inverse, une composition selon l'invention consiste en un mélange macroscopiquement homogène de deux phases gélifiées non miscibles. Ces deux phases possèdent toutes deux une texture de type gel. Cette texture se traduit notamment visuellement par un aspect consistant et/ou crémeux.

On entend par « *mélange macroscopiquement homogène* », un mélange dans lequel chacune des phases gélifiées ne peut être individualisé à l'œil nu. Plus précisément, dans une composition selon l'invention, la phase aqueuse gélifiée et la phase huileuse gélifiée interpénètrent et forment ainsi un produit stable et consistant. Cette consistance est atteinte par mélange des macro-domaines interpénétrés. Ces macro-domaines interpénétrés ne sont pas des objets mesurables. Ainsi, au microscope, la composition selon l'invention est très différente d'une émulsion. Une composition selon l'invention ne peut non plus être caractérisée comme ayant un « *sens* », i.e. un sens H/E ou E/H, en d'autres termes une phase continue et une phase dispersée ne peuvent être définies.

Ainsi, une composition selon l'invention possède une consistance de type gel. La stabilité de la composition peut être assurée sans la présence obligatoire d'un tensio-actif. Par conséquent, une composition cosmétique selon l'invention ne requiert pas nécessairement de tensio-actif ni d'émulsifiant siliconé pour assurer sa stabilité.

Une composition selon l'invention se distingue d'une émulsion selon au moins un des tests suivants: test réalisé au moyen d'une matière colorante, « test de la goutte » et test de dilution.

Test réalisé au moyen d'une matière colorante

Il est connu de l'état de l'art d'observer la nature intrinsèque d'un mélange de gels aqueux et huileux dans une composition de type gel/gel, par exemple, en introduisant une matière colorante soit dans la phase gélifiée aqueuse soit dans la phase gélifiée lipophile, avant la formation de la composition de type gel/gel. Lors de l'inspection visuelle, dans une composition de type gel/gel, la matière colorante semble uniformément dispersée, même si le colorant est présent uniquement dans la phase aqueuse gélifiée ou dans la phase huileuse gélifiée. En effet, si deux colorants différents de couleurs distinctes sont introduits respectivement dans la phase huileuse et dans la phase aqueuse, avant la formation de la composition de type gel/gel, les deux couleurs peuvent être observées comme uniformément dispersées dans toute la composition de type gel/gel. Cela est différent d'une émulsion dans laquelle, si un colorant, soluble dans l'eau ou soluble dans l'huile, est introduit respectivement dans les phases aqueuse et huileuse, avant de former l'émulsion, on observera uniquement la couleur du colorant présent dans la phase externe (Remington: The Science and Practice of Pharmacy, 19th Edition (1995), Chapitre 21, page 282).

Test de la goutte

Il est également connu de distinguer une composition de type gel/gel d'une émulsion en effectuant un « test de la goutte ». Ce test consiste à démontrer la nature bi-continue d'une composition de type gel/gel. En effet, comme mentionné précédemment, la consistance d'une composition est obtenue grâce à l'interpénétration des domaines gélifiés aqueux et huileux. Par conséquent, la nature bi-continue d'une composition de type gel/gel peut être mise en évidence par un simple test avec respectivement des solvants hydrophile et hydrophobe. Ce test consiste à déposer, d'une part, une goutte d'un solvant hydrophile sur un premier échantillon de la composition testée, et d'autre part, une goutte d'un solvant hydrophobe sur un second échantillon de la même composition testée, et d'analyser le comportement des deux gouttes de solvants. Dans le cas d'une émulsion H/E, la goutte de solvant hydrophile diffuse dans l'échantillon et la goutte de solvant hydrophobe reste à la surface de l'échantillon. Dans le cas d'une émulsion E/H, la goutte de solvant hydrophile reste à la surface de l'échantillon et la goutte de solvant hydrophobe diffuse dans tout l'échantillon. Finalement, dans le cas d'une composition de type gel/gel (système bi-continu), les gouttes hydrophile et hydrophobe diffusent dans tout l'échantillon.

Test de dilution

Dans le cas de la présente invention, le test qui sera privilégié pour distinguer une composition de type gel/gel d'une émulsion est un test de dilution. En effet, dans une composition de type gel/gel, les domaines gélifiés aqueux et huileux interpénètrent et forment une composition consistante et stable, dans laquelle le comportement dans l'eau et dans l'huile est différent du comportement d'une émulsion. Par conséquent, le comportement lors d'une dilution d'une composition de type gel/gel (système bi-continu) peut être comparé à celui d'une émulsion et conduiront bien entendu à des résultats différents.

Plus spécifiquement, le test de dilution consiste à mettre 40 g de produit et 160 g de solvant de dilution (eau ou huile) dans un bêcher. La dilution est effectuée sous agitation contrôlée pour éviter tout phénomène d'émulsification. En particulier, ceci est effectué en utilisant un mélangeur à mouvement planétaire : Speed Mixer TM DAC400FVZ. La vitesse du mélangeur est réglée à 1500 rpm pendant 4 minutes. Enfin, l'observation de l'échantillon résultant est effectuée à l'aide d'un microscope optique à un grossissement de x 100 (x10x10). Il peut être noté que les huiles comme Parleam® et Xiameter PMX-200 Silicone Fluid 5CS® commercialisés par Dow Corning conviennent comme solvant de dilution au même titre qu'une des huiles contenue dans la composition.

Dans le cas d'une composition de type gel/gel (système bi-continu), lorsqu'elle est diluée dans l'huile ou dans l'eau, un aspect hétérogène est toujours observé. Quand une composition de type gel/gel (système bi-continu) est dilué dans de l'eau, on observe des morceaux de gel huileux en suspension et quand une composition de type gel/gel (système bi-continu) est dilué dans de l'huile, on observe des morceaux de gel aqueux en suspension.

Au contraire, lors de la dilution, les émulsions présentent un comportement différent. Une émulsion H/E, lorsqu'elle est diluée dans un solvant aqueux, se réduit progressivement sans présenter d'aspect hétérogène et grumeleux. Cette même émulsion H/E, lors de la dilution avec l'huile, présente une apparence hétérogène (morceaux d'émulsion H/E suspendus dans l'huile). Une émulsion E/H, lorsqu'elle est diluée avec un solvant aqueux, présente une apparence hétérogène (morceaux d'émulsion E/H suspendus dans l'eau). Cette même émulsion E/H, lorsqu'elle est diluée dans l'huile se réduit progressivement sans présenter d'aspect hétérogène et grumeleux.

Selon la présente invention, la phase gélifiée aqueuse et la phase gélifiée huileuse formant une composition selon l'invention y sont présentes dans un rapport pondéral variant de 95/5 à 5/95. Plus préférentiellement, la phase aqueuse et la phase huileuse sont présentes dans un rapport pondéral variant de 20/80 à 80/20 et encore plus préférentiellement variant de 30/70 à 70/30.

Le rapport entre les deux phases gélifiées est ajusté selon les propriétés cosmétiques recherchées.

Ainsi, dans le cas d'une composition de soin ou de maquillage, en particulier du visage, il sera avantageux de favoriser un rapport pondéral phase gélifiée aqueuse/phase gélifiée huileuse supérieur ou égal à 1, notamment variant de 50 / 50 à 90/10, de préférence variant de 60/40 à 80/20,.Avantageusement, une composition selon l'invention peut se présenter donc sous l'aspect d'un gel crémeux possédant une contrainte minimale au dessous de laquelle elle ne s'écoule pas sauf à avoir été soumise à une sollicitation mécanique externe.

Comme il ressort de ce qui suit une composition selon l'invention peut posséder une contrainte seuil minimale de 1,5 Pa et en particulier supérieure à 10 Pa. La composition selon l'invention peut posséder une contrainte seuil inférieure à 10 000 Pa de préférence inférieure à 5 000 Pa.

Elle peut posséder également avantageusement un module de rigidité G* au moins égal à 400 Pa, et de préférence supérieur à 1000 Pa. La composition selon l'invention peut posséder un module de rigidité G* de préférence inférieur à 50 000Pa de manière préférée inférieur à 5 000Pa .

La viscosité de la phase hydrophile sur la viscosité de la phase lipophile (mesurée à 25°C et 100 $s^{-1}$) varie de préférence de 0,2 à 3, de préférence 0,5 à 1,5.

Selon une variante de réalisation avantageuse, les phases gélifiées considérées pour former une composition selon l'invention peuvent posséder respectivement une contrainte seuil supérieure à 1,5 Pa, et de préférence supérieure à 10 Pa.

Les phases gélifiées considérées pour former une composition selon l'invention peuvent posséder une contrainte seuil inférieure à 10 000 Pa de préférence inférieure à 5 000 Pa.

La caractérisation des contraintes seuil est effectuée par des mesures de rhéologie en oscillation. Une méthodologie est proposée dans le chapitre exemplification du présent texte.

D'une manière générale, les mesures correspondantes sont réalisées à 25 °C à l'aide d'un rhéomètre à contrainte imposée, RS600 HAAKE, équipé d'un corps de mesure plan-plan (diamètre 60 mm) muni d'un dispositif anti-évaporation (cloche). Pour chaque mesure, l'échantillon est mis en place délicatement et les mesures débutent 5 minutes après la mise en place de l'échantillon dans l'entrefer (2 mm). La composition testée est ensuite soumise à une rampe en contrainte de $10^{-2}$ à $10^{3}$ Pa à une fréquence fixée à 1 Hz.

Une composition selon l'invention peut posséder également une certaine élasticité. Cette élasticité est caractérisable par un module de rigidité G* qui sous ce seuil de contrainte minimale peut être au moins égal à 400 Pa, et de préférence supérieur à 1000 Pa. La valeur G* d'une composition peut être obtenue en soumettant la composition considérée à une rampe en contrainte de $10^{-2}$ à $10^{3}$ Pa à une fréquence fixée à 1 Hz.

## GELIFIANT HYDROPHILE

On entend par « *gélifiant hydrophile* » au sens de la présente invention, un composé apte à gélifier la phase aqueuse des compositions selon l'invention.

On entend par « *gélifiant hydrophile non amylacé* » au sens de la présente invention, un gélifiant hydrophile qui est différent d'un amidon.

Le gélifiant hydrophile est donc présent dans la phase aqueuse de la composition.

Le gélifiant peut être hydrosoluble ou hydrodispersible.

Les gélifiants hydrophiles sont de préférence non émulsionnants, de manière préférée ils ne contiennent pas de chaine(s) grasse(s) tels que des chaines alkyles supérieures à $C_7$ et notamment allant de $C_7$ à $C_{24}$.

Comme précisé ci-dessus, la phase aqueuse d'une composition selon l'invention est gélifiée par au moins un agent gélifiant hydrophile.

L'agent gélifiant hydrophile non amylacé peut être choisi parmi les gélifiants polymériques synthétiques, les silicates mixtes et les silices pyrogénées, les gélifiants polymériques naturels ou d'origine naturelle non amylacés notamment les polysaccharides non amylacés, et leurs mélanges.

De préférence, l'agent gélifiant hydrophile est choisi parmi les gélifiants polymériques synthétiques.

### I. Gélifiants polymériques naturels ou d'origine naturelle non amylacés

Les gélifiants hydrophiles polymériques convenant à l'invention peuvent être naturels ou d'origine naturelle.

Au sens de l'invention, l'expression « *d'origine naturelle* » entend désigner les gélifiants polymériques obtenus par modification des gélifiants polymériques naturels.

Ces gélifiants peuvent être particulaires ou non particulaires.

Plus précisément, ces gélifiants relèvent de la catégorie des polysaccharides.

#### Polysaccharides non amylacés

D'une manière générale, les polysaccharides non amylacés peuvent être choisis parmi les polysaccharides

élaborés par des microorganismes ; les polysaccharides isolés des algues, les polysaccharides des végétaux supérieurs, tels que les polysaccharides homogènes, en particulier les celluloses et ses dérivés ou les fructosanes, les polysaccharides hétérogènes tels que les gommes arabique, les galactomannanes, les glucomannanes, les pectines, et leurs dérivés ; et leurs mélanges.

En particulier, les polysaccharides peuvent être choisis parmi les fructanes, les gellanes, les glucanes, le glycogène, le pullulan, les dextranes, les celluloses et leurs dérivés, en particulier les méthylcelluloses, hydroxyalkylcelluloses, éthylhydroxyéthylcelluloses, et les carboxyméthylcelluloses, les mannanes, les xylanes, les lignines, les arabanes, les galactanes, les galacturonanes, les composés à base d'alginate, la chitine, les chitosanes, les glucoronoxylanes, les arabinoxylanes, les xyloglucanes, les glucomannanes, les acides pectiques et les pectines, les arabinogalactanes, les carraghénanes, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ioniques, en particulier l'hydroxypropyl guar, et ioniques, les gommes de biopolysaccharides d'origine microbienne, en particulier les gommes de scléroglucane ou de xanthane, les mucopolysaccharides, et en particulier les chondroïtines sulfate et leurs mélanges.

Ces polysaccharides peuvent être modifiés chimiquement, notamment par des groupements urée, uréthane, ou par réaction d'hydrolyse, d'oxydation, d'estérification, d'éthérification, de sulfatation, de phosphatation, d'amination, d'amidation, d'alkylation en $C_1$-$C_6$, ou par plusieurs de ces modifications.

Les dérivés obtenus peuvent être anioniques, cationiques, amphotères ou non-ioniques.

De manière avantageuse, les polysaccharides peuvent être choisis parmi les carraghénanes, en particulier la kappa-carraghenane, la gomme de gellane, l'agar-agar, la gomme de xanthane, les composés à base d'alginate, en particulier l'alginate de sodium, la gomme de scéroglucane, la gomme de guar, l'inuline, le pullulan, et leurs mélanges.

D'une manière générale, les composés de ce type, utilisables dans la présente invention, sont choisis parmi ceux qui sont notamment décrits dans « Encyclopedia of Chemical Technology, Kirk-Othmer, Third Edition, 1982, volume 3, pp. 896-900, et volume 15, pp 439-458 », dans « Polymers in Nature, par E. A. Mc GREGOR et C. T. GREENWOOD, Editions John Wiley & Sons, Chapter 6, pp 240-328, 1980 », dans l'ouvrage de Robert L. DAVIDSON intitulé « Handbook of Water soluble gums and resins » édité chez Mc Graw Hill Book Company (1980) et dans l'industrial Gums « Polysaccharides and their Derivatives, Edité par Roy L. WHISTLER, Second Edition, Edition Academic Press Inc. ».

Un tel gélifiant peut être mis en œuvre à raison de 0,1 % à 8 % en poids en matière sèche par rapport au poids total de la phase aqueuse, notamment de 0,1 % à 6 % en poids, de préférence entre 0,5 % et 2,5 % en poidspar rapport au poids total de la phase aqueuse.

Plus précisément, ces polysaccharides convenant à l'invention peuvent être distingués selon qu'ils sont issus des microorganismes, des algues ou des végétaux supérieurs, et sont détaillés ci-après.

Polysaccharides élaborés par des microorganismes

*Xanthane*

Le xanthane est un hétéropolysaccharide produit à l'échelle industrielle par la fermentation aérobie de la bactérie Xanthomonas campestris. Sa structure est constituée d'une chaîne principale de β-D-glucoses liés en β(1,4), semblable à la cellulose. Une molécule de glucose sur deux porte une chaîne latérale trisaccharidique composée d'un α-D-mannose, d'un acide β-D-glucuronique et d'un β-D-mannose terminal. Le résidu interne de mannose est généralement acétylé sur le carbone 6. Environ 30 % des résidus mannose terminal portent un groupement pyruvate lié sous forme chélatée entre les carbones 4 et 6. Les acides glucuroniques et les acides pyruviques chargés sont ionisables, et donc responsables de la nature anionique du xanthane (charge négative jusqu'à pH égal à 1). Le contenu des résidus pyruvate et acétate varie selon la souche de bactérie, le procédé de fermentation, les conditions après fermentation et les étapes de purification. Ces groupements peuvent être neutralisés dans les produits commerciaux avec des ions $Na^+$, $K^+$ ou $Ca^{2+}$ (Société SATIA, 1986). La forme neutralisée peut être convertie en forme acide par échange d'ions ou par dialyse d'une solution acide.

Les gommes de xanthane ont un poids moléculaire compris entre 1 000 000 à 50 000 000 et une viscosité comprise entre 0,6 à 1,65 Pa.s pour une composition aqueuse contenant 1 % de gomme xanthane (mesurée à 25 °C au viscosimètre Brookfield, type LVT à 60 tours par minute).

Les gommes de xanthane sont représentées par exemple par les produits vendus sous les dénominations Rhodicare par la société RHODIA CHIMIE, sous la dénomination SATIAXANE™ par la société Cargill Texturizing Solutions (pour l'industrie alimentaire, cosmétique et pharmaceutique), sous la dénomination NOVAXAN™ par la société ADM, et sous les dénominations Kelzan® et Keltrol® par la société CP-Kelco.

*Pullulan*

Le Pullulan est un polysaccharide constitué d'unités maltotriose, connues sous le nom d'α(1,4)-α(1,6)-glucane.

Trois unités de glucose dans le maltotriose sont connectées par une liaison glycosidiques en $\alpha(1,4)$, tandis que les unités maltotriose consécutives sont connectées l'une à l'autre par une liaison glycosidiques en $\alpha(1,6)$.

Le pullulan est par exemple produit sous la référence Pullulan PF 20 par le groupe Hayashibara au Japon.

*Dextrane et sulfate de dextrane*

Le dextrane est un polysaccharide neutre sans groupe chargé, biologiquement inerte, préparé par fermentation du sucre de betterave contenant uniquement des groupements hydroxyles.

Il est possible d'obtenir à partir du dextrane natif par hydrolyse et purification, des fractions de dextrane de poids moléculaires différents. Le dextrane peut en particulier se présenter sous la forme de sulfate de dextrane.

Le dextrane est représenté par exemple par les produits vendus, sous la dénomination Dextran ou Dextran T par la société Pharmacosmos, sous la dénomination Dextran 40 powder ou Dextran 70 powder par la société Meito Sangyo Co. Le sulfate de dextrane est commercialisé par la société PK Chemical A/S sous la dénomination Dextran sulphate.

*Succinoglycane*

Le succinoglycane est un polymère extracellulaire produit par fermentation bactérienne, de haut poids moléculaire et constitué d'unités répétées d'octasaccharides (répétition de 8 sucres). Les succinoglycanes sont par exemple commercialisés sous la dénomination Rheozan, par la société Rhodia.

*Scléroglucane*

Le scléroglucane est un homopolysaccharide ramifié non ionique, constitué de motifs β-D glucane. Les molécules sont constituées d'une chaine linéaire principale formée de motifs D-glucose liées par des liaisons $\beta(1,3)$ et dont un sur trois est lié à un motif D-glucose latéral par une liaison $\beta(1,6)$.

Une description plus complète des scléroglucanes et de leur préparation peut être trouvée dans le document US 3,301,848.

Le scléroglucane est par exemple vendu sous la dénomination AMIGEL par la Société ALBAN MULLER, ou sous la dénomination ACTIGUM™ CS par la société Cargill.

*Gomme de gellane*

La gomme de gellane est un hétéropolyoside linéaire anionique basé sur des unités d'oligoside composé de 4 oses (tétra-oside). Le D-glucose, le L-rhamnose et l'acide D-glucuronique en proportions 2:1:1 sont présents dans la gomme de gellane sous forme d'éléments monomères.

Elle est par exemple vendue sous la dénomination KELCOGEL CG LA par la société CP KELCO.

Polysaccharides isolés des algues

*Galactannes*

Le polysaccharide selon l'invention peut être un galactanne notamment choisi parmi l'agar ou les carraghénanes.

Les carraghénanes sont des polysaccharides anioniques constituant les parois cellulaires de diverses algues rouges (Rhodophycées) appartenant aux familles de Gigartinacae, Hypneaceae, Furcellariaceae et Polyideaceae. Ils sont généralement obtenus par extraction aqueuse à chaud à partir de souches naturelles desdites algues. Ces polymères linéaires, formés par des motifs disaccharides, sont composés par deux unités D-galactopyranoses liées alternativement par des liaisons $\alpha(1,3)$ et $\beta(1,4)$. Ce sont des polysaccharides très sulfatés (20-50 %) et les résidus α-D-galactopyranosyles peuvent être sous forme 3,6-anhydro. Selon le nombre et la position de groupements ester-sulfate sur le disaccharide de répétition de la molécule, on distingue plusieurs types de carraghénanes à savoir : les kappa-carraghénanes qui possèdent un groupement ester-sulfate, les iota-carraghénanes qui possèdent deux groupements ester-sulfate et les lambda-carraghénanes qui possèdent trois groupements ester-sulfate.

Les carraghénanes se composent essentiellement de sels de potassium, de sodium, de magnésium, de triéthanolamine et/ou de calcium et d'esters sulfates de polysaccharides.

Les carraghénanes sont notamment commercialisés par la société Seppic sous le nom de Solagum®, par la société Gelymar sous la dénomination de Carragel®, Carralact®, et Carrasol®, par la société Cargill, sous les dénominations SATIAGEL™ et SATIAGUM™, et par la société CP-Kelco sous la dénomination GENULACTA®, GENUGEL® et GENUVISCO®.

Les galactannes de type Agar sont des polysaccharides du galactose contenu dans la paroi cellulaire de certaines de ces espèces d'algues rouges (rhodophycées). Ils sont formés d'un groupe de polymère dont le squelette de base est une chaine $\beta$(1,3) D-galactopyranose et $\alpha$(1,4) L 3-6 anhydrogalactose, ces unités se répétant régulièrement et alternativement. Les différences à l'intérieur de la famille des agars sont dues à la présence ou non de groupes solvatés méthylés ou carboxyethylés. Ces structures hybrides sont en général présentes en pourcentage variable, suivant les espèces d'algues et la saison de récolte.

L'agar-agar est un mélange de polysaccharides (agarose et agaropectine) de masse moléculaire élevée, comprise entre 40 000 et 300 000 $g.mol^{-1}$. Il est obtenu en fabricant des jus d'extraction d'algues, généralement par autoclavage, et en traitant ces jus qui comprennent environ 2 % d'agar-agar, afin d'extraire ce dernier.

L'agar est par exemple produit par le groupe B&V Agar Producers, sous la dénomination Gold Agar, Agarite et Grand Agar par la société Hispanagar, et sous les dénominations Agar-Agar, QSA (Quick Soluble Agar), et Puragar par la société Setexam.

*Furcellarane*

Le furcellarane est obtenu commercialement à partir d'algues rouges Furcellaria fasztigiata. Le furcellarane est par exemple produit par la société Est-Agar.

*Composé à base d'alginate*

Par « *composé à base d'alginate* », on entend au sens de l'invention, l'acide alginique, les dérivés d'acide alginique et les sels d'acide alginique (alginates) ou desdits dérivés.

De préférence, le composé à base d'alginate est hydrosoluble.

L'acide alginique, substance naturelle issue des algues brunes ou de certaines bactéries, est un acide polyuronique composé de 2 acides uroniques liés par des liaisons (1,4)glycosidiques : l'acide $\beta$-D-manuronique (M) et l'acide $\alpha$-L-glucuronique (G).

L'acide alginique est apte à former des sels hydrosolubles (alginates) avec des métaux alcalins tels que le sodium, le potassium, le lithium, les cations d'amine inférieure et d'ammonium substitués tels que la méthylamine, l'éthanolamine, la diéthanolamine, la triéthanolamine. Ces alginates sont hydrosolubles en milieu aqueux à pH égal à 4 mais se dissocient en acide alginique à un pH inférieur à 4.

Ce(s) composé(s) à base d'alginate est(sont) apte(s) à réticuler en présence d'au moins un agent de réticulation, par formation de liaisons ioniques entre le(s)dit(s) composé(s) à base d'alginate et le(s)dit(s) agent(s) de réticulation. La formation de multiples réticulations entre plusieurs molécules du(es)dit(s) composé(s) à base d'alginate entraîne la formation d'un gel insoluble dans l'eau.

On utilise de préférence des composés à base d'alginate présentant une masse moléculaire moyenne en poids allant de 10 000 à 1 000 000, de préférence de 15 000 à 500 000, et mieux de 20 000 à 250 000.

Selon un mode de réalisation préféré, le composé à base d'alginate est l'acide alginique et/ou un de ses sels.

De manière avantageuse, le composé à base d'alginate est un sel d'alginate, et de préférence l'alginate de sodium.

Le composé à base d'alginate peut être modifié chimiquement, notamment par des groupements urée, uréthane, ou par réaction d'hydrolyse, d'oxydation, d'estérification, d'éthérification, de sulfatation, de phosphatation, d'amination, d'amidation, d'alkylation, ou par plusieurs de ces modifications.

Les dérivés obtenus peuvent être anioniques, cationiques, amphotères ou non-ioniques.

Les composés à base d'alginate convenant à l'invention peuvent être représentés, par exemple, par les produits vendus sous les dénominations KELCOSOL, SATIALGINE™, CECALGUM™ ou ALGOGEL™ par la société Cargill products, sous la dénomination Protanal™ par la société FMC Biopolymer, sous la dénomination GRINDSTED® Alginate par la société Danisco, sous la dénomination KIMICA ALGIN par la société KIMICA, et sous les dénominations Manucol® et Manugel® par la société ISP.

Polysaccharides des végétaux supérieurs

Cette catégorie de polysaccharides peut être divisée en les polysaccharides homogènes (une seule espèce d'osés) et les hétérogènes composés de plusieurs types d'osés.

a) Polysaccharides homogènes et leurs dérivés

Le polysaccharide selon l'invention peut être choisi parmi les celluloses et dérivés ou les fructosanes.

*Cellulose et dérivés*

Le polysaccharide selon l'invention peut également être une cellulose ou l'un de ses dérivés notamment éthers ou esters de cellulose (ex : méthylcellulose, carboxyméthylcellulo se, hydroxyméthylcellulo se, hydroxyéthylcellulo se, hydroxypropylcellulose, hydroxyméthylpropylcellulose, acétate de cellulose, nitrate de cellulose, nitrocellulose).

Par composé cellulosique, on entend selon l'invention tout composé polysaccharidique possédant dans sa structure des enchaînements linéaires de résidus anhydroglucopyranose (AGU) unis par des liaisons glycosidiques β (1,4). Le motif de répétition est le dimère cellobiose. Les AGU se trouvent en conformation chaise et possèdent 3 fonctions hydroxyles : 2 alcools secondaires (en position 2 et 3) et un alcool primaire (en position 6). Les polymères ainsi formés s'associent entre eux par des liaisons intermoléculaires de type liaisons hydrogène, conférant ainsi une structure fibrillaire à la cellulose (environ 1500 molécules par fibre).

Le degré de polymérisation diffère énormément selon l'origine de la cellulose ; sa valeur peut varier de quelques centaines à quelques dizaines de milliers.

La cellulose présente la structure chimique suivante :

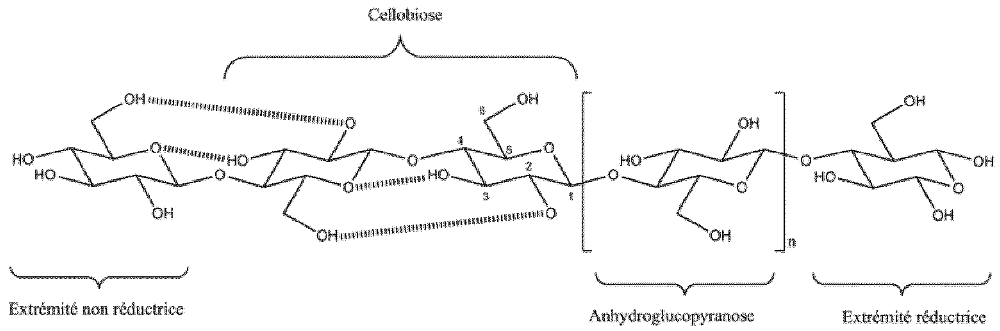

Les groupements hydroxyles de la cellulose peuvent réagir partiellement ou totalement avec différents réactifs chimiques pour donner des dérivés cellulosiques possédant des propriétés propres. Les dérivés de celluloses peuvent être anioniques, cationiques, amphotères ou non-ioniques. Parmi ces dérivés, on distingue les éthers de celluloses, les esters de celluloses et les esters éthers de celluloses.

Parmi les éthers de cellulose non ioniques, on peut citer les alkylcelluloses telles que les méthylcelluloses et les éthylcelluloses ; les hydroxyalkylcelluloses telles que les hydroxyméthylcelluloses, les hydroxyéthylcelluloses et les hydroxypropylcelluloses ; les celluloses mixtes hydroxyalkyl-alkylcelluloses telles que les hydroxypropylméthylcelluloses, les hydroxyéthylméthylcelluloses, les hydroxyéthyléthylcelluloses et les hydroxybutyl-méthylcelluloses.

Parmi les éthers de cellulose anioniques, on peut citer les carboxyalkylcelluloses et leurs sels. A titre d'exemple, on peut citer les carboxyméthylcelluloses, les carboxyméthylméthylcelluloses et les carboxyméthylhydroxyéthylcelluloses et leurs sels de sodium.

Parmi les éthers de cellulose cationiques, on peut citer les hydroxyéthylcelluloses quaternisées réticulées ou non.

Le quaternisant peut être notamment le chlorure de glycidyltriméthylammonium. Comme autre éther de cellulose cationique, on peut citer l'hydroxyéthylcellulosehydroxypropyltriméthylammonium.

Parmi les esters de celluloses, on trouve les esters inorganiques de cellulose (nitrates, sulfates, ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulfates d'éthylcellulose.

Les composés cellulosiques de l'invention peuvent être choisis parmi les celluloses non substituées et les celluloses substituées.

Les celluloses et dérivés sont représentés par exemple par les produits vendus sous les dénominations Avicel® (microcristalline cellulose, MCC) par la société FMC Biopolymers, sous la dénomination Cekol (carboxyméthylcellulose) par la société Noviant (CP-Kelco), sous la dénomination Akucell AF (sodium carboxyméthylcellulose) par la société Akzo Nobel, sous la dénomination MethocelTM (éthers de cellulose) et, sous les dénominations Aqualon® (carboxyméthylcellulose et sodium carboxyméthylcellulose), Benecel® (méthylcellulose), BlanoseTM (carboxyméthylcellulose), Culminal® (Méthylcellulose, hydroxypropyl méthylcellulose), Klucel® (hydroxypropylcellulose), et Natrosol® CS (hydroxyéthylcellulose) par la société Hercules Aqualon.

*Fructosanes*

Le polysaccharide selon l'invention peut notamment être un fructosane choisi parmi l'inuline et ses dérivés (notamment dicarboxy et carboxyméthyl inulines).

Les fructanes ou fructosanes sont des oligosaccharides ou des polysaccharides comprenant un enchaînement d'unités anhydrofructose éventuellement associé à un plusieurs résidus saccharidiques différents du fructose. Les fructanes peuvent être linéaires ou ramifiés. Les fructanes peuvent être des produits obtenus directement à partir d'une source végétale ou microbienne ou bien des produits dont la longueur de chaîne a été modifiée (augmentée ou réduite) par fractionnement, synthèse ou hydrolyse en particulier enzymatique. Les fructanes ont généralement un degré de polymérisation de 2 à environ 1000, et de préférence de 2 à environ 60.

On distingue 3 groupes de fructanes. Le premier groupe correspond à des produits dont les unités fructose sont pour la plupart liées par des liaisons $\beta(2,1)$. Ce sont des fructanes essentiellement linéaires tes que les inulines.

Le second groupe correspond également à des fructoses linéaires mais les unités fructose sont essentiellement liées par des liaisons $\beta(2,6)$. Ces produits sont des levanes.

Le troisième groupe correspond à des fructanes mixtes, c'est à dire ayant des enchainements $\beta(2,6)$ et $\beta(2,1)$. Ce sont des fructanes essentiellement ramifiés tes que les graminanes.

Les fructanes préférés dans les compositions selon l'invention sont les inulines. L'inuline peut être obtenue par exemple à partir de chicorée, de dahlia ou de topinambours, de préférence à partir de chicorée.

En particulier, le polysaccharide, notamment de l'inuline, présente un degré de polymérisation de 2 à environ 1000 et de préférence de 2 à environ 60, et un degré de substitution inférieur à 2 sur la base d'une unité fructose.

L'inuline utilisée pour cette invention est représentée par exemple par les produits vendus sous la dénomination BeneoTM inulin par la société Orafti, et sous la dénomination Frutafit® par la société Sensus.

b) Polysaccharides hétérogènes et leurs dérivés

Les polysaccharides utilisables selon l'invention peuvent être des gommes comme par exemple la gomme de cassia, karaya, konjac, adragante, de tara, acacia ou arabique.

*Gomme arabique*

La gomme arabique est un polysaccharide acide fortement ramifié qui se présente sous la forme de mélanges de sels de potassium, de magnésium et de calcium. Les éléments monomères de l'acide libre (acide arabique) sont le D-galactose, le L-arabinose, le L-rhamnose et l'acide D-glucuronique.

*Galactomannanes (guar, caroube, fenugrec, gomme tara) et dérivés (guar phosphaté, hydroxypropyl guar* ...)

Les galactomannanes sont des polyosides non ioniques extraits de l'albumen de graines de légumineuses dont ils constituent le glucide de réserve.

Les galactomannanes sont des macromolécules constituées d'une chaîne principale d'unités D-mannopyranose liées en $\beta(1,4)$, portant des branchements latéraux constitués d'une seule unité D-galactopyranose liée en $\alpha(1,6)$ à la chaîne principale. Les différents galactomannanes se distinguent d'une part par la proportion d'unités $\alpha$-D-galactopyranose présentes dans le polymère, et d'autre part par des différences significatives en terme de distribution des unités galactose le long la chaine de mannose.

Le rapport mannose/galactose (M/G) est de l'ordre de 2 pour la gomme guar, de 3 pour la gomme tara et de 4 pour la gomme de caroube.

Les galactomannanes présentent la structure chimique suivante :

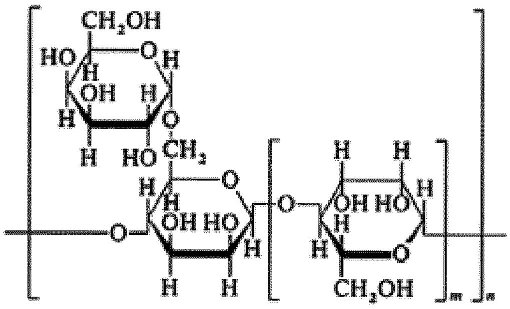

m = 3: Locust beam gum
m = 1: Guar gum
m = 2: Tara gum

*Guar*

La gomme de guar est caractérisée par un ratio mannose:galactose de l'ordre de 2:1. Le groupement galactose est régulièrement distribué le long de la chaîne de mannose.

Les gommes de guar utilisables selon l'invention peuvent être non ioniques, cationiques ou anioniques. Selon l'invention, on peut utiliser les gommes de guar non ioniques chimiquement modifiées ou non modifiées.

Les gommes de guar non ioniques non modifiées sont par exemple les produits vendus sous la dénomination Vidogum GH, Vidogum G et Vidocrem par la société Unipektin et sous la dénomination Jaguar par la société Rhodia, sous la dénomination Meypro® Guar par la société Danisco, sous la dénomination VISCOGUMTM par la société Cargill, et sous la dénomination Supercol® guar gum par la société Aqualon.

Les gommes de guar non-ioniques hydrolysées utilisables selon l'invention sont par exemple représentées par les produits vendus sous la dénomination Meyprodor® par la société Danisco.

Les gommes de guar non-ioniques modifiées utilisables selon l'invention sont de préférence modifiées par des groupements hydroxyalkyle en $C_1$-$C_6$, parmi lesquels, on peut mentionner à titre d'exemple, les groupements hydroxyméthyle, hydroxyéthyle, hydroxypropyle et hydroxybutyle.

De telles gommes de guar non-ioniques éventuellement modifiées par des groupements hydroxyalkyle sont par exemple vendues sous les dénominations commerciales Jaguar HP 60, Jaguar HP 105 et Jaguar HP 120 (hydroxypropyl guar), par la société Rhodia, ou sous la dénomination N-Hance® HP (hydroxypropyl guar) par la société AQUALON.

Les gommes de galactomannane cationiques ont de préférence une densité de charge cationique inférieure ou égale à 1,5 meq/g et plus particulièrement comprise entre 0,1 et 1 meq/g. La densité de charge peut être déterminée selon la méthode Kjeldahl. Elle correspond en général à un pH de l'ordre de 3 à 9.

De manière générale, au sens de la présente invention, on entend par « *gomme de galactomannane cationique* » toute gomme de galactomannane contenant des groupements cationiques et/ou des groupements ionisables en groupements cationiques.

Les groupements cationiques préférés sont choisis parmi ceux comportant des groupements amine primaires, secondaires, tertiaires et/ou quaternaires.

Les gommes de galactomannane cationiques utilisées ont généralement une masse moléculaire moyenne en poids comprise entre 500 et $5x10^6$ environ, et de préférence comprise entre $10^3$ et $3x10^6$ environ.

Les gommes de galactomannane cationiques utilisables selon la présente invention sont par exemple des gommes comportant des groupements cationiques trialkyl ($C_1$-$C_4$) ammonium. De préférence, 2 % à 30 % en nombre des fonctions hydroxyles de ces gommes porte des groupements cationiques trialkylammonium.

Parmi ces groupements trialkylammonium, on peut tout particulièrement citer les groupements triméthylammonium et triéthylammonium.

Encore plus préférentiellement, ces groupements représentent de 5 % à 20 % en poids du poids total de la gomme de galactomannane modifiée.

Selon l'invention, la gomme de galactomannane cationique est de préférence une gomme de guar comportant des groupements hydroxypropyl triméthylammonium, c'est à dire une gomme de guar modifiée par exemple par du chlorure de 2,3-époxypropyl triméthylammonium.

Ces gommes de galactomannane en particulier de guar modifiées par des groupements cationiques sont des produits déjà connus en eux-mêmes et sont par exemple décrits dans les brevets US 3 589 578 et US 4 031 307. De tels produits sont par ailleurs vendus notamment sous les dénominations commerciales de Jaguar EXCEL, Jaguar C13 S, Jaguar C 15, Jaguar C 17 et Jaguar C162 (Guar Hydroxypropyltrimonium Chloride) par la société Rhodia, sous la dénomination Amilan® Guar (Guar Hydroxypropyltrimonium Chloride) par la société Degussa, et sous la dénomination N-Hance® 3000 (Guar Hydroxypropyltrimonium Chloride) par la société Aqualon.

Les gommes de guar anioniques utilisables selon l'invention sont des polymères comportant des groupements dérivés d'acide carboxylique, sulfonique, sulfénique, phosphorique, phosphonique ou acide pyruvique. De préférence, le groupement anionique est un groupement acide carboxylique. Le groupement anionique peut également se présenter sous forme d'un sel d'acide, notamment un sel de sodium, de calcium, de lithium ou de potassium.

Les gommes de guar anioniques utilisables selon l'invention sont préférentiellement des dérivés de guar carboxyméthylés (carboxyméthyl guar ou carboxyméthyl hydroxypropyl guar).

*Caroube*

La gomme de caroube est extraite des graines de caroubier (Ceratonia siliqua).

La gomme de caroube non modifiée utilisable dans cette invention est vendue par exemple sous la dénomination Viscogum™ par la société Cargill, sous la dénomination Vidogum L par la société Unipektin, sous la dénomination Grinsted® LBG par la société Danisco.

Les gommes de caroube modifiée chimiquement utilisables dans cette invention peuvent être représentées par exemple par les caroubes cationiques vendues sous la dénomination Catinal CLB (caroube Hydroxypropyltrimonium Chloride) par la société Toho.

*Gomme TARA*

La gomme Tara utilisable dans le cadre de cette invention est vendue par exemple sous la dénomination Vidogum SP par la société Unipektin.

*Glucomannanes (somme de koniac)*

Le glucomannane est un polysaccharide de poids moléculaire élevé (500 000 < Mglucomannane < 2 000 000), composé d'unités de D-mannose et de D-glucose avec une ramification toutes les 50 ou 60 unités environ. On le trouve dans le bois mais c'est aussi le principal constituant de la gomme de Konjac. Le konjac (Amorphophallus konjac) est une plante de la famille des Araceae.

Les produits utilisables selon l'invention sont par exemple vendus sous la dénomination Propol® et Rheolex® par la société Shimizu.

*Pectines LM et HM, et dérivés*

Les pectines sont des polymères linéaires d'acide $\alpha$-D-galacturonique (au moins 65 %) liés en position 1 et 4, avec une certaine proportion de groupes carboxyliques estérifiés avec un groupement méthanol. Environ 20 % des sucres constituant la molécule de pectine sont des sucres neutres (L-rhamnose, D-glucose, D-galactose, L-arabinose, D-xylose). Les résidus de L-rhamnose se trouvent dans toutes les pectines, intégrés à la chaîne principale en positions 1,2.

Les molécules d'acide uroniques possèdent des fonctions carboxyles. Cette fonction confère aux pectines la capacité d'échanger des ions, lorsque ceux-ci sont sous forme $COO^-$. Les ions bivalents (calcium en particulier) ont la capacité de former des ponts ioniques entre deux groupements carboxyles de deux molécules de pectine différentes.

A l'état naturel, une certaine proportion des groupes carboxyliques sont estérifiés par un groupe méthanol. Le degré d'estérification naturel d'une pectine peut varier entre 70 % (pomme, citron) et 10 % (fraise) suivant la source utilisée. A partir de pectines de haut degré d'estérification, il est possible d'hydrolyser les groupements $-COOCH_3$, afin d'obtenir des pectines faiblement estérifiées. Selon la proportion de monomères méthylés ou non, la chaîne est donc plus ou moins acide. On définit ainsi les pectines HM (High methoxy), ayant un degré d'estérification supérieur à 50 %, et les pectines LM (Low Methoxy), ayant un degré d'estérification inférieur à 50 %.

Dans le cas des pectines amidées, le groupement -OCH3 est substitué par un groupement $-NH_2$.

Les pectines sont notamment commercialisées par la société Cargill sous la dénomination UnipectineTM, par la société CP-Kelco sous la dénomination GENU, par Danisco sous la dénomination GRINSTED Pectin.

Autres Polysaccharides

Parmi les autres polysaccharides utilisables selon l'invention, on peut également citer la chitine (Poly N-acétyl-D-glucosamine, $\beta$(1,4)-2-Acétamido-2-désoxy-D-glucose), le chitosane et dérivés (chitosan-beta-glycerophosphate, carboxymethylchitine, etc.) comme ceux vendus par la société France-Chitine ; les Glycosaminoglycanes (GAG) tels que l'acide hyaluronique, le chondroïtine sulfate, le dermatane sulfate, le kératane sulfate, et de préférence l'acide hyaluronique ; les xylanes (ou arabinoxylanes) et dérivés.

Les arabinoxylanes sont des polymères de xylose et d'arabinose, tous regroupés sous l'appellation « *pentosanes ».*

Les xylanes sont constitués d'une chaîne principale d'unités de D-xylose liées en $\beta$(1,4) et sur lesquelles on trouve trois substituants (Rouau & Thibault, 1987) : des unités acides, des unités de $\alpha$-L-arabinofuranose, des chaînes latérales pouvant contenir de l'arabinose, du xylose, du galactose et de l'acide glucuronique.

Selon cette variante, le polysaccharide est de préférence l'acide hyaluronique, ou l'un de ses sels tel que le sel de sodium (hyaluronate de sodium)

## II. Gélifiants polymériques synthétiques

Au sens de l'invention, le terme synthétique signifie que le polymère n'est ni naturellement existant ni ne dérive d'un polymère d'origine naturelle.

Le gélifiant hydrophile polymérique synthétique considéré selon l'invention peut être particulaire ou non.

Au sens de l'invention, le terme particulaire signifie que le polymère se présente sous forme de particules, de préférence sphériques.

Comme il ressort de ce qui suit, le gélifiant hydrophile polymérique est avantageusement choisi parmi les homo- ou co-polymères acryliques réticulés ; les polyacrylamides et les polymères et copolymères d'acide 2-acrylamido 2-méthylpropane sulfonique, réticulés et/ou neutralisés ; les polymères carboxyvinyliques, modifiés ou non, et leurs mélanges, notamment tel que défini ci-après.

### *II.A. Gélifiants polymériques synthétiques particulaires*

Ils sont de préférence choisis parmi les polymères réticulés.

Il peut notamment s'agir d'homo- ou co-polymères acryliques réticulés, de préférence partiellement neutralisés ou neutralisés, qui se présentent sous forme particulaire.

Selon un mode de réalisation, le gélifiant particulaire selon la présente invention est choisi parmi les polyacrylates de sodium réticulés. De préférence, il présente, à l'état sec ou non hydraté, une taille moyenne inférieure ou égale à 100 $\mu$m, de préférence inférieure ou égale à 50 $\mu$m. La taille moyenne des particules correspond au diamètre moyen en masse mesurée par granulométrie laser ou autre méthode équivalente connue de l'homme du métier.

Ainsi, de préférence, le gélifiant particulaire selon la présente invention est choisi parmi les polyacrylates de sodium réticulés, de préférence sous forme de particules ayant une taille moyenne (ou diamètre moyen) inférieure ou égale à 100 microns, de préférence encore sous forme de particules sphériques.

A titre d'exemple de polyacrylates de sodium réticulés on peut citer ceux commercialisés sous les dénominations Octacare X100, X110 et RM100 par la société Avecia, ceux commercialisés sous les dénominations Flocare GB300 et le Flosorb 500 par la société SNF, ceux commercialisés sous les dénominations Luquasorb 1003, Luquasorb 1010, Luquasorb 1280 et Luquasorb 1110 par la société BASF, ceux commercialisés sous les dénominations Water Lock G400 et G430 (nom INCI : Acrylamide/Sodium acrylate copolymer) par la société Grain Processing.

On peut également citer les microsphères de polyacrylate réticulées telles que par exemple celles commercialisées sous la dénomination AQUAKEEP® 10 SH NF proposé par la société Sumitomo Seika.

De tels gélifiants peuvent être mis en œuvre à raison de 0,1 % à 5 % en poids en matière sèche par rapport au poids total de la phase aqueuse, notamment de 0,5 % à 2 % en poids, et en particulier à raison d'environ de 0,8 % à 1,7 % en poids par rapport au poids total de la phase aqueuse.

### *II.B. Gélifiants polymériques synthétiques non particulaires*

Cette famille de gélifiant peut être détaillée en les sous-familles qui suivent :

1.. Les polyacrylamides et les polymères et copolymères d'acide 2- acrylamido 2 méthylpropane sulfonique, réticulés et/ou neutralisés, et

2. Les polymères carboxyvinyliques modifiés ou non.

#### *II.B.1 Les polyacrylamides et les polymères et copolymères d'acide 2-acrylamido 2-méthylpropane sulfonique, réticulés et/ou neutralisés*

Les polymères utilisés convenant à titre de gélifiant aqueux pour l'invention peuvent être des homopolymères ou copolymères, réticulés ou non-réticulés comportant au moins le monomère acide acrylamido 2-méthyl propane sulfonique (AMPS®), sous forme partiellement ou totalement neutralisée par une base minérale telle que la soude ou la potasse.

Ils sont de préférence neutralisés totalement ou pratiquement totalement neutralisés, c'est-à-dire neutralisés à

au moins 90 %.

Ces polymères d'AMPS® selon l'invention peuvent être réticulés ou non-réticulés.

Lorsque les polymères sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth) acrylate de d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Selon un mode préféré de réalisation de l'invention, l'agent de réticulation est choisi parmi le méthylène-bis-acrylamide, le méthacrylate d'allyle ou le triméthylol propane triacrylate (TMPTA). Le taux de réticulation va en général de 0,01 % à 10 % en moles et plus particulièrement de 0,2 % à 2 % en moles par rapport au polymère.

Les polymères d'AMPS® convenant à l'invention sont hydrosolubles ou hydrodispersibles. Ils sont dans ce cas :

- soit des « homopolymères » ne comportant que des monomères AMPS et, s'ils sont réticulés, un ou plusieurs agents de réticulation tels que ceux définis ci-dessus ;
- soit des copolymères obtenus à partir de l'AMPS® et d'un ou plusieurs monomères à insaturation éthylénique hydrophiles ou hydrophobes et, s'ils sont réticulés, un ou plusieurs agents de réticulation tels que ceux définis ci-dessus. Lorsque lesdits copolymères comportent des monomères à insaturation éthylénique hydrophobes, ces derniers ne comportent pas de chaîne grasse et sont de préférence présents dans de faibles quantités.

On entend par « *chaîne grasse* », au sens de la présente invention, toute chaîne hydrocarbonée comportant au moins 7 atomes de carbone.

Par « *hydrosoluble ou hydrodispersible* », on entend des polymères qui, introduits dans une phase aqueuse à 25 °C, à une concentration massique égale à 1 %, permettent l'obtention d'une solution macroscopiquement homogène et transparente, c'est à dire ayant une valeur de transmittance maximum de la lumière, à une longueur d'onde égale à 500 nm, à travers un échantillon de 1 cm d'épaisseur, d'au moins 60 %, de préférence d'au moins 70 %.

Les « *homopolymères* » selon l'invention sont de préférence réticulés et neutralisés, et ils peuvent être obtenus selon le procédé de préparation comprenant les étapes suivantes :

(a) on disperse ou on dissout le monomère tel que l'AMPS sous forme libre dans une solution de tertio-butanol ou d'eau et de tertio-butanol ;

(b) on neutralise la solution ou la dispersion de monomère obtenue en (a) par une ou plusieurs bases minérales ou organiques, de préférence l'ammoniaque $NH_3$, dans une quantité permettant d'obtenir un taux de neutralisation des fonctions acides sulfoniques du polymère allant de 90 % à 100 % ;

(c) on ajoute à la solution ou dispersion obtenue en (b), le ou les monomères réticulants ;

(d) on effectue une polymérisation radicalaire classique en la présence d'amorceurs de radicaux libres à une température allant de 10 °C à 150 °C ; le polymère précipitant dans la solution ou la dispersion à base de tertio-butanol.

Les copolymères hydrosolubles ou hydrodispersibles d'AMPS® selon l'invention contiennent des monomères à insaturation éthylénique hydrosolubles, des monomères hydrophobes ou leurs mélanges.

Les co-monomères hydrosolubles peuvent être ioniques ou non-ioniques.

Parmi les co-monomères hydrosolubles ioniques, on peut citer par exemple les composés suivants et leurs sels :

- l'acide (méth)acrylique,
- l'acide styrène sulfonique,
- l'acide vinylsulfonique et l'acide (méth)allylsulfonique,
- l'acide vinyl phosphonique,
- l'acide maléique,
- l'acide itaconique,
- l'acide crotonique,
- les monomères vinyliques hydrosolubles de formule (A) suivante :

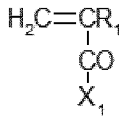 (A)

dans laquelle :

- $R_1$ est choisi parmi H, $-CH_3$, $-C_2H_5$ ou $-C_3H_7$;
- $X_1$ est choisi parmi :
- les oxydes d'alkyle de type $-OR_2$ où $R_2$ est un radical hydrocarboné, linéaire ou ramifié, saturé ou insaturé, ayant de 1 à 6 atomes de carbone, substitué par au moins un groupement sulfonique ($-SO_3-$) et/ou sulfate ($-SO_4-$) et/ou phosphate ($-PO_4H_2-$).

Parmi, les co-monomères hydrosolubles non-ioniques, on peut citer par exemple :

- le (méth)acrylamide,
- la N-vinylacétamide et la N-méthyl N-vinylacétamide,
- la N-vinylformamide et la N-méthyl N-vinylformamide,
- l'anhydride maléique,
- la vinylamine,
- les N-vinyllactames comportant un groupe alkyl cyclique ayant de 4 à 9 atomes de carbone, tels que la N-vinylpyrrolidone, la N-butyrolactame et la N-vinylcaprolactame,
- l'alcool vinylique de formule $CH_2$=CHOH,
- les monomères vinyliques hydrosolubles de formule (B) suivante :

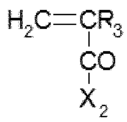 (B)

dans laquelle :

- $R_3$ est choisi parmi H, $-CH_3$, $-C_2H_5$ ou $-C_3H_7$;
- $X_2$ est choisi parmi les oxydes d'alkyle de type $-OR_4$ où $R_4$ est un radical hydrocarboné, linéaire ou ramifié, saturé ou insaturé, ayant de 1 à 6 carbones, éventuellement substitué par un atome d'halogène (iode, brome, chlore, fluor) ; un groupement hydroxy (-OH) ; éther.

Citons par exemple le (méth)acrylate de glycidyle, le méthacrylate d'hydroxyéthyle, et les (méth)acrylates d'éthylène glycol, de diéthylèneglycol ou de polyalkylèneglycol.

Parmi les comonomères hydrophobes sans chaîne grasse, on peut citer par exemple :

- le styrène et ses dérivés tel que le 4-butylstyrène, l'alpha méthylstyrène et le vinyltoluène ;
- l'acétate de vinyle de formule $CH_2$=CH-$OCOCH_3$;
- les vinyléthers de formule $CH_2$=CHOR dans laquelle R est un radical hydrocarboné, linéaire ou ramifié, saturé ou insaturé, ayant de 1 à 6 atomes de carbone ;
- l'acrylonitrile ;
- la caprolactone ;
- le chlorure de vinyle et le chlorure de vinylidène ;
- les dérivés siliconés, conduisant après polymérisation à des polymères siliconés tels que le méthacryloxypropyltris(triméthylsiloxy)silane et les méthacrylamides siliconés ;
- les monomères vinyliques hydrophobes de formule (C) suivante :

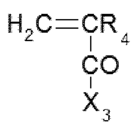 (C)

dans laquelle :

- $R_4$ est choisi parmi H, $-CH_3$, $-C_2H_5$ ou $-C_3H_7$;
- $X_3$ est choisi parmi :
- les oxydes d'alkyle de type $-OR_5$ où $R_5$ est un radical hydrocarboné, linéaire ou ramifié, saturé ou insaturé, ayant de 1 à 6 atomes de carbone.

Citons par exemple, le méthacrylate de méthyle, le méthacrylate d'éthyle, le (meth)acrylate de n-butyle, le (meth) acrylate de tertio-butyle, l'acrylate de cyclohexyle et l'acrylate d'isobornyle et l'acrylate d'éthyle 2-hexyle.

Les polymères d'AMPS® hydrosolubles ou hydrodispersibles de l'invention ont de préférence une masse molaire allant de 50 000 g/mole à 10 000 000 g/mole, de préférence de 80 000 g/mole à 8 000 000 g/mole, et de façon encore plus préférée de 100 000 g/mole à 7 000 000 g/mole.

Comme homopolymères hydrosolubles ou hydrodispersibles d'AMPS convenant à l'invention, on peut citer par exemple les polymères réticulés ou non d'acrylamido-2-methyl propane sulfonate de sodium tel que celui utilisé dans le produit commercial Simulgel 800 (nom CTFA: Sodium Polyacryloyldimethyl Taurate), les polymères réticulés d'acrylamido-2-methyl propane sulfonate d'ammonium (nom INCI : Ammonium Polyacryldiméthyltauramide) que ceux décrits dans le brevet EP 0 815 928 B1 et tel que le produit vendu sous le nom commercial Hostacerin AMPS® par la société Clariant.

Comme homopolymères hydrosolubles ou hydrodispersibles d'AMPS préférés conformes à l'invention, on peut citer les polymères réticulés d'acrylamido-2-methyl propane sulfonate d'ammonium.

Comme copolymères hydrosolubles ou hydrodispersibles d'AMPS conformes à l'invention, on peut citer par exemple :

- les copolymères réticulés acrylamide/acrylamido-2-methyl propane sulfonate de sodium tels que celui utilisé dans le produit commercial SEPIGEL 305 (nom CTFA : Polyacrylamide/$C_{13}$-$C_{14}$ Isoparaffin/ Laureth-7) ou celui utilisé dans le produit commercial vendu sous la dénomination Simulgel 600 (nom CTFA : Acrylamide/Sodium acryloyldiméthyltaurate/Isohexadécane/Polysorbate-80) par la société Seppic ;
- les copolymères d'AMPS® et de vinylpyrrolidone ou de vinylformamide tels que celui utilisé dans le produit commercial vendu sous la dénomination Aristoflex AVC® par la société Clariant (nom CTFA : Ammonium Acryloyldiméthyltaurate/ VP Copolymer) mais neutralisé par la soude ou la potasse ;
- les copolymères d'AMPS® et d'acrylate de sodium, comme par exemple le copolymère AMPS/acrylate de sodium tel que celui utilisé dans le produit commercial vendu sous la dénomination Simulgel EG® par la société Seppic ;
- les copolymères d'AMPS® et d'hydroxyéthyl acrylate, comme par exemple le copolymère AMPS®/hydroxyéthyl acrylate tel que celui utilisé dans le produit commercial vendu sous la dénomination Simulgel NS® par la société Seppic (nom CTFA : Hydroxyéthyl Acrylate/Sodium Acryloyldiméthyltaurate copolymer (And) Squalane (And) Polysorbate 60) ou comme le produit commercialisé sous le nom Copolymère Acrylamido-2-Méthyl propane Sulfonate de Sodium/Hydroxyéthylacrylate comme le produit commercial Sepinov EM ou Sepinov EMT 10 (nom INCI: Hydroxyéthyl Acrylate/Sodium Acryloyldiméthyl taurate copolymer).

Comme copolymères hydrosolubles ou hydrodispersibles d'AMPS préférés conformes à l'invention, on peut citer les copolymères d'AMPS® et d'hydroxyéthyl acrylate.

D'une manière générale, une phase aqueuse selon l'invention peut comprendre de 0,1 % à 12 % en poids en matière sèche de préférence de 0,3 % à 10 % en poids et plus préférentiellement de 0,5 % à 8 % en poids de polyacrylamide(s) et/ou de polymère(s) et copolymère(s) d'acide 2-acrylamido 2-méthylpropane sulfonique, réticulé (s) et/ou neutralisé(s) par rapport à son poids total.

*II.B.2 Les polymères carboxyvinyliques modifiés ou non*

Les polymères carboxyvinyliques modifiés ou non peuvent être des copolymères issus de la polymérisation d'au moins un monomère (a) choisi parmi les acides carboxyliques à insaturation a,b-éthylénique ou leurs esters, avec au moins un monomère (b) à insaturation éthylénique comportant un groupement hydrophobe.

On entend par « *copolymères* » aussi bien les copolymères obtenus à partir de deux sortes de monomères que ceux obtenus à partir de plus de deux sortes de monomères tels que les terpolymères obtenus à partir de trois sortes de monomères.

Notamment parmi les polymères carboxyvinyliques modifiés ou non, on peut encore citer les polyacrylates de sodium tels que ceux commercialisés sous la dénomination Cosmedia SP® contenant 90 % de matière sèche et 10 % d'eau, ou Cosmedia SPL® en émulsion inverse contenant environ 60 % de matière sèche, une huile (polydecene hydrogéné) et un tensio-actif (PPG-5 Laureth-5), tous deux vendus par la société Cognis.

On peut également citer les polyacrylates de sodium partiellement neutralisés se trouvant sous forme d'une émulsion inverse comprenant au moins une huile polaire, par exemple celui vendu sous la dénomination Luvigel® EM par

la société BASF.

Les polymères carboxyvinyliques modifiés ou non peuvent être également choisi parmi les homopolymères d'acide (méth)acrylique réticulés.

Par « *(méth)acrylique* » au sens de la présente demande, on entend « *acrylique ou méthacrylique* ».

A titre d'exemple, on peut citer ceux vendus par Lubrizol sous les dénominations Carbopol, 910, 934, 940, 941, 934 P, 980, 981, 2984, 5984, Carbopol Ultrez 10 Polymer, ou par 3V-Sigma sous la dénomination Synthalen® K, Synthalen® L, ou Synthalen® M.

Parmi les polymères carboxyvinyliques modifiés ou non, on peut en particulier citer le Carbopol (nom CTFA : carbomer) et le Pemulen (nom CTFA : Acrylates/$C_{10-30}$ akyl acrylate crosspolymer) commercialisés par la société Lubrizol

Les polymères carboxyvinyliques, modifiés ou non, peuvent être présents à raison de 0,1 % à 10 % en poids en matière sèche par rapport au poids de la phase aqueuse, en particulier de 0,3 % à 8 % en poids, de préférence entre 0,4 et 6 % par rapport au poids de la phase aqueuse.

Avantageusement, une composition selon l'invention comprend au moins un gélifiant polymérique synthétique, de préférence choisi parmi les polymères et copolymères d'acide 2-acrylamido 2-méthylpropane sulfonique, réticulés et/ou neutralisés et les polymères carboxyvinyliques modifiés ou non.

Selon une variante préférée, le gélifiant hydrophile polymérique synthétique est choisi parmi les polymères réticulés d'acrylamido-2-methyl propane sulfonate d'ammonium, les copolymères d'AMPS® et d'hydroxyéthyl acrylate, et les homopolymères d'acide (méth)acrylique réticulés, de préférence les copolymères d'AMPS® et d'hydroxyéthyl acrylate.

## III. Autres gélifiants hydrophiles

Ces gélifiants sont plus particulièrement choisis parmi les silicates mixtes et les silices pyrogénées.

### *III.A. Silicate mixte*

Au sens de la présente invention, on entend par silicate mixte tous les silicates d'origine naturelle ou synthétique renfermant plusieurs (deux ou plus) types de cations choisis parmi les métaux alcalins (par exemple Na, Li, K) ou alcalino-terreux (par exemple Be, Mg, Ca), les métaux de transition et l'aluminium.

Selon un mode de réalisation particulier, le ou les silicates mixtes se présentent sous la forme de particules solides contenant au moins 10 % en poids d'au moins un silicate par rapport au poids total des particules. Dans la suite du présent exposé, ces particules sont désignées par « *particules de silicate ».*

De préférence, les particules de silicate contiennent moins de 1 % en poids d'aluminium par rapport au poids total des particules. De manière encore plus préférée, elles contiennent de 0 % à 1 % en poids d'aluminium par rapport au poids total des particules.

De préférence, les particules de silicate contiennent au moins 50 % en poids de silicate, mieux encore au moins 70 % en poids par rapport au poids total des particules. Les particules contenant au moins 90 % en poids de silicates, par rapport au poids total des particules, sont particulièrement préférées.

En particulier, il s'agit d'un silicate ou d'un mélange de silicates de métaux alcalins ou alcalino terreux, d'aluminium ou de fer.

De préférence, il s'agit de silicate de sodium, de magnésium et/ou de litium.

Pour garantir de bonnes propriétés cosmétiques, ces silicates se présentent généralement sous une forme finement divisée, et en particulier sous forme de particules ayant une taille moyenne allant de 2 nm à 1 $\mu$m (de 2 nm à 1000 nm), et de préférence de 5 nm à 600 nm, et encore plus préférentiellement de 20 à 250 nm.

Les particules de silicate peuvent avoir une forme quelconque, par exemple la forme de sphères, de paillettes, d'aiguilles, de plaquettes, de disques, de feuillets, ou des formes totalement aléatoires. De préférence, les particules de silicates ont la forme de disques ou de feuillets.

Aussi, on entend par « *taille moyenne* » des particules la taille moyenne en nombre de la plus grande dimension (longueur) qu'il est possible de mesurer entre deux points diamétralement opposés d'une particule individuelle. La taille peut être déterminée par exemple par microscopie électronique à transmission, ou à partir de la mesure de la surface spécifique par la méthode BET, ou encore par l'intermédiaire d'un granulomètre laser.

Lorsque les particules sous forme de disques ou de feuillets, elles ont généralement une épaisseur allant d'environ 0,5 nm à 5 nm.

Les particules de silicate peuvent être constituées d'un alliage avec des oxydes de métaux ou métalloïdes, obtenu par exemple par fusion thermique de ses différents constituants. Lorsque les particules comprennent en outre un tel oxyde de métal ou métalloïde, celui-ci est de préférence choisi parmi l'oxyde de silicium, de bore ou d'aluminium.

Selon un mode de réalisation particulier de l'invention, les silicates sont des phyllosilicates, à savoir des silicates ayant une structure dans laquelle les tétraèdres $SiO_4$ sont organisés en feuillets entre lesquels se trouvent enfermés les

cations métalliques.

Les silicates mixtes convenant à l'invention peuvent être choisis par exemple parmi les montmorillonites, les hectorites, les bentonites, la beidellite, les saponites. Selon un mode préféré de réalisation de l'invention, les silicates mixtes utilisés sont plus particulièrement choisis parmi les hectorites et les bentonites, et encore mieux parmi les laponites.

Une famille de silicates particulièrement préférée dans les compositions de la présente invention est donc celle des laponites. Les laponites sont des silicates de magnésium, de sodium et éventuellement de lithium, ayant une structure en couches semblable à celle des montmorillonites. La laponite est la forme synthétique du minéral naturel appelé « *hectorite* ». L'origine synthétique de cette famille de silicates présente un avantage considérable par rapport à la forme naturelle car elle permet une bonne maîtrise de la composition du produit. En outre, les laponites ont l'avantage d'avoir une taille de particules bien inférieure à celles de l'hectorite et de la bentonite naturelles.

Comme laponites, on peut citer notamment les produits vendus sous les dénominations suivantes : Laponite® XLS, Laponite® XLG, Laponite® RD, Laponite® RDS, LAPONITE® XL21 (ces produits sont des silicates de sodium et de magnésium et des silicates de sodium, de lithium et de magnésium) par la société Rockwood Additives Limited.

De tels gélifiants peuvent être mis en œuvre à raison de 0,1 % à 8 % en poids en matière sèche par rapport au poids total de la phase aqueuse, notamment de 0,1 % à 5 % en poids, et en particulier de 0,5 % à 3 % en poids par rapport au poids total de la phase aqueuse.

***III.B. Silice pyrogénée hydrophile***

Les silices pyrogénées selon la présente invention sont hydrophiles.

Les silices hydrophiles pyrogénées sont obtenues par pyrolyse en flamme continue à 1000 °C du tétrachlorure de silicium ($SiCl_4$) en présence d'hydrogène et d'oxygène. Parmi les silices pyrogénées à caractère hydrophile utilisables selon la présente invention, on peut citer notamment celles vendues par la société DEGUSSA ou EVONIK DEGUSSA sous les dénominations commerciales AEROSIL® 90, 130, 150, 200, 300 et 380, ou encore par la société CABOT sous la dénomination Carbosil H5.

De tels gélifiants peuvent être mis en œuvre à raison de 0,1 % à 10 % en poids en matière sèche par rapport au poids total de la phase aqueuse, notamment de 0,1 % à 5 % en poids, et en particulier de 0,5 % à 3 % en poids par rapport au poids total de la phase aqueuse.

**POLYSACCHARIDES AMYLACES**

Selon un mode particulier, la composition selon la présente invention peut en outre comprendre au moins un gélifiant hydrophile additionnel choisi parmi les polysaccharides amylacés.

Le gélifiant additionnel amylacé est hydrosoluble ou hydrodispersible.

A titre représentatif de cette catégorie peuvent être tout particulièrement cités, les amidons natifs et les amidons modifiés.

Amidons natifs

Les amidons utilisables dans la présente invention sont plus particulièrement des macromolécules sous forme de polymères constitués de motifs élémentaires qui sont des unités anhydroglucose (dextrose), liées par liaisons α(1,4), de formule chimique $(C_6H_{10}O_5)_n$. Le nombre de ces motifs et leur assemblage permettent de distinguer l'amylose, molécule formée d'environ 600 à 1000 molécules de glucose chaînées linéairement, et l'amylopectine, polymère ramifié tous les 25 résidus glucoses environ (liaison α(1,6). La chaine totale peut faire entre 10000 et 100000 résidus glucoses.

L'amidon est décrit en particulier dans « KIRK-OTHMER ENCYCLOPEDIA OF CHEMICAL TECHNOLOGY, 3ème édition, volume 21, pages 492-507, Wiley Interscience, 1983 ».

Les proportions relatives d'amylose et d'amylopectine, ainsi que leur degré de polymérisation, varient en fonction de l'origine botanique des amidons. En moyenne, un échantillon d'amidon natif est constitué d'environ 25 % d'amylose et de 75 % d'amylopectine.

Parfois, il y a présence de phytoglycogène (entre 0 % et 20 % de l'amidon), un analogue de l'amylopectine mais ramifié tous les 10 à 15 résidus glucose.

L'amidon peut se présenter sous forme de granules semi-cristallines : l'amylopectine est organisée en feuillets, l'amylose forme une zone amorphe moins bien organisée entre les différents feuillets.

L'amylose s'organise en une hélice droite à six glucoses par tour. Il se dissocie en glucose assimilable sous l'action d'enzymes, les amylases, d'autant plus facilement s'il se trouve sous forme d'amylopectine. En effet, la formation hélicoïdale ne favorise pas l'accessibilité de l'amidon aux enzymes.

Les amidons se présentent généralement sous la forme d'une poudre blanche insoluble dans l'eau froide, dont la taille des particules élémentaires va de 3 à 100 microns.

En le traitant par l'eau chaude, on obtient l'empois. Il est exploité dans l'industrie pour ses propriétés d'épaississant et de gélifiant.

Les molécules d'amidons utilisés dans la présente invention peuvent avoir comme origine botanique les céréales ou encore les tubercules. Ainsi, les amidons sont par exemple choisis parmi les amidons de maïs, de riz, de manioc, de tapioca, d'orge, de pomme de terre, de blé, de sorgho, de pois.

Les amidons natifs sont représentés par exemple par les produits vendus sous les dénominations C*AmilogelTM, Cargill GelTM, C* GelTM, Cargill GumTM, DryGelTM, C*Pharm GelTM par la société Cargill, sous la dénomination Amidon de mais par la société Roquette, et sous la dénomination Tapioca Pure par la société National Starch.

Amidons modifiés

Les amidons modifiés utilisés dans la composition de l'invention peuvent être modifiés par une ou plusieurs des réactions suivantes : pré-gélatinisation, dégradation (hydrolyse acide, oxydation, dextrinisation), substitution (estérification, éthérification), réticulation (estérification), blanchiment.

De manière plus particulière, ces réactions peuvent être réalisées de la façon suivante :

- pré-gélatinisation en faisant éclater les granules d'amidon (par exemple séchage et cuisson dans un tambour sécheur) ;
- hydrolyse acide engendrant une rétrogradation très rapide au refroidissement ;
- oxydation par des oxydants forts (milieu alcalin, en présence d'hypochlorite de sodium NaOCl par exemple) conduisant à la dépolymérisation de la molécule d'amidon et à l'introduction de groupes carboxyle dans la molécule d'amidon (principalement oxydation du groupe hydroxyle en $C_6$);
- dextrinisation en milieu acide à haute température (hydrolyse puis repolymérisation) ;
- réticulation par des agents fonctionnels capables de réagir avec les groupes hydroxyles des molécules d'amidon qui vont ainsi être liées entre elles (par exemple avec des groupes glyceryl et/ou phosphate) ;
- estérification en milieu alcalin pour le greffage de groupes fonctionnels, notamment acyl en $C_1$-$C_6$ (acétyl), hydroxyalkylés en $C_1$-$C_6$ (hydroxyéthyl, hydroxypropyl), carboxyméthyl, octénylsuccinique.

On peut notamment obtenir par réticulation avec des composés phosphorés, des phosphates de monoamidon (du type AM-O-PO-$(OX)_2$), des phosphates de diamidon (du type Am-O-PO-(OX)-O-Am) ou même de triamidon (du type Am-O-PO- $(O\text{-}Am)_2$) ou leurs mélanges.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

Selon l'invention, on peut aussi utiliser des amidons amphotères, ces amidons amphotères contiennent un ou plusieurs groupements anioniques et un ou plusieurs groupements cationiques. Les groupements anioniques et cationiques peuvent être liés au même site réactif de la molécule d'amidon ou à des sites réactifs différents, de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes :

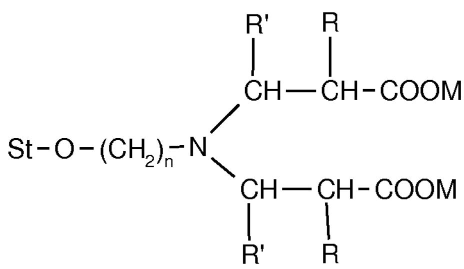

(I)

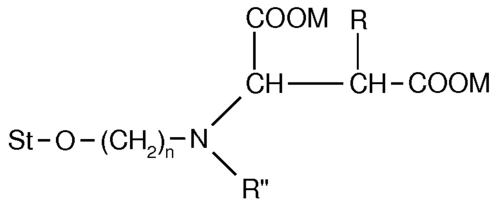

(II)

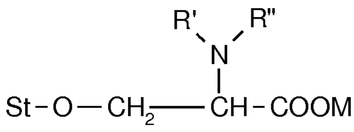

(III)

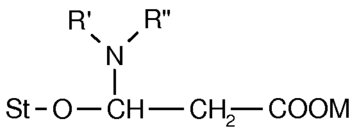

(IV)

dans lesquelles :

- St-O représente une molécule d'amidon ;
- R, identique ou différent, représente un atome d'hydrogène ou un radical méthyle ;
- R', identique ou différent, représente un atome d'hydrogène, un radical méthyle ou un groupement -COOH ;
- n est un entier égal à 2 ou 3 ;
- M, identique ou différent, désigne un atome d'hydrogène, un métal alcalin ou alcalinoterreux tels que Na, K, Li, $NH_4$, un ammonium quaternaire ou une amine organique ;
- R" représente un atome d'hydrogène ou un radical alkyle ayant de 1 à 6 atomes de carbone.

Ces composés sont notamment décrits dans les brevets US 5,455,340 et US 4,017,460.

Les molécules d'amidon peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus.

Les amidons modifiés sont représentés par exemple par les produits vendus sous les dénominations C*Tex-Instant (adipate pré-gélatinisé), C*StabiTex-Instant (phosphate pré-gélatinisé), C*PolarTex-Instant (hydroxypropylé pré-gélatinisé), C*Set (hydrolyse acide, oxydation), C*size (oxydation), C*BatterCrisp (oxydation), C*DrySet (dextrinisation), C*TexTM (adipate de diamidon acetylé), C*PolarTexTM (phosphate de diamidon hydroxypropylé), C* StabiTexTM (phosphate de diamidon, phosphate de diamidon acetylé) par la société Cargill, par les phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

A titre d'exemples d'amidons oxydés, on utilisera notamment ceux commercialisés sous la dénomination C*size de la société Cargill.

Les amidons natifs ou modifiés décrits ci-dessus, peuvent avantageusement être mis en œuvre à raison de 0,1 % à 8 % en poids en matière sèche, et de préférence à environ 1 % en poids, par rapport au poids total de la phase aqueuse.

<u>Amidons particulaires</u>

A titre d'amidons particulaires peuvent être en particulier cités :

- les amidons greffés par un polymère acrylique (homopolymère ou copolymère) et notamment par le polyacrylate de sodium, tels que par exemple ceux commercialisés sous la dénomination Sanfresh ST-100MC par la société Sanyo Chemical Industries ou Makimousse 25, Makimousse 12 par la société Daito Kasei (nom INCI Sodium polyacrylate Starch) ;
- les amidons hydrolysés greffés par un polymère acrylique (homopolymère ou copolymère) et notamment le copolymère acryloacrylamide/acrylate de sodium, comme par exemple ceux commercialisées sous les dénominations Water Lock A-240, A-180, B-204, D-223, A-100, C-200, D-223, par la société Grain Processing (nom INCI : Starch/acrylamide/sodium acrylate copolymer) ;

- les polymères à base

d'amidon, de gomme et de dérivé cellulosique, tels que celui contenant de l'amidon et de la carboxyméthylcellulose de sodium, comme par exemple celui commercialisé sous la dénomination Lysorb 220 par la société Lysac.

Peuvent tout particulièrement être cités les carboxyalkyles en ($C_1$-$C_4$) d'amidon, dits encore ci-après « *carboxyalkylamidon* ». Ces composés sont obtenus par greffage de groupements carboxyalkyles sur une ou plusieurs fonctions alcool de l'amidon, notamment par réaction d'amidon et de monochloroacétate de sodium en milieu alcalin.

Les groupements carboxyalkyles sont généralement fixés par l'intermédiaire d'une fonction éther, plus particulièrement sur le carbone 1. Le degré de substitution en motif carboxyalkyle du carboxyalkyle en ($C_1$-$C_4$) d'amidon va de préférence de 0,1 à 1, et plus particulièrement de 0,15 à 0,5. Le degré de substitution est défini selon la présente invention comme étant le nombre moyen de groupements hydroxyles substitués par un groupement ester ou éther par unité mono saccharidique du polysaccharide.

Les carboxyalkylamidons sont avantageusement mis en œuvre sous la forme de sels et notamment de sels de métal alcalin ou alcalinoterreux tels que Na, K, Li, $NH_4$, d'un ammonium quaternaire ou d'une amine organique telle que la mono, di ou triéthanolamine. Les carboxyalkyles en ($C_1$-$C_4$) amidon sont avantageusement dans le cadre de la présente invention des carboxyméthylamidons. Les carboxyméthylamidons comprennent de préférence des motifs de formule suivante :

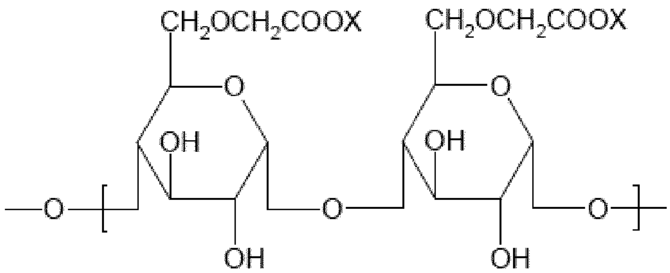

dans laquelle X, lie ou non de manière covalente au motif carboxylique, désigne un atome d'hydrogène, un métal alcalin ou alcalinoterreux tels que Na, K, Li, $NH_4$, un ammonium quaternaire ou une amine organique telle que par exemple telle que la mono, di ou triéthanolamine.

De préférence, X désigne un cation $Na^+$. Les carboxyalkylamidons utilisables selon la présente invention sont de préférence les carboxyalkylamidons non prégélatinisés. Les carboxyalkylamidons utilisables selon la présente invention sont de préférence les carboxyalkylamidons réticulés, partiellement ou totalement.

D'une manière générale, un carboxyalkylamidon réticulé possède par opposition à un carboxyalkylamidon non réticulé, une viscosité augmentée, contrôlable et de stabilité accrue. La réticulation permet ainsi de diminuer les phénomènes de synérèse et d'augmenter la résistance du gel aux effets de cisaillement.

Les carboxyalkylamidons considérés selon l'invention sont plus particulièrement des carboxyalkylamidons de pommes de terre. Ainsi, les carboxyalkylamidons utilisables selon la présente invention sont de préférence des sels de sodium de carboxyalkylamidon, en particulier un sel de sodium de carboxyméthylamidon de pomme de terre vendus notamment sous la dénomination PRIMOJEL® par la société DMV International ou GLYCOLYS® et GLYCOLYS® LV par la Société Roquette.

Selon un mode particulier, on utilisera les carboxyméthylamidons de pomme de terre vendus notamment sous la dénomination GLYCOLYS® par la Société Roquette. Comme précisé précédemment, les particules de carboxyalkyle($C_1$-$C_4$) d'amidon sont présentes dans les compositions selon l'invention sous une forme gonflée et non éclatée. Ce gonflement peut être caractérisé par un pouvoir de gonflement Q qui peut être avantageusement compris entre 10 et 30 ml/g de préférence entre 15 et 25 ml (volume de liquide absorbe)/g de matériau particulaire sec.

Ainsi, la taille des particules gonflées de carboxyalkylamidon mises en œuvre selon la présente invention varie généralement de 25 a 300 $\mu$m. Par exemple, le gel PRIMOJEL® à 10 % en poids de carboxyalkylamidon de pomme de terre et sel de sodium dans l'eau, contient plus de 80 % de particules gonflées de cet amidon ayant un diamètre supérieur à 50 microns, et plus particulièrement supérieur à 100 microns.

Selon une variante de réalisation préférée de l'invention, ces particules sont mises en œuvre pour la préparation des compositions selon l'invention, dans cet état particulaire gonflé. Pour se faire, ces particules sont avantageusement mises en œuvre sous la forme d'un gel aqueux soit préparé préalablement soit déjà disponible commercialement. Les gels considérés selon l'invention sont avantageusement translucides.

Par exemple, un gel de carboxyméthylamidon comme le PRIMOJEL® qui est à une concentration de 10 % en

poids peut être ajusté à la concentration requise avant d'être utilisé pour préparer la composition cosmétique attendue.

Un tel amidon particulaire peut être mis en œuvre à raison de 0,1 % à 5 % en poids en matière sèche par rapport au poids total de la phase aqueuse, de préférence entre 0,5 % et 2,5 % en poids, et en particulier à raison de environ 1,5 % en poids, par rapport au poids total de la phase aqueuse

## GELIFIANT LIPOPHILE

On entend par « *gélifiant lipophile* » au sens de la présente invention, un composé apte à gélifier la phase huileuse des compositions selon l'invention.

On entend par « *gélifiant lipophile non-celullosique»* au sens de la présente invention, un composé apte à gélifier la phase huileuse des compositions selon l'invention et qui ne comporte dans sa structure aucun groupement cellulose de formule chimique :

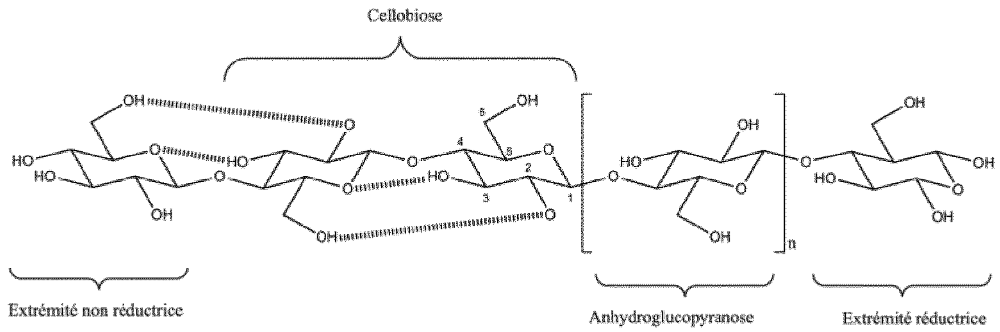


ni aucun autre groupement dérivé de cellulose résultant de la réaction des groupements OH de la cellulose avec des réactifs chimiques tel qu'un éther de cellulose (éthylcellulose), ester de cellulose (carboxymethylcellulose) ou ester-éther de cellulose.

Le gélifiant lipophile est donc présent dans la phase huileuse de la composition.

Le gélifiant est liposoluble ou lipodispersible.

L'agent gélifiant lipophile est choisi parmi les élastomères d'organopolysiloxane, les polymères semi-cristallins, les esters de dextrine, les polyamides hydrocarbonés, les gélifiants particulaires choisis parmi les cires polaires, les cires apolaires hydrocarbonées de point de fusion inférieur ou égal à 75,0 °C, les cires siliconées, les argiles modifiées, les silices, ainsi que leurs mélanges.

### I. Les gélifiants particulaires

Le gélifiant particulaire mis en œuvre dans la composition selon l'invention se présente sous forme de particules, de préférence sphériques.

A titre représentatif des gélifiants particulaires lipophiles convenant à l'invention peuvent être tout particulièrement cité les cires, polaires et apolaires, les argiles modifiées, les silices comme les silices pyrogénées et les aérogels de silice hydrophobes.

Les cires

Les cires sont définies ci-avant.

Les cires susceptibles d'être utilisées dans les compositions selon l'invention sont choisies parmi les cires, solides, à température ambiante d'origine animale, végétale, minérale ou de synthèse et leurs mélanges.

Les cires, au sens de l'invention, peuvent être celles utilisées généralement dans les domaines cosmétiques ou dermatologiques. Elles peuvent notamment être polaires, apolaires hydrocarbonées de point de fusion inférieur ou égal à 75,0 °C, siliconées et/ou fluorées, comportant éventuellement des fonctions ester ou hydroxyle. Elles peuvent être également d'origine naturelle ou synthétique.

a) Cire polaire

Par « *cire polaire* », au sens de la présente invention, on entend une cire dont le paramètre de solubilité à 25 °C $\delta a$ est différent de 0 $(J/cm3)^{½}$.

En particulier, par *« cire polaire »*, on entend une cire dont la structure chimique est formée essentiellement, voire

constituée, d'atomes de carbone et d'hydrogène, et comprenant au moins un hétéroatome fortement électronégatif tel qu'un atome d'oxygène, d'azote, de silicium ou de phosphore.

Les cires polaires peuvent notamment être hydrocarbonées, fluorées ou siliconées.

Préférentiellement, les cires polaires peuvent être hydrocarbonées.

Par « *cire hydrocarbonée* », on entend une cire formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Par « *cire ester* », on entend selon l'invention une cire comprenant au moins une fonction ester. Par « *cire alcool* », on entend selon l'invention une cire comprenant au moins une fonction alcool, c'est-à-dire comprenant au moins un groupe hydroxyle (OH) libre.

On peut notamment utiliser en tant que cire ester :

- les cires esters telles que celles choisies parmi :

  i) les cires de formule $R_1$ $COOR_2$ dans laquelle $R_1$ et $R_2$ représentent des chaines aliphatiques linéaires, ramifiées ou cycliques dont le nombre d'atomes varie de 10 à 50, pouvant contenir un hétéroatome tel que O, N ou P et dont la température de point de fusion varie de 25 à 120 °C.

  ii) le tétrastéarate de di-(triméthylol-1,1,1 propane), vendu sous la dénomination de Hest 2T-4S® par la société Heterene.

  iii) les cires diesters d'un diacide carboxylique de formule générale $R^3$-(-OCO-$R^4$-COO-$R^5$), dans laquelle $R^3$ et $R^5$ sont identiques ou différents, de préférence identiques et représentent un groupe alkyle en $C_4$-$C_{30}$ (groupe alkyle comprenant de 4 à 30 atomes de carbone) et $R_4$ représente un groupe aliphatique en $C_4$-$C_{30}$ (groupe alkyle comprenant de 4 à 30 atomes de carbone) linéaire ramifié pouvant contenir ou non une ou plusieurs insaturation(s), et de préférence linéaire et insaturé.

  iv) On peut aussi citer les cires obtenues par hydrogénation catalytique d'huiles animales ou végétales ayant des chaînes grasses, linéaires ou ramifiées, en $C_8$-$C_{32}$, par exemple telles que l'huile de jojoba hydrogénée, l'huile de tournesol hydrogénée, l'huile de ricin hydrogénée, l'huile de coprah hydrogénée, ainsi que les cires obtenues par hydrogénation d'huile de ricin estérifiée avec l'alcool cétylique.

  v) la cire d'abeille, la cire d'abeille synthétique, la cire d'abeille polyglycérolée, la cire de carnauba, la cire de candellila, la cire de lanoline oxypropylénée, la cire de son de riz, la cire d'Ouricury, la cire d'Alfa, la cire de fibres de liège, la cire de canne à sucre, la cire du Japon, la cire de sumac, la cire de montan, la cire d'Orange, la cire de Laurier, la cire de Jojoba hydrogénée, la cire de tournesol, cire de citron, cire d'olive, cire du berry.

Selon un autre mode de réalisation, la cire polaire peut être une cire alcool. Par *« cire alcool »*, on entend selon l'invention une cire comprenant au moins une fonction alcool, c'est-à-dire comprenant au moins un groupe hydroxyle (OH) libre. A titre de cire alcool, on peut citer par exemple la cire $C_{30-50}$ Alcools Performacol® 550 Alcohol commercialisé par la société New Phase Technologie, l'alcool stéarique, l'alcool cétylique.

b) <u>Cires siliconées</u>

On peut aussi utiliser des cires siliconées qui peuvent être avantageusement des polysiloxanes substitués, de préférence à bas point de fusion.

Par « *cire siliconée* », on entend une huile comprenant au moins un atome de silicium, et notamment comprenant des groupes Si-O.

Parmi les cires de silicones commerciales de ce type, on peut citer notamment celles vendues sous les dénominations Abilwax 9800, 9801 ou 9810 (Goldschmidt), KF910 et KF7002 (Shin Etsu), ou 176-1118-3 et 176-11481 (General Electric).

Les cires de silicone utilisables peuvent également être des alkyl ou alcoxydiméthicones, ainsi que les ($C_{20}$-$C_{60}$) alkyldiméthicones, en particulier les ($C_{30}$-$C_{45}$)alkyldiméthicones comme la cire siliconée vendue sous la dénomination SF-1642 par la société GE-Bayer Silicones ou la $C_{30-45}$ Alkyldiméthylsilyl Polypropylsilsesquioxane sous la dénomination SW-8005® C30 Resin Wax commercialisé par la société Dow Corning.

Les cires, au sens de l'invention, peuvent être celles utilisées généralement dans les domaines cosmétiques ou dermatologiques.

Dans le cadre de la présente invention, on peut citer à titre de cires particulièrement avantageuses la cire de Carnauba, les cires de polyéthylène, la cire de jojoba, la cire de candelilla et les cires siliconées, en particulier la cire de candelilla.

Elles peuvent être présentes dans la phase huileuse à raison de 0,5 % à 30 % en poids par rapport au poids de la phase huileuse, par exemple entre 5 % et 20 % de la phase huileuse, et plus particulièrement de 2 % à 15 % en poids par

rapport au poids de la phase huileuse.

Les argiles modifiées

La composition selon l'invention peut comprendre au moins une argile lipophile.
Les argiles peuvent être naturelles ou synthétiques et elles sont rendues lipophiles par un traitement avec un sel d'alkyl ammonium comme un chlorure d'ammonium en $C_{10}$ à $C_{22}$, par exemple le chlorure de di-stéaryl di-méthyl ammonium.
Elles peuvent être choisies parmi les bentonites en particulier les hectorites et les montmorillonites, les beidellites, les saponites, les nontronites, les sépiolites, les biotites, les attapulgites, les vermiculites et les zéolites.
De préférence, elles sont choisies parmi les hectorites.
De préférence, on utilise à titre d'argiles lipophiles les hectorites modifiées par un sel d'ammonium en $C_{10}$ à $C_{22}$, de préférence un chlorure d'ammonium en $C_{10}$ à $C_{22}$, comme l'hectorite modifiée par du chlorure de di-stéaryl di-méthyl ammonium telle que, par exemple, celle commercialisée sous la dénomination de Bentone 38V® par la société Elementis ou le gel de bentone dans isododécane commercialisé sous la dénomination Bentone Gel ISD V® (Isododécane 87 %/Disteardimonium Hectorite 10 %/Propylène carbonate 3 %) par la société Elementis.
Avantageusement on utilise les hectorites modifiées par un seld'ammonium en $C_{10}$ à $C_{22}$, en particulier les hectorites modifiées par un chlorure d'ammonium en $C_{10}$ à $C_{22}$.
L'argile lipophile peut notamment être présente en une teneur allant de 0,1 % à 25 % en poids, en particulier de 0,5 % à 20 %, plus particulièrement de 1 % à 18 % en poids par rapport au poids total de la phase huileuse.

Les silices

La phase huileuse d'une composition selon l'invention peut également comprendre à titre de gélifiant une silice pyrogénée ou des particules d'aérogel de silice.

a) Silice pyrogénée

Convient tout particulièrement à l'invention, la silice pyrogénée traitée hydrophobe en surface. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe.
Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812® par la société Degussa, CAB-O-SIL TS-530® par la société Cabot.
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972®, et Aerosil R974® par la société Degussa, CAB-O-SIL TS-610® et CAB-O-SIL TS-720® par la société Cabot.

Les silices pyrogénées peuvent être présentes dans une composition selon la présente invention à raison d'une teneur comprise entre 0,1 % et 40 % en poids, plus particulièrement entre 1 % et 15 % en poids et encore plus particulièrement entre 2 % et 10 % en poids, par rapport au poids total de la phase huileuse.

b) Aérogels de silice hydrophobes

Selon une variante particulièrement préférée, la phase huileuse d'une composition selon l'invention comprend à titre de gélifiant au moins des particules d'aérogels de silice.
Les aérogels de silice sont des matériaux poreux obtenus en remplaçant (par séchage) la composante liquide d'un gel de silice par de l'air.
Ils sont généralement synthétisés par procédé sol-gel en milieu liquide puis séchés usuellement par extraction d'un fluide supercritique, le plus communément utilisé étant le $CO_2$ supercritique. Ce type de séchage permet d'éviter la contraction des pores et du matériau. Le procédé sol-gel et les différents séchages sont décrits en détail dans Brinker CJ., and Scherer G.W., Sol-Gel Science : New York : Academic Press, 1990.

Les particules d'aérogels de silice hydrophobe utilisées dans la présente invention présentent une surface spécifique par unité de masse (SM) allant de 500 à 1500 $m^2/g$, de préférence de 600 à 1200 $m^2/g$ et mieux de 600 à 800 $m^2/g$, et une taille exprimée en diamètre moyen en volume (D[0,5]) allant de 1 à 1500 μm, mieux de 1 à 1000 μm, de préférence de 1 à 100 μm, en particulier de 1 à 30 μm, de préférence encore de 5 à 25 μm, mieux de 5 à 20 μm et encore mieux de mieux de 5 à 15 μm.

Selon un mode de réalisation, les particules d'aérogels de silice hydrophobe utilisées dans la présente invention présentent une taille exprimée en diamètre moyen en volume (D[0,5]) allant de 1 à 30 μm, de préférence de 5 à 25 μm, mieux de 5 à 20 μm et encore mieux de mieux de 5 à 15 μm.

La surface spécifique par unité de masse peut être déterminée par la méthode d'absorption d'azote appelée méthode BET (Brunauer - Emmet - Teller) décrite dans « The journal of the American Chemical Society », vol. 60, page 309, février 1938 et correspondant à la norme internationale ISO 5794/1 (annexe D). La surface spécifique BET correspond à la surface spécifique totale des particules considérées.

Les tailles des particules d'aérogel de silice peuvent être mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., « Light Scattering by Small Particles », Chapitres 9 et 10, Wiley, New York, 1957.

Selon un mode de réalisation avantageux, les particules d'aérogels de silice hydrophobe utilisées dans la présente invention présentent une surface spécifique par unité de masse (SM) allant de 600 à 800 $m^2/g$.

Les particules d'aérogel de silice utilisées dans la présente invention peuvent avantageusement présenter une densité tassée $\rho$ allant de 0,02 $g/cm^3$ à 0,10 $g/cm^3$, de préférence de 0,03 $g/cm^3$ à 0,08 $g/cm^3$, en particulier allant de 0,05 $g/cm^3$ à 0,08 $g/cm^3$.

Dans le cadre de la présente invention, cette densité peut être appréciée selon le protocole suivant, dit de la densité tassée :

On verse 40 g de poudre dans une éprouvette graduée; puis on place l'éprouvette sur l'appareil STAV 2003 de chez Stampf Volumeter ; l'éprouvette est ensuite soumise à une série de 2500 tassements (cette opération est recommencée jusqu'à ce que la différence de volume entre 2 essais consécutifs soit inférieure à 2 %) ; puis on mesure directement sur l'éprouvette le volume final Vf de poudre tassée. La densité tassée est déterminée par le rapport m/Vf, en l'occurrence 40/Vf (Vf étant exprimé en $cm^3$ et m en g).

Selon un mode de réalisation préféré, les particules d'aérogels de silice hydrophobe utilisées dans la présente invention présentent une surface spécifique par unité de volume SV allant de 5 à 60 $m^2/cm^3$, de préférence de 10 à 50 $m^2/cm^3$ et mieux de 15 à 40 $m^2/cm^3$.

La surface spécifique par unité de volume est donnée par la relation : $Sv = S_M \times \rho$ ; où $\rho$ est la densité tassées exprimée en $g/cm^3$ et SM est la surface spécifique par unité de masse exprimée en $m^2/g$, telles que définie plus haut.

De préférence, les particules d'aérogels de silice hydrophobe selon l'invention ont une capacité d'absorption d'huile mesurée au Wet Point allant de 5 à 18 ml/g, de préférence de 6 à 15 ml/g et mieux de 8 à 12 ml/g.

La capacité d'absorption mesurée au Wet Point, et notée Wp, correspond à la quantité d'huile qu'il faut additionner à 100 g de particules pour obtenir une pâte homogène.

Elle est mesurée selon la méthode dite de Wet Point ou méthode de détermination de prise d'huile de poudre décrite dans la norme NF T 30-022. Elle correspond à la quantité d'huile adsorbée sur la surface disponible de la poudre et/ou absorbée par la poudre par mesure du Wet Point, décrite ci-dessous :

On place une quantité m = 2 g de poudre sur une plaque de verre puis on ajoute goutte à goutte l'huile (isononyl isononanoate). Après addition de 4 à 5 gouttes d'huile dans la poudre, on mélange à l'aide d'une spatule et on continue d'ajouter de l'huile jusqu'à la formation de conglomérats d'huile et de poudre. A partir de ce moment, on ajoute l'huile à raison d'une goutte à la fois et on triture ensuite le mélange avec la spatule. On cesse l'addition d'huile lorsque l'on obtient une pâte ferme et lisse. Cette pâte doit se laisser étendre sur la plaque de verre sans craquelures ni formation de grumeaux. On note alors le volume Vs (exprimé en ml) d'huile utilisé.

La prise d'huile correspond au rapport Vs/m.

Les aérogels utilisés selon la présente invention sont des aérogels de silice hydrophobe, de préférence de silice silylée (nom INCI : silica silylate).

Par « *silice hydrophobe* », on entend toute silice dont la surface est traitée par des agents de silylation, par exemple par des silanes halogénés tels que des alkylchlorosilanes, des siloxanes, en particulier des dimethylsiloxanes tel que l'hexamethyldisiloxane, ou des silazanes, de manière à fonctionnaliser les groupements OH par des groupements silyles Si-Rn, par exemple des groupements triméthylsilyles.

Concernant la préparation de particules d'aérogels de silice hydrophobe modifiés en surface par silylation, on peut se référer au document US 7,470,725.

On utilisera de préférence des particules d'aérogels de silice hydrophobe modifiée en surface par groupements triméthylsilyles, de préférence de nom INCI Silica silylate.

A titre de d'aérogels de silice hydrophobe utilisables dans l'invention, on peut citer par exemple l'aérogel commercialisé sous la dénomination VM-2260 ou VM-2270 (nom INCI : Silica silylate), par la société Dow Corning, dont les particules présentent une taille moyenne d'environ 1000 microns et une surface spécifique par unité de masse allant de 600 à 800 $m^2/g$.

On peut également citer les aérogels commercialisés par la société Cabot sous les références Aerogel TLD 201, Aerogel OGD 201, Aerogel TLD 203, ENOVA® Aerogel MT 1100, ENOVA Aerogel MT 1200.

On utilisera de préférence l'aérogel commercialisé sous la dénomination VM-2270 (nom INCI Silica silylate), par la société Dow Corning, dont les particules présentent une taille moyenne allant de 5-15 microns et une surface spécifique par unité de masse allant de 600 à 800 $m^2/g$.

Un tel aérogel permet avantageusement de favoriser la résistance du dépôt au sébum et à la sueur.

De façon préférée, les particules d'aérogels de silice hydrophobe sont présentes dans la composition selon l'invention en une teneur en matière sèche allant de 0,1 % à 30 % en poids, de préférence de 0,2 % à 20 % en poids, de préférence de 0,2 % à 10 % en poids par rapport au poids total de la phase huileuse.

## II. Elastomère d'organopolysiloxane

Selon une autre variante particulièrement préférée, la phase huileuse d'une composition selon l'invention comprend à titre de gélifiant au moins un élastomère d'organopolysiloxane.

L'élastomère d'organopolysiloxane, utilisable à titre de gélifiant lipophile, a pour avantage de conférer à la composition selon l'invention de bonnes propriétés d'application. Il procure un toucher très doux et matifiant après l'application, avantageux notamment pour une application sur la peau. Il peut également permettre un comblement efficace des creux présents sur les matières kératiniques.

Par « *élastomère d'organopolysiloxane* » ou « *élastomère de silicone* », on entend un organopolysiloxane souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement.

Il s'agit plus particulièrement d'un élastomère d'organopolysiloxane réticulé.

Ainsi, l'élastomère d'organopolysiloxane peut être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'élastomère d'organopolysiloxane est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine, comme par exemple décrit dans la demande EP-A-295886.

En particulier, l'élastomère d'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins deux atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles tels que méthyle, éthyle, propyle, butyle, octyle ; des groupes alkyles substitués tels que 2-phényléthyl, 2-phénylpropyl, 3,3,3-trifluoropropyl ; des groupes aryles tels que phényle, tolyle, xylyle ; des groupes aryles substitués tels que phényléthyl ; et des groupes hydrocarbonés monovalents substitués tels qu'un groupe époxy, un groupe ester carboxylate, ou un groupe mercapto.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques

diméthylsiloxane-méthylhydrogénosiloxane.

Le composé (B) est avantageusement un diorganopolysiloxane ayant au moins deux groupes alkényles inférieurs (par exemple en $C_2$-$C_4$) ; le groupe alkényle inférieur peut être choisi parmi les groupes vinyle, allyle, et propényle. Ces groupements alkényles inférieurs peuvent être situés en toute position de la molécule organopolysiloxane mais sont de préférence situés aux extrémités de la molécule organopolysiloxane. L'organopolysiloxane (B) peut avoir une structure à chaîne ramifiée, à chaîne linéaire, cyclique ou en réseau mais la structure en chaîne linéaire est préférée. Le composé (B) peut avoir une viscosité allant de l'état liquide à l'état de gomme. De préférence, le composé (B) a une viscosité d'au moins 100 centistokes à 25 °C.

Outre les groupes alkényle précités, les autres groupes organiques liés aux atomes de silicium dans le composé (B) peuvent être des groupes alkyles tels que méthyle, éthyle, propyle, butyle ou octyle ; des groupes alkyles substitués tels que 2-phényléthyle, 2-phénylpropyle ou 3,3,3-trifluoropropyle ; des groupes aryles tels que phényl, tolyl ou xylyl ; des groupes aryles substitués tels que phényléthyle ; et des groupes hydrocarbonés monovalents substitués tels qu'un groupe époxy, un groupe ester carboxylate, ou un groupe mercapto.

Les organopolysiloxanes (B) peuvent être choisis parmi les méthylvinylpolysiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)-polysiloxane à terminaisons diméthylvinylsiloxy, et les copolymères diméthylsiloxane-méthyl(3,3,3-trifluoropropyl)siloxane à terminaisons diméthylvinylsiloxy.

En particulier, l'organopolysiloxane élastomère peut être obtenu par réaction de diméthylpolysiloxane à terminaisons diméthylvinylsiloxy et de méthylhydrogénopolysiloxane à terminaisons triméthylsiloxy, en présence de catalyseur platine.

Avantageusement, la somme du nombre de groupements éthyléniques par molécule du composé (B) et du nombre d'atomes d'hydrogène liés à des atomes de silicium par molécule du composé (A) est d'au moins 5.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1,5/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère est avantageusement un élastomère non émulsionnant.

Le terme « *non émulsionnant* » définit des élastomères d'organopolysiloxane ne contenant pas de chaîne hydrophile, et en particulier ne contenant pas de motifs polyoxyalkylène (notamment polyoxyéthylène ou polyoxypropylène), ni de motif polyglycéryle. Ainsi, selon un mode particulier de l'invention, la composition comprend un élastomère d'organopolysiloxane dénué de motifs polyoxyalkylène et de motif polyglycéryle.

En particulier, l'élastomère de silicone utilisé dans la présente invention est choisi parmi des Dimethicone Crosspolymer (Nom INCI), Vinyl Dimethicone Crosspolymer (Nom INCI), Dimethicone/Vinyl Dimethicone Crosspolymer (Nom INCI), Dimethicone Crosspolymer-3 (Nom INCI).

Les particules d'élastomères d'organopolysiloxane peuvent être véhiculées sous forme d'un gel constitué d'un organopolysiloxane élastomérique inclus dans au moins une huile hydrocarbonée et/ou une huile siliconée. Dans ces gels, les particules d'organopolysiloxanes sont souvent des particules non-sphériques.

Des élastomères non émulsionnants sont notamment décrits dans les brevets EP 242 219, EP 285 886, EP 765 656 et dans la demande JP-A-61-194009.

L'élastomère de silicone se présente généralement sous la forme d'un gel, d'une pâte ou d'une poudre mais avantageusement sous la forme d'un gel dans lequel l'élastomère de silicone est dispersé dans une huile siliconée linéaire (dimethicone) ou cyclique (ex : cyclopentasiloxane), avantageusement dans une huile siliconée linéaire.

Comme élastomères non émulsionnants, on peut plus particulièrement utiliser ceux vendus sous les dénominations « KSG-6 », « KSG-15 », « KSG-16 », « KSG-18 », « KSG-41 », « KSG-42 », « KSG-43 », « KSG-44 », par la société Shin Etsu, « DC9040 », « DC9041 », par la société Dow Corning, « SFE 839 » par la société General Electric.

Selon un mode particulier, on utilise un gel d'élastomère de silicone dispersé dans une huile siliconée choisie parmi une liste non exhaustive comprenant la cyclopentadimethylsiloxane, les dimethicones, les dimethylsiloxanes, la methyl trimethicone, phenylmethicone, phenyldimethicone, phenyltrimethicone, et la cyclomethicone, de préférence une huile siliconée linéaire choisie parmi les polydiméthylsiloxanes (PDMS) ou dimethicones de viscosité à 25 °C allant de 1 à 500 est à 25 °C, éventuellement modifiées par des groupements aliphatiques, éventuellement fluorés, ou par des groupements fonctionnels tels que des groupements hydroxyles, thiols et/ou aminés.

On peut citer notamment les composés de nom INCI suivants :

- Dimethicone/Vinyl Dimethicone Crosspolymer, tels que «USG-105» et « USG-107A » de la société Shin Etsu ; « DC9506 » et « DC9701 » de la société Dow Corning,
- Dimethicone/Vinyl Dimethicone Crosspolymer (and) Dimethicone, tels que « KSG-6 » et « KSG-16 » de la société Shin Etsu ;
- Dimethicone/Vinyl Dimethicone Crosspolymer (and) Cyclopentasiloxane, tels que « KSG-15 » ;
- Cyclopentasiloxane (and) Dimethicone Crosspolymer, tels que « DC9040 », « DC9045 » et « DC5930 » de la société Dow Corning ;
- Dimethicone (and) Dimethicone Crosspolymer, tels que « DC9041 » de la société Dow Corning ;
- Dimethicone (and) Dimethicone Crosspolymer, tels que « Dow Corning EL-9240® silicone elastomer blend » de la société Dow Corning (mélange de polydiméthylsiloxane reticulé avec hexadiène/polydiméthysiloxane (2 cSt)) ;
- $C_{4}$-$_{24}$ Alkyl Dimethicone/DivinylDimethicone Crosspolymer, tels que NuLastic Silk MA par la société Alzo.

Comme exemples d'élastomères de silicone dispersés dans une huile de silicone linéaire utilisables avantageusement selon l'invention, on peut citer notamment les références suivantes :

- Dimethicone/Vinyl Dimethicone Crosspolymer (and) Dimethicone, tels que « KSG-6 » et « KSG-16 » de la société Shin Etsu ;
- Dimethicone (and) Dimethicone Crosspolymer, tels que « DC9041 » de la société Dow Corning ; et
- Dimethicone (and) Dimethicone Crosspolymer, tels que « Dow Corning EL-9240® silicone elastomer blend » de la société Dow Corning (mélange de polydiméthylsiloxane reticulé par Hexadiène/Polydiméthysiloxane (2 cSt)).
- DIPHENYLSILOXY PHENYL TRIMETHICONE (and) DIMETHICONE (and) PHENYL VINYL DIMETHICONE CROSSPOLYMER (nom INCI) tels que KSG 18A commercialisé par la société SHIN ETSU).

Les particules d'élastomères d'organopolysiloxane peuvent également être utilisées sous forme de poudre, on peut notamment citer les poudres vendues sous les dénominations « Dow Corning 9505 Powder », « Dow Corning 9506 Powder » par la société Dow Corning, ces poudres ont pour nom INCI : dimethicone/vinyl dimethicone crosspolymer.

La poudre d'organopolysiloxane peut également être enrobée de résine silsesquioxane, comme décrit par exemple dans le brevet US 5,538,793. De telles poudres d'élastomères sont vendues sous les dénominations « KSP-100 », « KSP-101 », « KSP-102 », « KSP-103 », « KSP-104 », « KSP-105 » par la société Shin Etsu, et ont pour nom INCI : vinyl dimethicone/methicone silsesquioxane Crosspolymer.

Comme exemples de poudres d'organopolysiloxane enrobées de résine silsesquioxane utilisables avantageusement selon l'invention, on peut citer notamment les élastomères d'organopolysiloxane de nom INCI VINYL DIMETHICONE / METHICONE SILSESQUIOANE CROSSPOLYMER comme ceux vendus sous la référence commerciale « KSP-100 » de la société Shin Etsu.

A titre d'agent gélifiant lipophile préféré de type élastomère d'organopolysiloxane, on peut notamment mentionner les élastomères d'organopolysiloxane réticulé choisi parmi les Dimethicone Crosspolymer (nom INCI), Vinyl Dimethicone Crosspolymer (nom INCI), Dimethicone/Vinyl Dimethicone Crosspolymer (nom INCI), Dimethicone Crosspolymer-3 (nom INCI), VINYL DIMETHICONE / METHICONE SILSESQUIOANE CROSSPOLYMER, PHENYL VINYL DIMETHICONE CROSSPOLYMER (nom INCI) et en particulier les Dimethicone Crosspolymer (nom INCI).

A titre d'agent gélifiant lipophile préféré de type élastomère d'organopolysiloxane, on peut notamment mentionner les produits choisi parmi les Dimethicone Crosspolymer (nom INCI), Dimethicone (and) Dimethicone Crosspolymer (nom INCI), Vinyl Dimethicone Crosspolymer (nom INCI), Dimethicone/Vinyl Dimethicone Crosspolymer (nom INCI), Dimethicone Crosspolymer-3 (nom INCI), VINYL DIMETHICONE / METHICONE SILSESQUIOANE CROSSPOLYMER, DIPHENYLSILOXY PHENYL TRIMETHICONE (and) DIMETHICONE (and) PHENYL VINYL DIMETHICONE CROSSPOLYMER (nom INCI) et en particulier les Dimethicone Crosspolymer (nom INCI).

L'élastomère d'organopolysiloxane peut être présent dans une composition de la présente invention à raison d'une teneur comprise entre 0,1 % et 70 % en poids en matière sèche notamment entre 0,2 % et 60 % en poids, avantageusement entre 0.5% et 40% et de façon prérérée de 1% à 20% en poids par rapport au poids total de la phase huileuse.

## III. Polymères semi-cristallins

La composition selon l'invention peut comprendre au moins un polymère semi-cristallin. De préférence, le polymère semi-cristallin a une structure organique, et une température de fusion supérieure ou égale à 30 °C.

Par « *polymère semi-cristallin* », on entend au sens de l'invention, des polymères comportant une partie cristallisable et une partie amorphe et présentant une température de changement de phase réversible du premier ordre,

en particulier de fusion (transition solide-liquide). La partie cristallisable est soit une chaîne latérale (ou chaîne pendante), soit une séquence dans le squelette.

Lorsque la partie cristallisable du polymère semi-cristallin est une séquence du squelette polymérique, cette séquence cristallisable est de nature chimique différente de celle des séquences amorphes; le polymère semi-cristallin est dans ce cas un copolymère séquencé par exemple du type dibloc, tribloc ou multibloc. Lorsque la partie cristallisable est une chaîne pendante au squelette, le polymère semi cristallin peut être un homopolymère ou un copolymère.

La température de fusion du polymère semi-cristallin est de préférence inférieure à 150 °C.

La température de fusion du polymère semi-cristallin est de préférence supérieure ou égale à 30 °C et inférieure à 100 °C. De préférence encore, la température de fusion du polymère semi-cristallin est supérieure ou égale à 30 °C et inférieure à 70 °C.

Le ou les polymères semi-cristallins selon l'invention servant sont des solides à température ambiante (25 °C) et pression atmosphérique (760 mm de Hg), dont la température de fusion est supérieure ou égale à 30 °C. Les valeurs de point de fusion correspondent au point de fusion mesuré à l'aide d'un calorimètre à balayage différentiel (DSC), tel que le calorimètre vendu sous la dénomination DSC 30 par la société Mettler, avec une montée en température de 5 ou 10 °C par minute (Le point de fusion considéré est le point correspondant à la température du pic le plus endotherme du thermogramme).

Le ou les polymères semi-cristallins selon l'invention ont de préférence une température de fusion supérieure à la température du support kératinique destiné à recevoir ladite composition, en particulier la peau, les lèvres.

Selon l'invention les polymères semi-cristallins sont avantageusement solubles dans la phase grasse, notamment à au moins 1 % en poids, à une température supérieure à leur température de fusion. En dehors des chaînes ou séquences cristallisables, les séquences des polymères sont amorphes.

Par « *chaîne ou séquence cristallisable* », on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère.

De préférence, le squelette polymérique des polymères semi-cristallins est soluble dans la phase grasse à une température supérieure à leur température de fusion.

De préférence, les séquences ou chaînes cristallisables des polymères semi-cristallins représentent au moins 30 % du poids total de chaque polymère et mieux au moins 40 %. Les polymères semi-cristallins à chaînes latérales cristallisables sont des homo- ou des co-polymères. Les polymères semi-cristallins de l'invention à séquences cristallisables sont des copolymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique.

Selon un mode de réalisation préféré, le polymère semi-cristallin est choisi parmi :

- les homopolymères et copolymères comportant des motifs résultant de la polymérisation de un ou plusieurs monomères porteurs de chaîne(s) latérale(s) hydrophobe(s) cristallisable(s),
- les polymères portant dans le squelette au moins une séquence cristallisable,
- les polycondensats de type polyester, aliphatique ou aromatique ou aliphatique/aromatique,
- les copolymères d'éthylène et de propylène préparés par catalyse métallocène, et
- les copolymères acrylates/silicone.

Les polymères semi-cristallins utilisables dans l'invention peuvent être choisis en particulier parmi :

- les copolymères séquencés de polyoléfines à cristallisation contrôlée, dont les monomères sont décrits dans EP 0 951 897,
- les polycondensats et notamment de type polyester, aliphatique ou aromatique ou aliphatique/aromatique,
- les copolymères d'éthylène et de propylène préparés par catalyse métallocène,
- les homo- ou co-polymères portant au moins une chaîne latérale cristallisable et les homo- ou co-polymères portant dans le squelette au moins une séquence cristallisable, comme ceux décrits dans le document US 5,156,911, tels que les ($C_{10}$-$C_{30}$)alkyle polyacrylates correspondant aux Intelimer® de la société Landec décrits dans la brochure « Intelimer® polymers », Landec IP22 (Rev. 4-97) et par exemple le produit Intelimer® IPA 13-1 de la société Landec, qui est un polyacrylate de stéaryle de poids moléculaire d'environ 145 000 et dont la température de fusion est égale à 49 °C,
- les homo- ou co-polymères portant au moins une chaîne latérale cristallisable en particulier à groupement(s) fluoré(s), tels que décrits dans le document WO 01/19333,

- les copolymères acrylates/silicone, tels que les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane. On peut citer en particulier les copolymères commercialisés par la société SHIN-ETSU sous les dénominations KP-561 (nom CTFA : acrylates/dimethicone), KP-541 (nom CTFA : acrylates/dimethicone and Isopropyl alcohol), KP-545 (nom CTFA : acrylates/dimethicone and Cyclopentasiloxane),
- et leurs mélanges.

De préférence, la quantité de polymère(s) semi-cristallin(s), de préférence choisi parmi les polymères semi cristallins à chaînes latérales cristallisables représente de 0,1 % à 30 % en poids en matière sèche par rapport au poids total de la phase huileuse, par exemple de 0,2 % à 25 % en poids, mieux de 0,5 % à 20 %, ou encore de 0,5 % à 12 % en poids, par rapport au poids total de la phase huileuse.

## IV. Esters de dextrine

La composition selon l'invention peut comprendre à titre de gélifiant lipophile au moins un ester de dextrine.
En particulier, la composition comprend de préférence au moins un ester de dextrine et d'acide gras, de préférence en $C_{12}$ à $C_{24}$, en particulier en $C_{14}$ à $C_{18}$, ou leurs mélanges.
De préférence, l'ester de dextrine est un ester de dextrine et d'acide gras en $C_{12}$-$C_{18}$, en particulier en $C_{14}$-$C_{18}$.
De préférence, l'ester de dextrine est choisi parmi le myristate de dextrine et/ou le palmitate de dextrine, et leurs mélanges.
Selon un mode de réalisation particulier, l'ester de dextrine est le myristate de dextrine, tel que celui notamment commercialisé sous le nom de Rheopearl MKL-2 par la société Chiba Flour Milling.
Selon un mode de réalisation préféré, l'ester de dextrine est le palmitate de dextrine. Celui-ci peut par exemple être choisi parmi ceux commercialisés sous les dénominations Rheopearl TL® ou Rheopearl KL® ou Rheopearl® KL2 par la société Chiba Flour Milling.
De façon particulièrement préférée, la phase huileuse d'une composition selon l'invention peut comprendre de 0,1 % à 30 % en poids d'ester(s) de dextrine, de préférence de 0,2 % à 25 % et de préférence de 0,5 % à 18 % en poids, par rapport au poids total de la phase huileuse.

## V. Les polymères à liaison hydrogène

A titre représentatif des polymères à liaison hydrogène convenant à l'invention peuvent être tout particulièrement cité les polyamides hydrocarbonés.
La phase huileuse d'une composition selon l'invention peut comprendre au moins un polyamide hydrocarboné.
De façon préférée, la teneur totale en polyamide(s) hydrocarboné (s) est comprise entre 0,1 % et 30 % en poids exprimé en matière sèche, de préférence entre 1 % et 20 % en poids, de préférence entre 1 % et 12 % en poids, par rapport au poids total de la phase huileuse.
Par « *polyamide* », on entend au sens de l'invention un composé ayant au moins 2 motifs de répétition amide, de préférence au moins 3 motifs de répétition amide et mieux encore 10 motifs de répétition amide.
Par « *polyamide hydrocarboné* », on entend un polyamide formé essentiellement, voire constitué, d'atomes de carbone et d'hydrogène, et éventuellement d'atomes d'oxygène, d'azote, et ne contenant pas d'atome de silicium ou de fluor. Il peut contenir des groupes alcool, ester, éther, acide carboxylique, amine et/ou amide.
Par « *chaîne fonctionnalisée* » au sens de l'invention, on entend une chaîne alkyle comportant un ou plusieurs groupes fonctionnels ou réactifs notamment choisis parmi les groupes hydroxyle, éther, les esters, oxyalkylène ou polyoxyalkylène.
Avantageusement, ce polyamide de la composition selon l'invention présente une masse moléculaire moyenne en poids inférieure à 100 000 g/mol notamment allant de 1000 à 100 000 g/mol, en particulier inférieure à 50 000 g/mol notamment allant de 1000 à 50 000 g/mol, et plus particulièrement allant de 1000 à 30 000 g/mol, de préférence de 2000 à 20 000 g/mol, et mieux de 2000 à 10 000 g/mol.
Ce polyamide, est non soluble dans l'eau, notamment à 25 °C.
Selon un premier mode de réalisation de l'invention le polyamide utilisé est un polyamide de formule (I) :

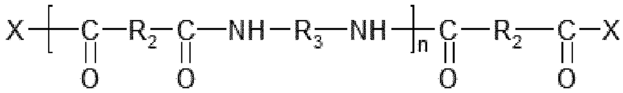
(I)

dans laquelle X représente un groupe -N($R_1$)$_2$ ou un groupe -O$R_1$ dans lequel $R_1$ est un radical alkyl linéaire ou ramifié en $C_8$ à $C_{22}$, pouvant être identique ou différents l'un de l'autre, $R_2$ est un résidu de dimère diacide en $C_{28}$-$C_{42}$, $R_3$ est un radical éthylène diamine, n est compris entre 2 et 5 ;
et leurs mélanges.

Selon un mode particulier, le polyamide utilisé est un polyamide à terminaison amide de formule (Ia) :

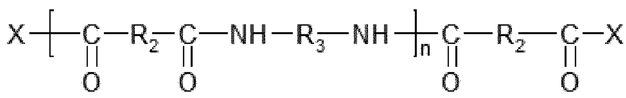
(Ia)

dans laquelle X représente un groupe -N($R_1$)$_2$ dans lequel $R_1$ est un radical alkyl linéaire ou ramifié en $C_8$ à $C_{22}$, pouvant être identique ou différents l'un de l'autre, $R_2$ est un résidu de dimère diacide en $C_{28}$-$C_{42}$, $R_3$ est un radical éthylène diamine, n est compris entre 2 et 5 ;
et leurs mélanges.

La phase huileuse d'une composition selon l'invention peut comprendre en outre, de façon additionnelle dans ce cas, au moins un polyamide additionnel de formule (Ib) :

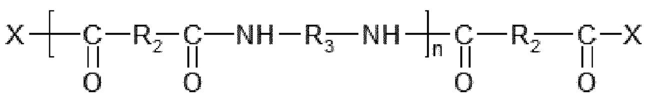
(Ib)

dans laquelle X représente un groupe -O$R_1$ dans lequel $R_1$ est un radical alkyl linéaire ou ramifié en $C_8$ à $C_{22}$, de préférence en $C_{16}$ à $C_{22}$, pouvant être identique ou différents l'un de l'autre, $R_2$ est un résidu de dimère diacide en $C_{28}$-$C_{42}$, $R_3$ est un radical éthylène diamine, n est compris entre 2 et 5, tels que les produits commerciaux vendus par la société Arizona Chemical sous les noms Uniclear 80 et Uniclear 100 ou encore Uniclear 80 V, Uniclear 100 V et Uniclear 100 VG, dont le nom INCI est « éthylènediamine/stéaryl dimère dilinoléate copolymère ».

Avantageusement, le polymère à liaison hydrogène est le copolymère éthylènediamine/stéaryl dimère dilinoléate.

## VI. COPOLYMERE SEQUENCE HYDROCARBONE

A titre représentatif de gélifiants lipophiles peuvent être en outre cités d'autres gélifiants polymériques que sont les copolymères séquencés hydrocarbonés également nommés copolymères blocs.

Le gélifiant polymérique est capable d'épaissir ou de gélifier la phase hydrocarbonée de la composition.

Par « polymère amorphe », on entend un polymère qui n'a pas de forme cristalline.

Le gélifiant polymérique est de préférence également filmogène, c'est-à-dire qu'il est capable de former un film lors de son application sur la peau et/ou les lèvres.

Le copolymère bloc hydrocarboné peut être notamment un copolymère dibloc, tribloc, multibloc, radial, étoile, ou leurs mélanges.

De tels copolymères blocs hydrocarbonés sont décrits dans la demande US-A-2002/005562 et dans le brevet US-A-5 221 534.

Le copolymère peut présenter au moins un bloc dont la température de transition vitreuse, est de préférence inférieure à 20 °C, de préférence inférieure ou égale à 0 °C, de préférence inférieure ou égale à -20 °C, de préférence encore inférieure ou égale à -40 °C. La température de transition vitreuse dudit bloc peut être comprise entre -150 °C et 20 °C, notamment entre -100 °C et 0 °C.

Le copolymère bloc hydrocarboné présent dans la composition selon l'invention est un copolymère amorphe formé par polymérisation d'une oléfine. L'oléfine peut être notamment un monomère à insaturation éthylénique élastomérique.

Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène, ou le pentadiène.

Avantageusement, le copolymère bloc hydrocarboné est un copolymère bloc amorphe de styrène et d'oléfine.

On préfère notamment les copolymères séquencés comprenant au moins un bloc styrène et au moins un bloc comprenant des motifs choisis parmi le butadiène, l'éthylène, le propylène, le butylène, l'isoprène ou un de leurs

mélanges.

Selon un mode préféré de réalisation, le copolymère bloc hydrocarboné est hydrogéné pour réduire les insaturations éthyléniques résiduelles après la polymérisation des monomères.

En particulier, le copolymère bloc hydrocarboné est un copolymère, éventuellement hydrogéné, à blocs styrène et à blocs éthylène/alkylène en C3-C4.

Selon un mode de réalisation préféré, la composition selon l'invention comprend au moins un copolymère dibloc, de préférence hydrogéné, de préférence choisi parmi les copolymères de styrène-éthylène/propylène, les copolymères de styrène-éthylène/butadiène, les copolymères de styrène-éthylène/butylène. Des polymères diblocs sont notamment vendus sous la dénomination Kraton® G1701E par la société Kraton Polymers.

Avantageusement, on utilise comme gélifiant polymérique un copolymère dibloc tel que ceux décrits précédemment, en particulier un copolymère dibloc de styrène-éthylène/propylène, ou un mélange de dibloc, tel que décrit précédemment.

Ainsi, selon une variante de réalisation préférée, une composition selon l'invention comprend au moins un copolymère séquencé hydrocarboné, de préférence un copolymère, éventuellement hydrogéné, à blocs styrène et à blocs éthylène/alkylène en $C_3$-$C_4$, encore plus préférentiellement un copolymère dibloc, de préférence hydrogéné, tel qu'un copolymère de styrène-éthylène/propylène, un copolymère de styrène-éthylène/butadiène.

Le copolymère bloc hydrocarboné (ou le mélange de copolymères blocs hydrocarbonés) peut être présent en une teneur allant de 0,1 % à 15 % en poids, de préférence allant de 0,1 % à 10 % en poids, plus préférentiellement allant de 0,5 % à 5 % en poids, mieux allant de 0,5 % à 3 % en poids, par rapport au poids total de la composition.

Selon un premier mode préféré, le gélifiant lipophile non cellulosique est choisi parmi les aérogels de silice hydrophobes.

Selon un deuxième mode préféré, le gélifiant lipophile non cellulosique est choisi parmi les élastomères d'organopolysiloxane.

Selon un troisième mode préféré, le gélifiant lipophile non cellulosique est choisi parmi les argiles modifiées.

Avantageusement, une composition selon l'invention comprend, en tant que gélifiant lipophile, un système comprenant au moins une argile modifiée et au moins un aérogel de silice hydrophobe.

Avantageusement, une composition selon l'invention comprend, en tant que gélifiant lipophile, un système comprenant au moins une argile modifiée et au moins un élastomère d'organopolysiloxane.

Avantageusement, une composition selon l'invention comprend, en tant que gélifiant lipophile, un système comprenant au moins un aérogel de silice hydrophobe et au moins un élastomère d'organopolysiloxane.

Avantageusement, une composition selon l'invention comprend, en tant que gélifiant lipophile, un système comprenant au moins une argile modifiée et au moins un aérogel de silice hydrophobe et au moins un élastomère d'organopolysiloxane.

Selon une variante préférée, une composition selon l'invention comprend un gélifiant lipophile choisi parmi :

- les homo- ou co-polymères semi-cristallins portant au moins une chaîne latérale cristallisable et les homo- ou co-polymères semi-cristallins portant dans le squelette au moins une séquence cristallisable tels que les ($C_{10}$-$C_{30}$)alkyle polyacrylates correspondant aux Intelimer® de la société Landec et par exemple le produit Intelimer® IPA 13-1 de la société Landec, qui est un polyacrylate de stéaryle de poids moléculaire d'environ 145 000 et dont la température de fusion est égale à 49 °C ;
- les polyamides hydrocarbonés et notamment les mélanges de polyamides à terminaison amide de formule (Ia) :

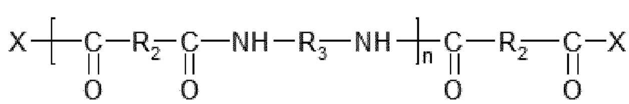

(Ia)

dans laquelle X représente un groupe $-N(R_1)_2$ dans lequel $R_1$ est un radical alkyl linéaire ou ramifié en $C_8$ à $C_{22}$, pouvant être identique ou différents l'un de l'autre, $R_2$ est un résidu de dimère diacide en $C_{28}$-$C_{42}$, $R_3$ est un radical éthylène diamine, n est compris entre 2 et 5 et de polyamides additionnels de formule (Ib) :

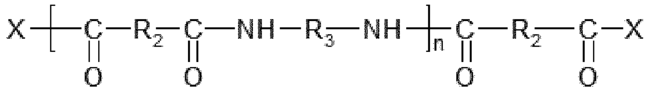

(Ib)

dans laquelle X représente un groupe $-OR_1$ dans lequel $R_1$ est un radical alkyl linéaire ou ramifié en $C_8$ à $C_{22}$, de préférence en $C_{16}$ à $C_{22}$, pouvant être identique ou différents l'un de l'autre, $R_2$ est un résidu de dimère diacide en $C_{28}$-$C_{42}$, $R_3$ est un radical éthylène diamine, n est compris entre 2 et 5, tels que les produits commerciaux vendus par

la société Arizona Chemical sous les noms Uniclear 80 et Uniclear 100 ou encore Uniclear 80 V, Uniclear 100 V et Uniclear 100 VG, dont le nom INCI est « éthylènediamine/stéaryl dimère dilinoléate copolymère » ;

- les aérogels de silice hydrophobes,
- les hectorites modifiées par un sel, de préférence un chlorure, d'ammonium en $C_{10}$ à $C_{22}$, comme l'hectorite modifiée par du chlorure de di-stéaryl di-méthyl ammonium telle que, par exemple, celle commercialisée sous la dénomination de Bentone 38V® par la société Elementis ou le gel de bentone dans isododécane commercialisé sous la dénomination Bentone Gel ISD V® (Isododécane 87 %/Disteardimonium Hectorite 10 %/Propylène carbonate 3 %) par la société Elementis,
les élastomères d'organopolysiloxane.

Selon une variante encore préférée, une composition selon l'invention comprend un gélifiant lipophile choisi parmi :

- les homo- ou co-polymères semi-cristallins portant au moins une chaîne latérale cristallisable et les homo- ou co-polymères semi-cristallins portant dans le squelette au moins une séquence cristallisable,
- les polyamides hydrocarbonés,
- les aérogels de silice hydrophobes,
- les hectorites modifiées par un chlorure d'ammonium en $C_{10}$ à $C_{22}$.

Selon une variante particulièrement préférée, une composition selon l'invention comprend un gélifiant lipophile choisi parmi les hectorites modifiées par un chlorure d'ammonium en $C_{10}$ à $C_{22}$
les élastomères d'organopolysiloxane.

Selon une première variante particulière, une composition selon l'invention comprend :

- au moins un gélifiant hydrophile non amylacé choisi parmi les copolymères d'acide 2-acrylamido 2-méthylpropane sulfonique (AMPS®), réticulés et ou neutralisés, et plus particulièrement les copolymères d'AMPS® et d'hydroxyéthyl acrylate et
- au moins un gélifiant lipophile non cellulosique choisis parmi lese hectorites modifiées par un chlorure d'ammonium en C10 à C22, et particulièrement les hectorites modifiées par du chlorure de di-stéaryl di-méthyl ammonium.

Selon une deuxième variante particulière, la composition selon l'invention comprend :

- au moins un gélifiant hydrophile non amylacé choisi parmi les copolymères d'acide 2-acrylamido 2-méthylpropane sulfonique (AMPS®), réticulés et ou neutralisés, et plus particulièrement les copolymères d'AMPS® et d'hydroxyéthyl acrylate et
- au moins un gélifiant lipophile choisi parmi les aérogels de silice hydrophobes,
- au moins un filtre UV,

et éventuellement un gélifiant additionnel choisi parmi choisi parmi les gélifiants particulaires, les élastomères d'organopolysiloxane, les polymères semi-cristallins, les esters de dextrine, les polymères à liaison hydrogène, les copolymères séquencés hydrocarbonés et leurs mélanges, et en particulier une argile modifiée et plus particulièrement les hectorites modifiées par du chlorure de di-stéaryl di-méthyl ammonium.

Notamment, la composition selon l'invention comprend :

- au moins un gélifiant hydrophile non amylacé choisi parmi les copolymères d'acide 2-acrylamido 2-méthylpropane sulfonique (AMPS®), réticulés et ou neutralisés, et plus particulièrement les copolymères d'AMPS® et d'hydroxyéthyl acrylate et
- au moins un gélifiant lipophile choisi parmi les aérogels de silice hydrophobes,
- au moins un filtre UV,

et éventuellement un gélifiant additionnel choisi parmi choisi parmi les gélifiants particulaires, les polymères semi-cristallins, les esters de dextrine, les polymères à liaison hydrogène, les copolymères séquencés hydrocarbonés et leurs mélanges, et en particulier une argile modifiée et plus particulièrement les hectorites modifiées par du chlorure de di-stéaryl di-méthyl ammonium

Selon une troisième variante particulière, la composition selon l'invention comprend :

- au moins un gélifiant hydrophile non amylacé choisi parmi les copolymères d'acide 2-acrylamido 2-méthylpropane sulfonique (AMPS®), réticulés et/ou neutralisés, et plus particulièrement les copolymères d'AMPS® et d'hydroxyéthyl

acrylate et
- au moins un gélifiant lipophile choisi parmi les élastomères d'organopolysiloxane,
- au moins un filtre UV,

et éventuellement un gélifiant additionnel choisi parmi les gélifiants particulaires, les polymères semi-cristallins, les esters de dextrine, les polymères à liaison hydrogène, les copolymères séquencés hydrocarbonés et leurs mélanges, et en particulier choisi parmi les aérogels de silice hydrophobes, les argiles modifiées et leurs mélanges en particulier et plus particulièrement les hectorites modifiées par du chlorure de di-stéaryl di-méthyl ammonium, et leurs mélanges.

Notamment, la composition selon l'invention comprend

- au moins un gélifiant hydrophile non amylacé choisi parmi les copolymères d'acide 2-acrylamido 2-méthylpropane sulfonique (AMPS®), réticulés et ou neutralisés, et plus particulièrement les copolymères d'AMPS® et d'hydroxyéthyl acrylate et
- au moins un gélifiant lipophile choisi parmi les élastomères d'organopolysiloxane
  et éventuellement un gélifiant additionnel choisi parmi les gélifiants particulaires, les polymères semi-cristallins, les esters de dextrine, les polymères à liaison hydrogène, les copolymères séquencés hydrocarbonés et leurs mélanges, et en particulier les aérogels de silice hydrophobes ; et
- au moins un filtre UV.De façon préférée, la teneur totale en gélifiant(s) lipophile(s) est comprise entre 0,1 % et 80 % en poids de matière sèche, en particulier de 0,2 % à 60 % en poids, de préférence entre 2 % et 12 % en poids, par rapport au poids total de la phase huileuse.

Encore plus particulièrement, ledit filtre UV est choisi parmi les filtres UV organiques hydrosolubles, les filtres organiques liposolubles et leurs mélanges et plus particulièrement les filtres UV organiques liposolubles.

## SYSTÈME GÉLIFIANT(S) HYDROPHILE(S)/GÉLIFIANT(S) LIPOPHILE(S)

A titre illustratif et non limitatif de systèmes gélifiant(s) hydrophile(s)/gélifiant(s) lipophile(s) convenant tout particulièrement à l'invention peuvent notamment être cités les systèmes suivants

- copolymères d'acide 2-acrylamido 2-méthylpropane sulfonique, réticulés et ou neutralisés / homo- ou co-polymères semi-cristallins portant au moins une chaîne latérale cristallisable, ladite chaine cristallisable étant latérale ou dans le squelette ;
- copolymères d'acide 2-acrylamido 2-méthylpropane sulfonique, réticulés et ou neutralisés / polyamides hydrocarbonés ;
- copolymères d'acide 2-acrylamido 2-méthylpropane sulfonique, réticulés et ou neutralisés / aérogels de silice hydrophobes ;
- et plus particulièremenet : copolymères d'acide 2-acrylamido 2-méthylpropane sulfonique, réticulés et ou neutralisés / hectorites modifiées par un sel, de préférence un chlorure, d'ammonium en $C_{10}$ à $C_{22}$ ;
- polymères carboxyvinyliques modifiés ou non / homo- ou co-polymères semi-cristallins portant au moins une chaîne latérale cristallisable, ladite chaine cristallisable étant latérale ou dans le squelette ;
- polymères carboxyvinyliques modifiés ou non / polyamides hydrocarbonés ;
- polymères carboxyvinyliques modifiés ou non / aérogels de silice hydrophobes ;
- et plus particulièremenet : polymères carboxyvinyliques modifiés ou non / hectorites modifiées par un sel, de préférence un chlorure, d'ammonium en $C_{10}$ à $C_{22}$.

Selon une forme particulèrement préférée, la composition selon l'invention comprend :

- au moins un gélifiant hydrophile non amylacé choisi parmi les copolymères d'acide 2-acrylamido 2-méthylpropane sulfonique (AMPS®), réticulés et ou neutralisés, et plus particulièrement les copolymères d'AMPS® et d'hydroxyéthyl acrylate et
- au moins un gélifiant lipophile non cellulosique choisi parmi les hectorites modifiées par un sel, de préférence un chlorure, d'ammonium en C10 à C22, et plus particulièrment les hectorites modifiées par du chlorure de di-stéaryl di-méthyl ammonium,
- au moins un filtre UV.

Selon une variante préférée, le système gélifiant comprent au moins un aérogels de silice hydrophobes

A titre illustratif et non limitatif de systèmes gélifiant(s) hydrophile(s)/gélifiant(s) lipophile(s) convenant tout particulièrement à l'invention peuvent notamment être cités les systèmes suivants :

copolymères d'acide 2-acrylamido 2-méthylpropane sulfonique, réticulés et ou neutralisés /aérogels de silice hydrophobes ;
copolymères d'acide 2-acrylamido 2-méthylpropane sulfonique, réticulés et ou neutralisés /aérogels de silice hydrophobes et argiles modifiées ;
copolymères d'acide 2-acrylamido 2-méthylpropane sulfonique, réticulés et ou neutralisés /aérogels de silice hydrophobes et elastomères de silicone ;
copolymères d'acide 2-acrylamido 2-méthylpropane sulfonique, réticulés et ou neutralisés /aérogels de silice hydrophobes, argiles modifiées et elastomères de silicone.

Selon une variante préférée, le système gélifiant comprent au moins un élastomère d'organopolysiloxane. A titre illustratif et non limitatif de systèmes gélifiant(s) hydrophile(s)/gélifiant(s) lipophile(s) convenant tout particulièrement à l'invention peuvent notamment être cités les systèmes suivants :

copolymères d'acide 2-acrylamido 2-méthylpropane sulfonique, réticulés et ou neutralisés / elastomères de silicone ;
copolymères d'acide 2-acrylamido 2-méthylpropane sulfonique, réticulés et ou neutralisés / elastomères de silicone et aérogels de silice hydrophobes;
copolymères d'acide 2-acrylamido 2-méthylpropane sulfonique, réticulés et ou neutralisés / elastomères de silicone et argiles modifiées;
copolymères d'acide 2-acrylamido 2-méthylpropane sulfonique, réticulés et ou neutralisés / elastomères de silicone,aérogels de silice hydrophobes et argiles modifiées.

## FILTRES UV

Les compositions selon l'invention, contiennent au moins un filtre UV choisi parmi les filtres organiques hydrosolubles, les filtres organiques liposolubles et les filtres organiques insolubles.
De manière préférentielle, le ou les filtres UV sont choisis parmi les filtres UV organiques hydrosolubles, les filtres organiques liposolubles et leurs mélanges et plus particulièrement les filtres UV organiques liposolubles.
Par "filtre UV hydrosoluble" on entend tout composé filtrant les radiations UV susceptible d'être complètement dissous ou miscibilisé à l'état moléculaire dans une phase aqueuse ou bien susceptible d'être solubilisé sous forme colloïdale (par exemple sous forme micellaire) dans une phase aqueuse.
Par "filtre liposoluble" on entend tout composé filtrant les radiations UV susceptible d'être complètement dissous ou miscibilisé à l'état moléculaire dans une phase grasse ou bien susceptible d'être solubilisé, sous forme colloïdale (par exemple sous forme micellaire) dans une phase grasse.
Par « filtre UV insoluble », on entend tout composé filtrant les radiations UV ayant une solubilité dans l'eau inférieure à 0,5 % en poids et une solubilité inférieure à 0,5 % en poids dans la plupart des solvants organiques comme l'huile de paraffine, les benzoates d'alcools gras et les triglycérides d'acides gras, par exemple le Miglyol 812® commercialisé par la société DYNAMIT NOBEL. Cette solubilité, réalisée à 70 °C est définie comme la quantité de produit en solution dans le solvant à l'équilibre avec un excès de solide en suspension après retour à la température ambiante. Elle peut facilement être évaluée au laboratoire.

## I/ FILTRES UV ORGANIQUE HYDROSOLUBLES

### A/ FILTRES UVA ORGANIQUES HYDROSOLUBLES

Par « filtre UVA organique hydrosolubl » on entend tout composé organique filtrant le rayonnement UVA dans le domaine de longueurs d'onde 320 à 400nm susceptible d'être complètement dissous ou miscibilisé à l'état moléculaire dans une phase aqueuse ou bien susceptible d'être solubilisé sous forme colloïdale (par exemple sous forme micellaire) dans une phase aqueuse.
Parmi les filtres UVA organiques hydrosolubles utilisables selon la présente invention, on peut citer : L'acide benzène 1,4-di(3-méthylidène-10-camphosulfonique) (nom INCI: Terephthalylidene Dicamphor Sulfonic Acid) et ses différents sels, décrits notamment dans les demandes de brevets FR-A-2528420 et FR-A-2639347.
Ces filtres répondent à la formule générale (I) suivante :

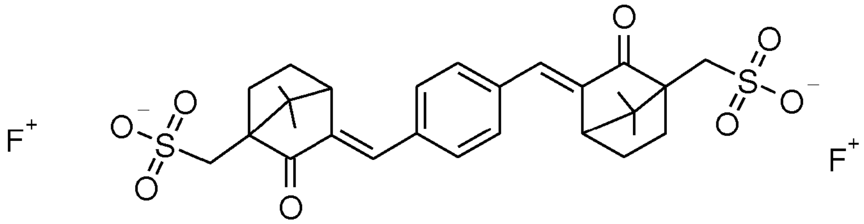

(I)

dans laquelle F désigne un atome d'hydrogène, un métal alcalin ou encore un radical NH(R1)3+ dans lequel les radicaux R1, qui peuvent être identiques ou différents, désignent un atome d'hydrogène, un radical alkyle ou hydroxyalkyle en C1-C4 ou encore un groupement Mn+ /n, Mn+ désignant un cation métallique polyvalent dans lequel n est égal à 2 ou 3 ou 4, Mn+ désignant de préférence un cation métallique choisi parmi Ca2+, Zn2+, Mg2+, Ba2+, A13+ et Zr4+. Il est bien entendu que les composés de formule (I) ci-dessus peuvent donner lieu à l'isomère « cis-trans » autour d'une ou plusieurs double(s) liaison(s) et que tous les isomères rentrent dans le cadre de la présente invention.

Parmi les filtres UVA organiques hydrosolubles utilisables selon la présente invention, on peut citer également les composés comportant au moins deux groupes benzoazolyle à groupes sulfoniques tels que ceux décrits dans la demande de brevet EP-A-0669323. Ils sont décrits et préparés selon les synthèses indiquées dans le brevet US 2,463,264 ainsi que la demande de brevet EP-A-0669323.

Les composés comportant au moins deux groupes benzoazolyle conformes à l'invention répondent à la formule générale (II) suivante :

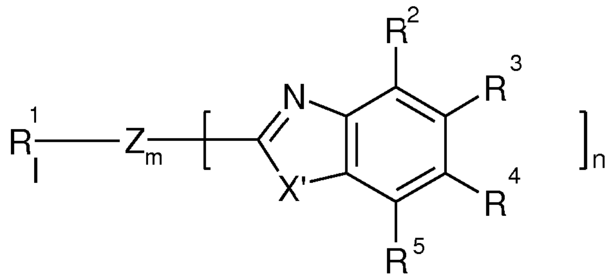

(II)

dans laquelle :

- Z représente un reste organique de valence (1 + n) comportant une ou plusieurs doubles liaisons placées de telle sorte que qu'elle complète le système de doubles liaisons d'au moins deux groupes benzoazolyle tels que définis à l'intérieur des crochets pour former un ensemble totalement conjugué ;
- X' désigne S, O ou NR6
- R1 désigne hydrogène, alkyle en C1-C18, alcoxy en C1-C4, un aryle en C5-C15, un acyloxy en C2-C18, SO3Y ou COOY ;
- les radicaux R2, R3, R4 et R5, identiques ou différents, désigne un groupe nitro ou un radical R1 ;
- R6 désigne hydrogène, un alkyle en C1-C4 ou un hydroxyalkyle en C1-C4 ;
- Y désigne hydrogène, Li, Na, K, NH4, 1/2Ca, 1/2Mg, 1/3Al ou un cation résultant de la neutralisation d'un groupe acide libre par une base azotée organique ;
- m est 0 ou 1 ;
- n est un nombre de 2 à 6 ;
- 1 est un nombre de 1 à 4 ;
- sous réserve que 1 + n ne dépasse pas la valeur 6.

Parmi ces composés, on préfère ceux pour lesquels le groupe Z est choisi dans le groupe constitué par :

(a) un radical hydrocarboné en C2-C6 aliphatique linéaire oléfine pouvant être interrompu par un groupe aryle en C5-C12 ou un hétéroaryle en C4-C10 en particulier choisi parmi les groupes suivants :

-CH=CH-, -CH=CH-CH=CH-

ou bien

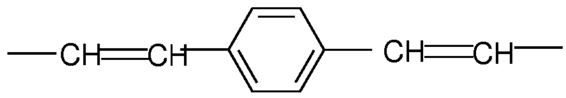

(b) un groupe aryle en $C_5$-$C_{15}$ pouvant être interrompu par un radical hydrocarboné en $C_2$-$C_6$ aliphatique linéaire oléfine en particulier choisi parmi les groupes suivants :

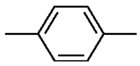

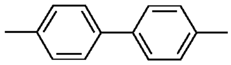

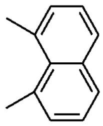

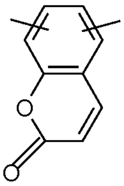

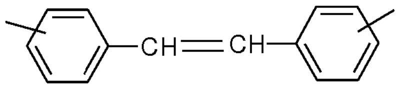

(c) un reste hétéroaryle en $C_3$-$C_{10}$ en particulier choisi parmi les groupes suivants :

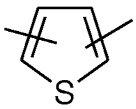
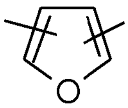
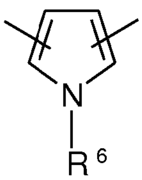
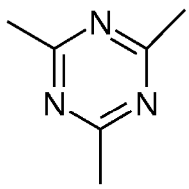

où $R^6$ a la même signification indiquée ci-dessus ; lesdits radicaux Z tels que définis dans les paragraphes (a), (b) et (c) pouvant être substitués par des radicaux alkyle en $C_1$-$C_6$, alcoxy en $C_1$-$C_6$, phénoxy, hydroxy, méthylènedioxy ou amino éventuellement substitués par un ou 2 radicaux alkyle en $C_1$-$C_5$.

De préférenceles composés de formule (II) comportent par molécule de 1, 3 ou 4 groupes $SO_3Y$.
A titre d'exemples de composés de formule (II) utilisables, on peut citer les composés de formules (a) à (j) et de structure suivante ainsi que leurs sels :

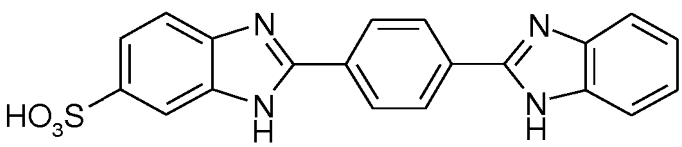
(a)

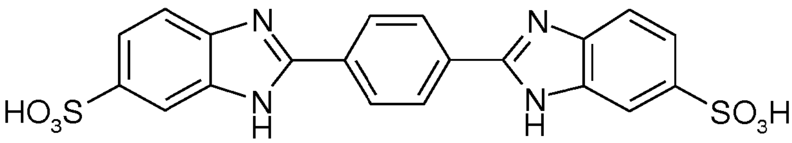
(b)

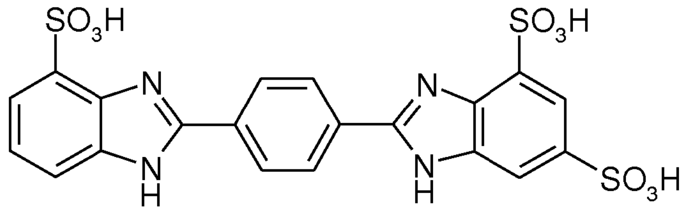

(c)

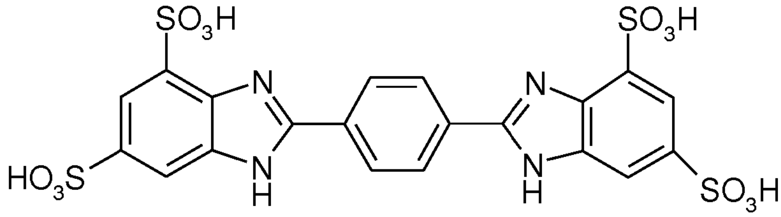

(d)

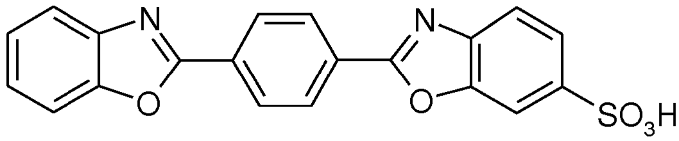

(e)

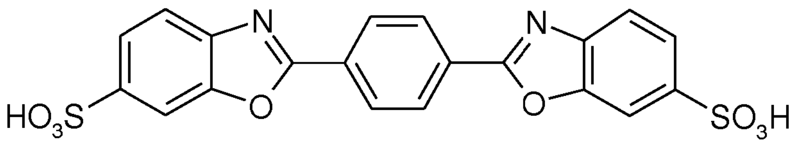

(f)

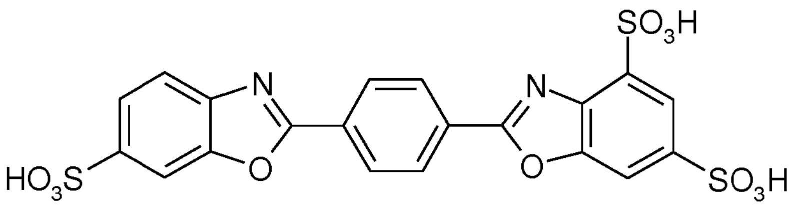

(g)

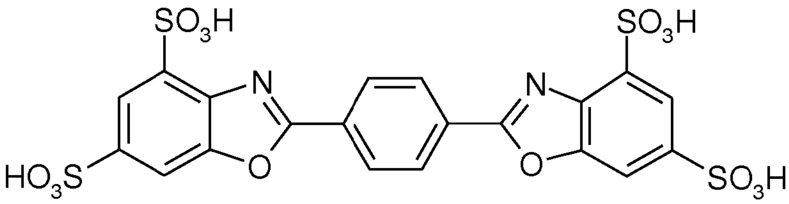

(h)

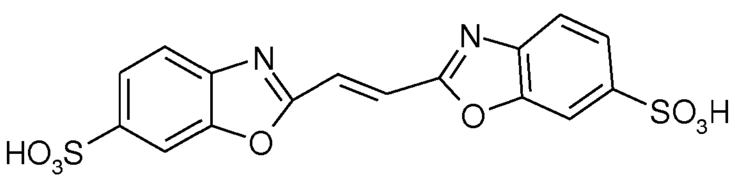

(i)

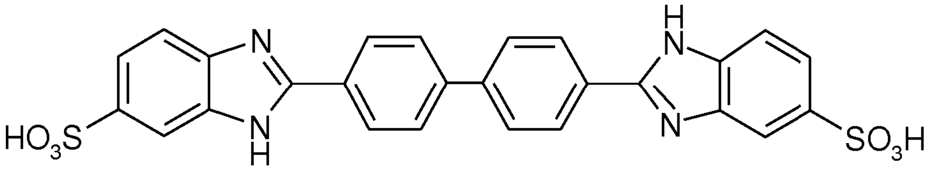

(j)

Parmi tous ces composés on préférera tout particulièrement l'acide 1,4-bis-benzimidazolyl-phènylèn-3,3',5,5'-tétrasulfonique (nom INCI: Disodium Phenyl Dibenzimidazole Tetra-sulfonate) (composé (d)) ou l'un de ses sels de structure suivante vendu notamment sous la dénomination de NEOHELIOPAN AP® par la société Symrise :

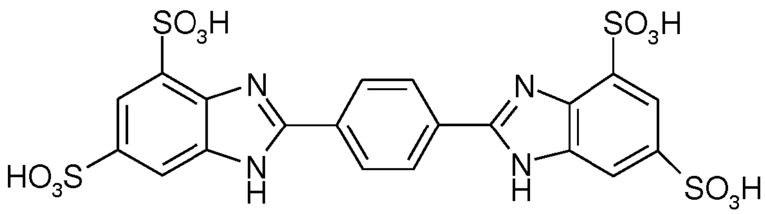

Parmi les filtres UVA organiques hydrosolubles utilisables selon la présente invention, on peut citer également les composés benzophénones comprenant au moins une fonction acide sulfonique, comme par exemple les composés suivants :

Benzophenone-4, vendu notamment par la société BASF sous le nom Uvinul MS40® :

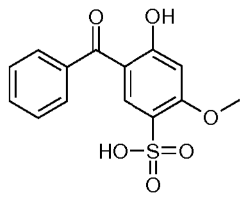

Benzophenone-5 de structure

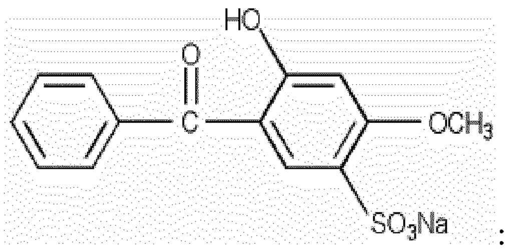

;

Benzophenone-9, vendu notamment par la société BASF sous le nom Uvinul DS49® :

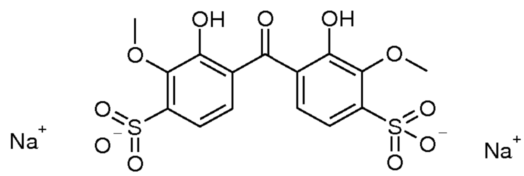

Parmi les filtres UVA organiques hydrosolubles, on utilisera plus particulièrement l'acide benzène 1,4-di(3-méthylidène-10-camphosulfonique) et ses différents sels (nom INCI: Terephthalylidene Dicamphor Sulfonic Acid) notamment fabriqué par la société CHIMEX sous le nom commercial MEXORYL SX®.

Le ou les filtres UVA organiques hydrosolubles conformes à l'invention sont de préférence présents dans les compositions selon l'invention à une concentration en matière active allant de 0,01 à 30 %, de préférence de 0,1 à 15 % en poids par rapport au poids total de la composition.

**B/ FILTRES UVB ORGANIQUES HYDROSOLUBLES**

Par « filtre UVB organique hydrosoluble » on entend tout composé organique filtrant le rayonnement UVB dans le domaine de longueurs d'onde allant de 280 à 320 nm susceptible d'être complètement dissous ou miscibilisé à l'état moléculaire dans une phase aqueuse ou bien susceptible d'être solubilisé sous forme colloïdale (par exemple sous forme micellaire) dans une phase aqueuse.

Les filtres UVB organiques hydrosolubles sont notamment choisis parmi :

les dérivés cinnamiques hydrosolubles comme l'acide férulique ou acide 3-méthoxy-4-hydroxycinnamique
les composés benzylidène camphre hydrosolubles ;
les composés phenylbenzimidazole hydrosolubles ;
les composés p-aminobenzoïques (PABA) hydrosolubles ;
les composés salicyliques hydrosolubles
et leurs mélanges.

Comme exemples de filtres UVB organiques hydrosolubles, on peut citer ceux désignés ci-dessous sous leur nom INCI :

Composés para-aminobenzoique :

PABA,

PEG-25 PABA vendu notamment sous le nom « UVINUL P 25® » par BASF.

Composés salicyliques :

Dipropyleneglycol Salicylate vendu notamment sous le nom « DIPSAL ® » par SCHER,
**[0478]** TEA Salicylate, vendu notamment sous le nom « NEO HELIOPAN TS® » par Symrise,

Composés benzylidène camphre :

Benzylidene Camphor Sulfonic Acid commercialisé notamment sous le nom « MEXORYL SL® » par CHIMEX, Camphor Benzalkonium Methosulfate commercialisé notamment sous le nom « MEXORYL SO® » par CHIMEX.

Composés phenyl benzimidazole :

Phenylbenzimidazole Sulfonic Acid vendu notamment sous le nom commercial « EUSOLEX 232® » par MERCK.
On utilisera plus particulièrement le filtre Phenylbenzimidazole Sulfonic Acid vendu notamment sous le nom commercial « EUSOLEX 232® » par MERCK.
Le ou les filtres UVB organiques hydrosolubles conformes à l'invention sont de préférence présents dans les compositions selon l'invention à une concentration en matière active comprise allant de 0,01 à 30 %, de préférence de 0,1 à 15 %, en poids par rapport au poids total de la composition.

**II/ FILTRES UV ORGANIQUE LIPOSOLUBLES**

Par "filtre UVB organique liposoluble" on entend tout composé organique filtrant le rayonnement UVB dans le domaine de longueurs d'onde allant de 280 à 320 nm susceptible d'être complètement dissous ou miscibilisé à l'état moléculaire dans une phase grasse ou bien susceptible d'être solubilisé sous forme colloïdale (par exemple sous forme micellaire) dans une phase grasse. Parmi les filtres UV organiques liposolubles, certains d'entre eux sont liquides à température ambiante.
Les filtres UV organiques liposolubles sont notamment choisis parmi les dérivés cinnamiques ; les anthranilates ; les dérivés salicyliques, les dérivés de dibenzoylméthane, les dérivés du camphre ; les dérivés de la benzophénone ; les dérivés de β,β-diphénylacrylate ; les dérivés de triazine ; les dérivés de benzotriazole ; les dérivés de benzalmalonate notamment ceux cités dans le brevet US5624663 ; les imidazolines ; les dérivés de l'acide p-aminobenzoïque (PABA) ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'a-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 ; les dérivés de mérocyanine les mérocyanines telles que décrites dans le brevet US4195999, la demande WO2004/006878, les demandes WO2008/090066, WO2011113718, WO2009027258, et les documents IP COM JOURNAL N°000179675D publié le 23 février 2009, IP COM JOURNAL N°000182396D publié le 29 avril 2009, IP COM JOURNAL N° 000189542D publié le 12 novembre 2009, IP COM Journal N°IPCOM000011179D publié le 04/03/2004 et leurs mélanges.
Comme exemples de filtres UV organiques liposolubles, on peut citer ceux désignés ci-dessous sous leur nom INCI :

Dérivé de dibenzoylméthane

Butyl Methoxy Dibenzoylmethane ou avobenzone, proposé à la vente notamment sous la dénomination commerciale de « PARSOL 1789 » par la Société DSM NUTRITIONAL PRODUCTS ;

Dérivés de l'acide para-aminobenzoique :

Ethyl PABA,
Ethyl Dihydroxypropyl PABA,
Ethylhexyl Diméthyl PABA vendu notamment sous le nom « ESCALOL 507 » par ISP ;

Dérivés salicyliques :

Homosalate vendu notamment sous le nom « Eusolex HMS » par Rona/EM Industries,
Ethylhexyl Salicylate vendu notamment sous le nom « NEO HELIOPAN OS » par SYMRISE ;

Dérivés cinnamiques :

Ethylhexyl Methoxycinnamate vendu notamment sous le nom commercial « PARSOL MCX » par DSM NUTRITIONAL PRODUCTS,
Isopropyl Methoxy cinnamate,
Isoamyl Methoxy cinnamate vendu notamment sous le nom commercial « NEO HELIOPAN E 1000 » par SYMRISE,
Cinoxate,
Diisopropyl Methylcinnamate ;

Dérivés de β,β-diphénylacrylate :

Octocrylene vendu notamment sous le nom commercial « UVINUL N539 » par BASF,
Etocrylene, vendu notamment sous le nom commercial « UVINUL N35 » par BASF;

Dérivés de la benzophénone :

Benzophenone-1 vendu notamment sous le nom commercial « UVINUL 400 » par BASF,
Benzophenone-2 vendu notamment sous le nom commercial « UVINUL D50 » par BASF,
Benzophenone-3 ou Oxybenzone, vendu notamment sous le nom commercial « UVINUL M40 » par BASF,
Benzophenone-6 vendu notamment sous le nom commercial « Helisorb 11 » par Norquay,
Benzophenone-8 vendu notamment sous le nom commercial « Spectra-Sorb UV-24 » par American Cyanamid,
Benzophenone-12,
2-(4-diethylamino-2-hydroxybenzoyl)-benzoate de n-hexyle vendu notamment sous le nom commercial « UVINUL A +» tel que « UVINUL A + GRANULAR» ou sous forme de mélange avec l'octylmethoxycinnamate notamment sous le nom commercial« UVINUL A + B» par BASF ;

Dérivés du benzylidène camphre :

3-Benzylidene camphor commercialisé notamment sous le nom « MEXORYL SD» par CHIMEX,
4-Methylbenzylidene camphor vendu notamment sous le nom « EUSOLEX 6300 » par MERCK ,
Polyacrylamidomethyl Benzylidene Camphor commercialisé notamment sous le nom « MEXORYL SW » par CHIMEX ;

Dérivés du phenyl benzotriazole :

Drometrizole Trisiloxane vendu notamment sous le nom « Silatrizole » par RHODIA CHIMIE ;

Dérivés de triazine :

Bis-Ethylhexyloxyphenol Methoxyphenyl Triazine vendu notamment sous le nom commercial «TINOSORB S » par BASF,
Ethylhexyl triazone vendu notamment sous le nom commercial «UVINUL T150 » par BASF,
Diethylhexyl Butamido Triazone vendu notamment sous le nom commercial « UVASORB HEB » par SIGMA 3V,

- les silicones triazines substituées par deux groupes aminobenzoates telles que décrites que le brevet EP0841341 en particulier le 2,4-bis-(n-butyl 4'-aminobenzalmalonate)-6-[(3-11,3,3,3-tetramethyl-1-[(trimethylsilyloxy]-disiloxanyl} propyl)amino]-s-triazine ;

Dérivés anthraniliques :

Menthyl anthranilate vendu notamment sous le nom commercial commercial « NEO HELIOPAN MA » par SYMRISE,

Dérivés d'imidazolines :

Ethylhexyl Dimethoxybenzylidene Dioxoimidazoline Propionate ;

Dérivés du benzalmalonate :

Di-néopentyl 4 '-méthoxybenzalmalonate,
Polyorganosiloxane à fonctions benzalmalonate comme le Polysilicone-15 vendu notamment sous la dénomination commerciale « PARSOL SLX » par DSM ;

Dérivés de 4,4-diarylbutadiène :

1,1-dicarboxy (2,2'-diméthyl-propyl)-4,4-diphénylbutadiène,

Dérivés de benzoxazole :

2,4-bis-[5-1(diméthylpropyl)benzoxazol-2-yl-(4-phenyl)-imino]-6-(2-ethylhexyl)-imino-1,3,5-triazine vendu notamment sous le nom d'Uvasorb K2A par Sigma 3V,
et leurs mélanges ;

Les dérivés de mérocyanine lipophiles

- Octyl-5-N,N-diethylamino-2-phenysulfonyl-2,4-pentadienoate, et leurs mélanges ;

Les filtres organiques liposolubles préférentiels sont choisis parmi
Butyl Methoxy Dibenzoylmethane
Ethylhexyl Methoxycinnamate
Ethylhexyl Salicylate,
Homosalate,
Butyl Methoxydibenzoylmethane
Octocrylene,
Benzophenone-3,
2-(4-diethylamino-2-hydroxybenzoyl)-benzoate de n-hexyle.
4-Methylbenzylidene camphor,
Bis-Ethylhexyloxyphenol Methoxyphenyl Triazine
Ethylhexyl triazone,
Diethylhexyl Butamido Triazone,
la 2,4,6-tris(4'-amino benzalmalonate de dinéopentyle)-s-triazine
la 2,4,6-tris-(4'-amino benzalmalonate de diisobutyle)-s- triazine
la 2,4-bis (4'-aminobenzalmalonate de dinéopentyle)-6-(4'-aminobenzoate de n-butyle)-s-triazine, Drometrizole

Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine et leurs mélanges.
Les filtres organiques liposolubles préférentiels sont choisis plus particulièrement parmi
Butyl Methoxydibenzoylmethane
Octocrylene,
Ethylhexyl Salicylate,
2-(4-diethylamino-2-hydroxybenzoyl)-benzoate de n-hexyle.
Bis-Ethylhexyloxyphenol Methoxyphenyl Triazine
Ethylhexyl triazone,
Diethylhexyl Butamido Triazone,
Drometrizole Trisiloxane et leurs mélanges.

Le ou les filtres UV organiques liposolubles sont de préference présents dans les compositions selon l'invention à une teneur allant de 0,1 % à 50 % en poids et en particulier de 0,5 à 30 %, en poids, par rapport au poids total de la composition.

## III/ FILTRES UV ORGANIQUES ET INORGANIQUES INSOLUBLES

### A/ FILTRES UV ORGANIQUES INSOLUBLES

Les filtres UV organiques insolubles selon l'invention ont de préference une taille moyenne des particules qui varie de 0,01 à 5μm et plus préférentiellement de 0,01 à 2 μm et plus particulièrement de 0,020 à 2 μm.

Le diamètre moyen des particules étant mesuré par un analyseur de distribution de taille de particules du type Culter N4 PLUS fabriqué par Bechman Coulter INC.

Les filtres organiques insolubles selon l'invention peuvent être amenés sous la forme particulaire souhaitée par tout moyen ad-hoc tel que notamment broyage à sec ou en milieu solvant, tamisage, atomisation, micronisation, pulvérisation.

Les filtres organiques insolubles selon l'invention sous forme micronisée peuvent en particulier être obtenus par un procédé de broyage d'un filtre UV organique insoluble sous forme de particules de taille grossière en présence d'un tensio-actif approprié permettant d'améliorer la dispersion des particules ainsi obtenues dans les formulations cosmétiques.

Un exemple de procédé de micronisation de filtres organiques insolubles est décrit dans les demandes GB-A-2 303 549 et EP-A-893119. L'appareil de broyage utilisé selon ces documents peut être un broyeur à jet, à billes, à vibration ou à marteau et de préférence un broyeur à haute vitesse d'agitation ou un broyeur à impact et plus particulièrement un broyeur à billes rotatives, un broyeur vibrant, à broyeur à tube ou un broyeur à tige.

Selon ce procédé particulier, on utilise à titre de tensio-actifs pour le broyage desdits filtres, les alkylpolyglucosides de structure $C_nH_{2n+1}$ $O(C_6H_{10}O_5)_xH$ dans laquelle n est un entier de 8 à 16 et x est le degré moyen de polymérisation de l'unité ($C_6H_{10}O_5$) et varie de 1,4 à 1,6. Ils peuvent être choisis parmi des esters en $C_1$-$C_{12}$ d'un composé de structure $C_nH_{2n+1}$ $O(C_6H_{10}O_5)_xH$ et plus précisément un ester obtenu par réaction d'un acide carboxylique en $C_1$-$C_{12}$ tel que l'acide formique, acétique, propionique, butyrique, sulfosuccinique, citrique ou tartrique avec une ou plusieurs fonctions OH libres sur l'unité glucoside ($C_6H_{10}O_5$). On peut citer comme alkylpolyglucoside en particulier le decylglucoside.

Lesdits tensio-actifs sont utilisés en général à une concentration allant de 1 à 50 % en poids et plus préférentiellement de 5 à 40 % en poids par rapport au filtre insoluble dans sa forme micronisée.

Les filtres UV organiques insolubles conformes à l'invention peuvent être choisis notamment parmi les filtres UV organiques du type oxalanilide, du type triazine, du type benzotriazole ; du type amide vinylique ; du type cinnamide ; du type comportant un ou plusieurs groupements benzazole et/ou benzofuranne, benzothiophènene ou du type indole ; du type aryl vinylène cétone ; du type dérivé de phénylène bis-benzoxazinone, du type dérivé amide, sulfonamide ou carbamate d'acrylonitrile ou leurs mélanges.

Au sens où on l'utilise dans la présente invention, le terme benzazole englobe à la fois les benzothiazoles, benzoxazoles et benzimidazoles.

A/ Oxalanides

Parmi les filtres UV du type oxalanilide conformes à l'invention, on peut citer ceux répondant à la structure :

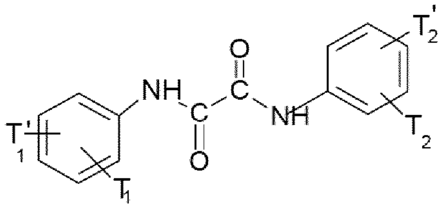

(I)

dans laquelle $T_1$, $T'_1$, $T_2$ et $T'_2$ désignent, identiques et différents, un radical alkyle en $C_1$-$C_8$ ou un radical alcoxy en $C_1$-$C_8$. Ces composés sont décrits dans la demande de brevet WO95/22959.

A titre d'exemples, on peut citer les produits commerciaux TINUVIN 315® et TINUVIN 312® vendus par la Société BASF et respectivement de structure :

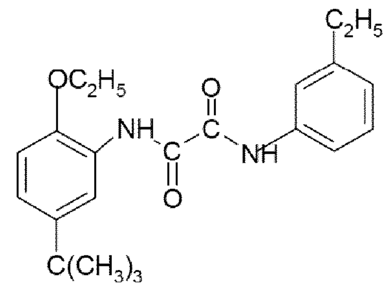

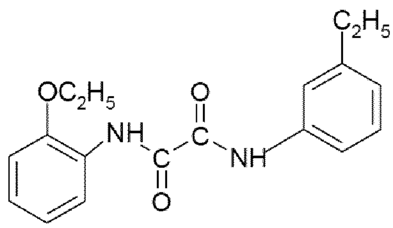

B/ Triazines

Parmi les filtres UV insolubles du type triazine conformes à l'invention, on peut également mentionner ceux répondant à la formule (II) suivante :

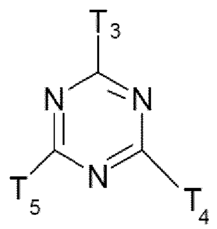

(II)

dans laquelle $T_3$, $T_4$, $T_5$, indépendamment, sont phenyle, phenoxy, pyrrolo, dans lesquels les phenyle, phenoxy, pyrrolo non substitués ou substitués par un, deux ou trois substituants choisis parmi OH, $C_1$-$C_{18}$alkyle ou $C_1$-$C_{18}$alkoxy, $C_1$-$C_{18}$carboxyalkyle, $C_5$-$C_8$cycloalkyle, un groupe méthylbenzylidènecamphre, un groupe $-(CH=CH)_n(CO)-OT_6$, avec $T_6$ soit $C_1$-$C_{18}$alkyle soit cinnamyle.

Ces composés sont décrits dans WO 97/03642, GB 2286774, EP-743309, WO 98/22447, GB 2319523.

Parmi les filtres UV du type triazine conformes à l'invention, on peut également mentionner les dérivés insolubles de s-triazine portant des groupements benzalmalonates et/ou phenylcyanoacrylates tels que ceux décrits dans la demande EP-A-0790243 (faisant partie intégrante du contenu de la description).

Parmi ces filtres UV insolubles du type triazine, on citera plus particulièrement les composés suivants :

- la 2,4,6-tris(4'-amino benzalmalonate de diéthyle)-s-triazine,
- la 2,4,6-tris(4'-amino benzalmalonate de diisopropyle)-s-triazine,
- la 2,4,6-tris(4'-amino benzalmalonate de diméthyle)-s-triazine,

- la 2,4,6-tris(α-cyano-4-aminocinnamate d'éthyle)-s-triazine.

Parmi les filtres UV du type triazine conformes à l'invention, on peut encore mentionner les dérivés insolubles de s-triazine portant des groupements benzotriazoles et/ou benzothiazoles tels que ceux décrits dans la demande WO98/25922 (faisant partie intégrante du contenu de la description).

Parmi ces composés, on peut citer plus particulièrement :

- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

On peut citer également les triazines symétriques substituées par des groupes naphthalenyles ou des groupes polyphényles décrits dans le brevet US6,225,467, la demande WO2004/085412 (voir composés 6 et 9) ou le document « Symetrical Triazine Derivatives» IP.COM Journal , IP.COM INC WEST HENRIETTA, NY, US (20 septembre 2004) notamment la 2,4,6-tris(di-phenyl)-triazine et la 2,4,6-tris(ter-phenyl)-triazine qui est repris dans les demandes de brevet WO06/035000, WO06/034982, WO06/034991, WO06/035007, WO2006/034992, WO2006/034985.

C/ Benzotriazoles

Parmi les filtres UV organiques insolubles du type benzotriazole conformes à l'invention, on peut citer ceux de formule suivante (III) tels que décrits dans la demande WO95/22959 (faisant partie intégrante du contenu de la description) :

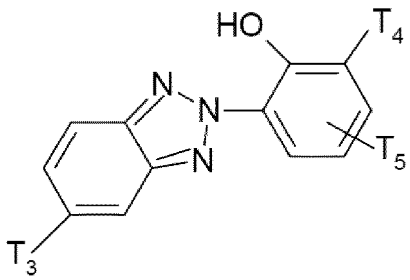

(III)

dans laquelle $T_7$ désigne un atome d'hydrogène ou un radical alkyle en $C_1$-$C_{18}$ ; $T_8$ et $T_9$, identiques ou différents, désignent un radical alkyle en $C_1$-$C_{18}$ éventuellement substitué par un phényle.

A titre d'exemple de composés de formule (III), on peut citer les produits commerciaux TINUVIN 328 , 320, 234 et 350 de la Société BASF de structure suivante :

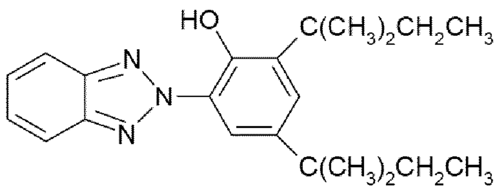

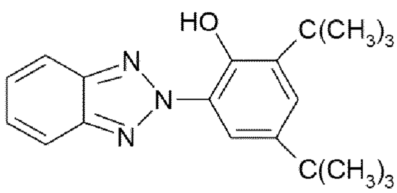

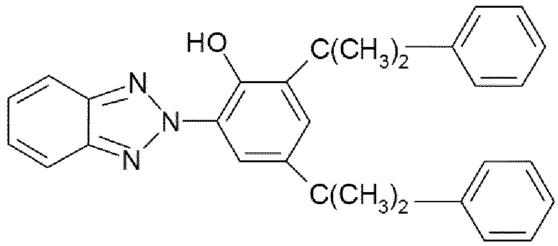

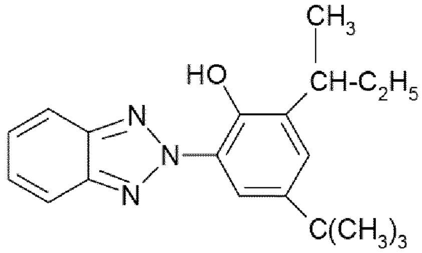

Parmi les filtres UV organiques insolubles du type benzotriazole conformes à l'invention, on peut citer les composés tels que décrits dans les brevets US 5 687 521, US5 373 037, US 5 362 881 et en particulier le [2 ,4'-dihydroxy-3-(2H-benzotriazol-2-yl)-5-(1,1,3,3-tétraméthylbutyl)-2'-n-octoxy-5'-benzoyl] diphénylméthane vendu notamment sous le nom MIXXIM PB30® par la société FAIRMOUNT CHEMICAL de structure :

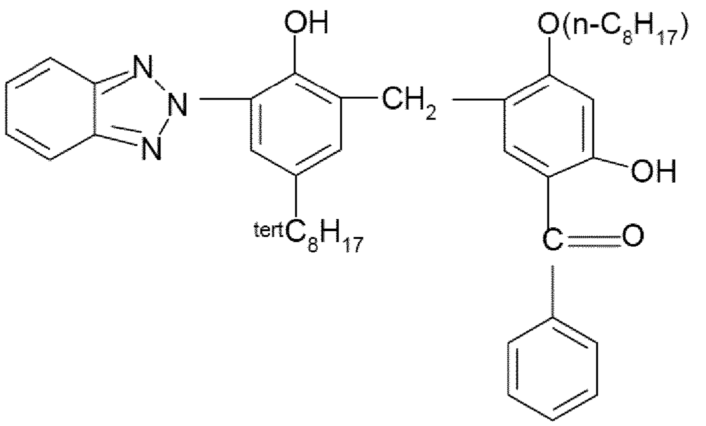

Parmi les filtres UV organiques insolubles du type benzotriazole conformes à l'invention, on peut citer les dérivés de méthylène bis-(hydroxyphényl benzotriazole) de structure suivante :

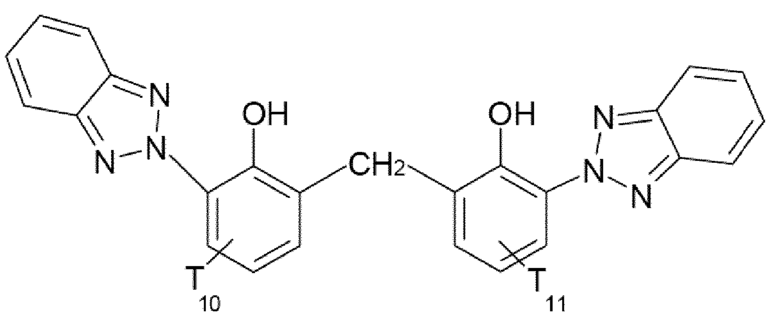

(IV)

dans laquelle les radicaux $T_{10}$ et Tu, identiques ou différents, désignent un radical alkyle en $C_1$-$C_{18}$ pouvant être substitué par un ou plusieurs radicaux choisis parmi alkyle en $C_1$-$C_4$, cycloalkyle en $C_5$-$C_{12}$ ou un reste aryle. Ces composés sont connus en soi et décrits dans les demandes 5 US 5237 071, US 5 166 355, GB-A-2 303 549, DE 197 26 184 et EP-A-893 119 (faisant partie intégrante de la description).

Dans la formule (I) définie ci-dessus : les groupes alkyle en $C_1$-$C_{18}$ peuvent être linéaires ou ramifiés et sont par exemple méthyle, éthyle, n-propyle, isopropyle, nbutyle, isobutyle, tert-butyle, tert-octyle, n-amyle, n-hexyle, n-heptyle, n-octyle, isooctyle, n-nonyle, n-décyle, n-undécyle, n-dodécyle, tétradécyle, hexydécyle, ou octadécyle ; les groupes cycloalkyle en $C_5$-$C_{12}$ sont par exemple cyclopentyle, cyclohexyle, cyclooctyle ; les groupes aryle sont par exemple phényle, benzyle.

Parmi les composés de formule (IV), on peut citer ceux de structure suivante :

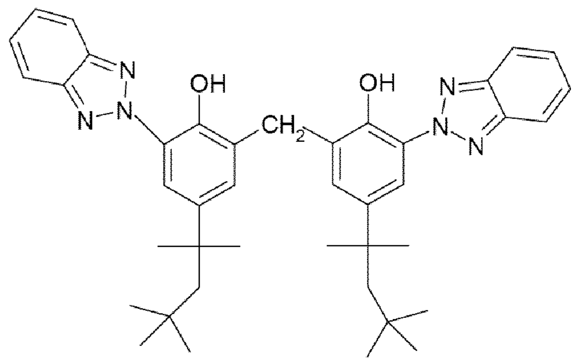

composé (a)

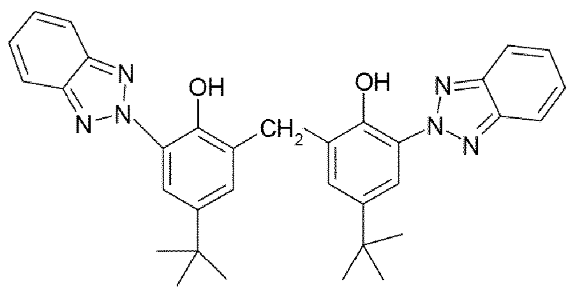

composé (b)

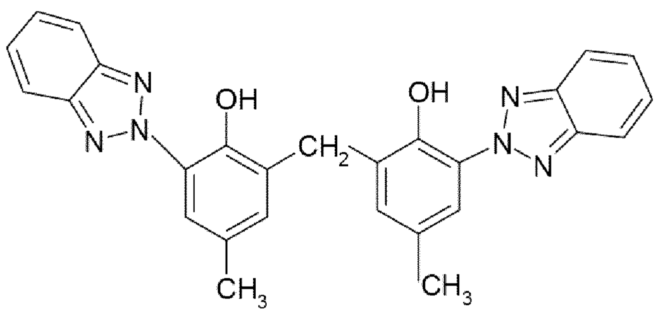

composé (c)

Le composé (a) de nomenclature 2,2'-méthylène-bis-[6-(2H-benzotriazol-2-yl)-4-(1,1,3,3-tetraméthylbutyl)phénol] est notamment vendu sous le nom commercial MIXXIM BB/200® par la société FAIRMOUNT CHEMICAL.

Le composé (c) de nomenclature 2,2'-méthylène-bis-[6-(2H-benzotriazol-2-yl)-4-(methyl)phénol] est notamment vendu sous forme solide sous le nom commercial MIXXIM BB/200® par la société FAIRMOUNT CHEMICAL.

D/ Amides vinyliques

Parmi les filtres organiques insolubles du type amide vinylique, on peut citer par exemple les composés de formule suivante qui sont décrits dans la demande WO95/22959 (faisant partie intégrante du contenu de la description) :

$$T_{12}\text{-}(Y)r\text{-}C(=O)\text{-}C(T_{13})=C(T_{14})\text{-}N(T_{15})(T_{16}) \qquad (V)$$

dans laquelle $T_{12}$ est un radical alkyle en $C_1$-$C_{18}$, de préférence en $C_1$-$C_5$ ou un groupe phényle éventuellement substitué par un, deux ou trois radicaux choisis parmi OH, alkyle en $C_1$-$C_{18}$, alcoxy en $C_1$-$C_8$, ou un groupe -C(=O)-O$T_{17}$ où $T_{17}$ est un alkyle en $C_1$-$C_{18}$ ; $T_{13}$, $T_{14}$, $T_{15}$ et $T_{16}$ identiques ou différents désignent un radical alkyle en $C_1$-$C_{18}$, de préférence en $C_1$-$C_5$ ou un atome d'hydrogène ; Y est N ou O et r vaut 0 ou 1.

Parmi ces composés, on citera notamment:

- la 4-octylamino-3-pentèn-2-one ;

- l'éthyl-3-octylamino-2-buténoate ;
- la 3-octylamino-1-phényl-2-butèn-1-one
- la 3-dodecylamino-1-phenyl-2-buten-1-one.

E/ Cinnamamides

Parmi les filtres organiques insolubles du type cinnamamide conformes à l'invention, on peut citer également les composés tels que décrits dans la demande WO95/22959 (faisant partie intégrante du contenu de la description) et répondant à la structure suivante :

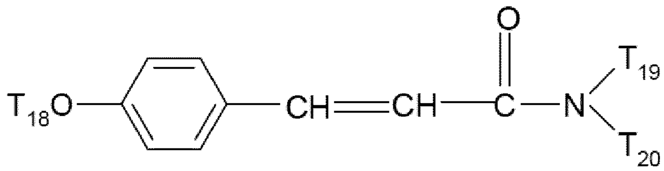
(VI)

dans laquelle $OT_{18}$ est un radical hydroxy ou alcoxy en $C_1$-$C_4$, de préférence méthoxy ou éthoxy ; $T_{19}$ est hydrogène, alkyle en $C_1$-$C_4$, de préférence méthyle ou éthyle ; $T_{20}$ est un groupe -$(CONH)_s$-phényle ou s vaut 0 ou 1 et le groupe phényle peut être substitué par un, deux ou trois groupes choisis parmi OH, alkyle en $C_1$-$C_{18}$, alcoxy en $C_1$-$C_8$, ou un groupe -C(=O)-$OT_{21}$ où $T_{21}$ est un alkyle en $C_1$-$C_{18}$ et plus préférentiellement $T_{21}$ est un groupe phényle, 4-méthoxyphényle ou phénylaminocarbonyle.

On peut également citer les dimères cinnamamides tels que ceux décrits dans le brevet US 5888481 comme par exemple le composé de structure :

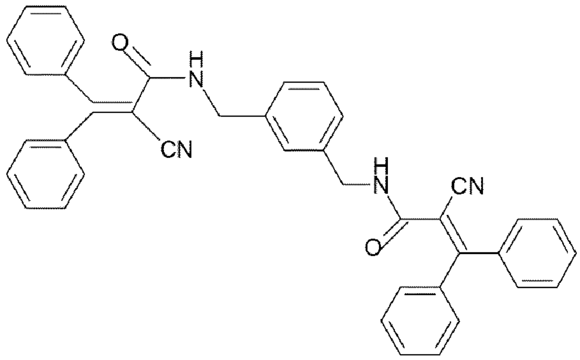

F/ Benzazoles

Parmi les filtres organiques insolubles du type benzazole, on peut citer ceux répondant à l'une des formules suivantes :

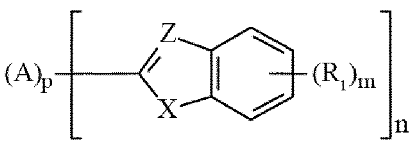
(VII)

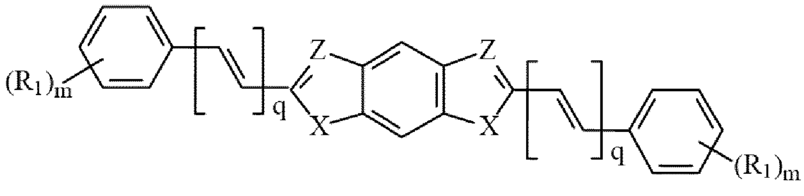
(VIII)

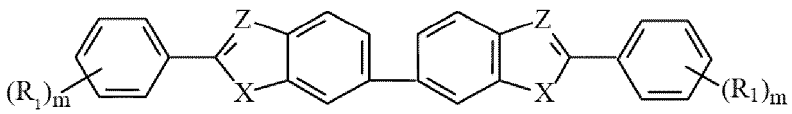

(IX)

dans lesquelles chacun des symboles X représente indépendamment un atome d'oxygène ou de soufre ou un groupe $NR_2$, chacun des symboles Z représente indépendamment un atome d'azote ou un groupe CH,
chacun des symboles $R_1$ représente indépendamment un groupe OH, un atome d'halogène, un groupe alkyle en $C_{1-8}$, linéaire ou ramifié, contenant éventuellement un atome de silicium, ou un groupe alcoxy en $C_{1-8}$, linéaire ou ramifié,
chacun des nombres m vaut indépendamment 0, 1 ou 2,
n représente un nombre entier compris entre 1 et 4 inclus,
p est égal à 0 ou 1,
chacun des nombres q est égal indépendamment à 0 ou 1,
chacun des symboles R2 représente indépendamment un atome d'hydrogène, un groupe benzyle ou alkyle en $C_{1-8}$, linéaire ou ramifié, contenant éventuellement un atome de silicium,

A représente un radical de valence n choisi parmi ceux de formules :

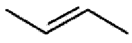

(a)

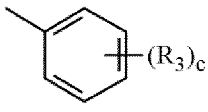

(b)

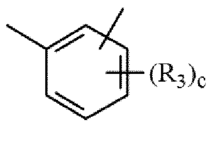

(c)

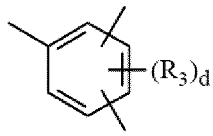

(d)

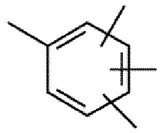

(e)

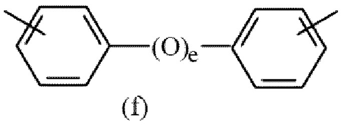

(f)

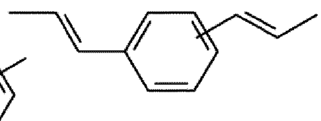

(g)

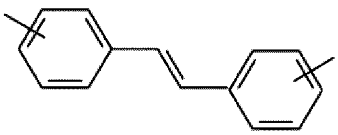

(h)

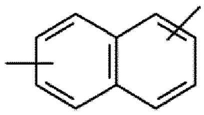

(i)

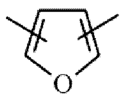

(j)

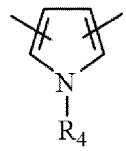

(k)

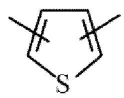

(l)

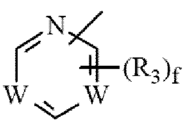

(m)

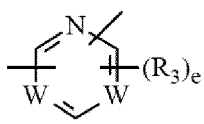

(n)

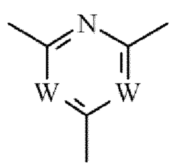

(o)

dans lesquelles chacun des symboles $R_3$ représente indépendamment un atome d'halogène ou un groupe alkyle ou alcoxy en $C_{1-4}$, linéaire ou ramifié, ou hydroxy, $R_4$ représente un atome d'hydrogène ou un groupe alkyle en $C_{1-4}$, linéaire ou ramifié, c = 0 - 4, d = 0 - 3, e = 0 ou 1, et f = 0 - 2.

Ces composés sont notamment décrits dans les brevets DE 676 103 et CH 350 763, le brevet US 5 501 850, le brevet US 5 961 960, la demande de brevet EP0669323, le brevet US 5 518 713, le brevet US 2 463 264, l'article du J. Am. Chem. Soc., 79, 5706 - 5708, 1957, l'article du J. Am. Chem. Soc., 82, 609 - 5 611, 1960, la demande de brevet EP0921126, la demande de brevet EP712855.

A titre d'exemples de composés préférés de formule (VII) de la famille des 2-arylbenzazoles, on peut mentionner le 2-benzoxazol-2-yl-4-méthylphénol, le 2-(1H-benzimidazol-2-yl)-4-méthoxyphénol ou le 2-benzothiazol-2-ylphénol, ces composés pouvant être préparés par exemple selon les procédés décrits dans le brevet CH 350 763.

A titre d'exemples de composés préférés de formule (VII) de la famille des benzimidazolylbenzazoles, on citera le

2,2'-bis-benzimidazole, le 5,5',6,6'-tétraméthyl-2,2'-bis-benzimidazole, le 5,5'-diméthyl-2,2'-bis-benzimidazole, le 6-méthoxy-2,2'-bis-benzimidazole, le 2-(1H-benzimidazol-2-yl)-benzothiazole, le 2-(1H-benzimidazol-2-yl)-benzoxazole et le N,N'-diméthyl-2,2'-bis-benzimidazole, ces composés pouvant être préparés selon les modes opératoires décrits dans les brevets US 5 961 960 et US 2 463 264.

A titre d'exemples de composés préférés de formule (VII) de la famille des phénylène-benzazoles, on citera le 1,4-phénylène-bis-(2-benzoxazolyle), le 1,4-phénylène-bis-(2-benzimidazolyle), le 1,3-phénylène-bis-(2-benzoxazolyle), le 1,2-phénylène-bis-(2-benzoxazolyle), le 1,2-phénylène-bis-(benzimidazolyle), le 1,4-phénylène-bis-(N-2-éthylhexyl-2-benzimidazolyle) et le 1,4-phénylène-bis-(Ntriméthylsilylméthyl-2-benzimidazolyle), ces composés pouvant être préparés selon les modes opératoires décrits dans le brevet US 2 463 264 et dans les publications J. Am. Chem. Soc., 82, 609 (1960) et J. Am. Chem. Soc., 79, 5706 -5708 (1957).

A titre d'exemples de composés préférés de formule (VII) de la famille des benzofuranyl-benzoxazoles, on citera le 2-(2-benzofuranyl)-benzoxazole, le 2-(benzofuranyl)-5-méthylbenzoxazole et le 2-(3-méthyl-2-benzofuranyle)-benzoxazole, ces composés pouvant être préparés selon les modes opératoires décrits dans le brevet US 5 518 713.

Comme composés préférés de formule (VIII), on peut citer par exemple le 2,6-diphényl-1,7-dihydro-benzo[1,2-d;4,5-d']-di-imidazole correspondant à la formule

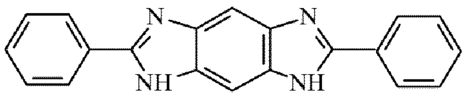

ou le 2,6-distyryl-1,7-dihydro-benzo[1,2-d ; 4,5-d']-di-imidazole ou encore le 2,6-di(p-tert-butylstyryl)-1,7-dihydrobenzo [1,2-d ; 4,5-d']-di-imidazole, qui peuvent être préparés selon la demande EP 0 669 323.

Comme composé préféré de formule (IX), on peut citer le 5,5'-bis-[(phényl-2)-benzimidazole] de formule :

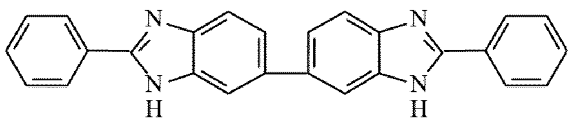

dont la préparation est décrite dans J. Chim. Phys., 64, 1602 (1967).

Parmi ces composés organiques insolubles filtrant le rayonnement UV, on préfère tout particulièrement le 2-(1H-benzimidazol-2-yl)benzoxazole, 5 ole, le 6-méthoxy-2,2'-bis-benzimidazole, le 2-(1H-benzimidazol-2-yl)-benzothiazole, le 1,4-phénylènebis-(2-benzoxazolyle), le 1,4-phénylène-bis-(2-benzimidazolyle), le 1,3-phénylènebis-(2-benzoxazolyle), le 1,2-phénylène-bis-(2-benzoxazolyle), le 1,2-phénylènebis-(2-benzimidazolyle) et le 1,4-phénylène-bis-(N-triméthylsilylméthyl-2-benzimidazolyle).

G/ Aryl vinylène cétones

Parmi les filtres organiques insolubles du type aryl vinylène cétone, on peut citer ceux correspondant à l'une des formules (X) et (XI) suivantes :

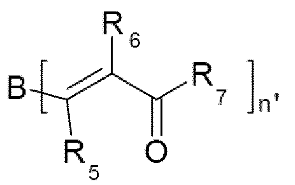

(X)

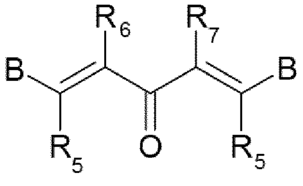

(XI)

dans lesquelles :

n' = 1 ou 2,

B, dans la formule (X) lorsque n'=1 ou dans la formule (XI), est un radical aryle choisi parmi les formules (a') à (d') suivantes, ou dans la formule (X) lorsque n'=2, est un radical choisi parmi les formules (e') à (h') suivantes :

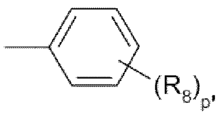
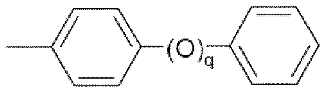
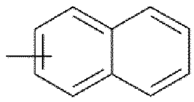
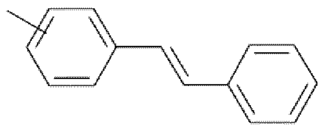

(a') (b') (c') (d')

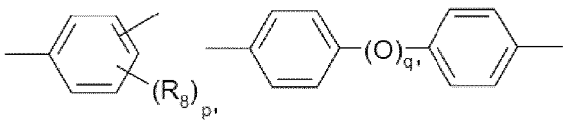
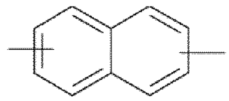
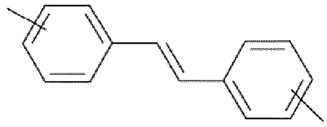

(e') (f') (g') (h')

dans lesquelles :

chacun des symboles $R_8$ représente indépendamment un groupe OH, un atome d'halogène, un groupe alkyle en $C_{1-6}$, linéaire ou ramifié et contenant éventuellement un atome de silicium, un groupe alcoxy en $C_{1-6}$, linéaire ou ramifié et contenant éventuellement un atome de silicium, un groupe alcoxycarbonyle en $C_{1-5}$, linéaire ou ramifié, ou un groupe alkylsulfonamide en $C_{1-6}$, linéaire ou ramifié et contenant éventuellement un atome de silicium ou une fonction aminoacide,
p' représente un nombre entier compris entre 0 et 4 inclus,
q' représente 0 ou 1,
$R_5$ représente l'hydrogène ou un groupe OH,
$R_6$ représente l'hydrogène, un groupe alkyle en $C_{1-6}$, linéaire ou ramifié et contenant éventuellement un atome de silicium, un groupe cyano, un groupe alkylsulfonyle en $C_{1-6}$, un groupe phénylsulfonyle,
$R_7$ représente un groupe alkyle en $C_{1-6}$, linéaire ou ramifié et contenant éventuellement un atome de silicium ou un groupe phényle pouvant former un bicycle et éventuellement substitué par un ou deux radicaux $R_4$,
ou $R_6$ et $R_7$ forment ensemble un reste hydrocarboné en $C_{2-10}$ monocyclique, bicyclique ou tricyclique, éventuellement interrompu par un ou des atomes d'azote, de soufre et d'oxygène et pouvant contenir un autre carbonyle, et éventuellement substitué par un groupe alkylsulfonamide en $C_1$-$C_8$, linéaire ou ramifié, et contenant éventuellement un atome de silicium ou une fonction aminoacide ; à condition que lorsque n'=1, $R_6$ et $R_7$ ne forment pas un noyau camphre.

A titre d'exemples de composés de formule (X) dans laquelle n' =1, insolubles, filtrant le rayonnement UV, ayant une taille moyenne de particules comprise entre 10 nm et 5 nm, on peut mentionner les familles suivantes :

- les composés du type styryl cétone tels que décrits dans la demande JP 04 134 042 telle que la 1-(3,4-diméthoxy-phényl)-4,4-diméthyl-pent-1-èn-3-one :

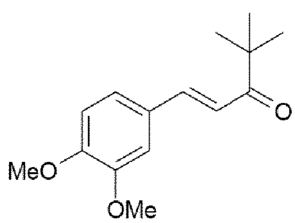

- les composés du type benzylidène cinéole tels que ceux décrits dans l'article de E. Mariani et al, 16th IFSCC Congress, New York (1990)) tel la 1,3,3-triméthyl-5-(4-méthoxy-benzylidène)-2-oxa-bicyclo[2.2.2]octan-6-one :

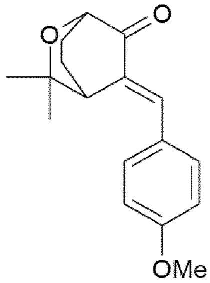

- les composés du type benzylidène chromanone tels que ceux décrits dans la demande JP 04 134 043 comme la 3-(4-méthoxy-benzylidène)-2,3,4a,8atétrahydro-chromèn-4-one :

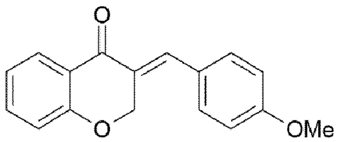

- les composés du type benzylidène thiochromanone tels que ceux décrits dans la demande JP 04 134 043 comme la 3-(4-méthoxy-benzylidène)-2,3,4a,8a-tétrahydro-chromèn-4-thione :

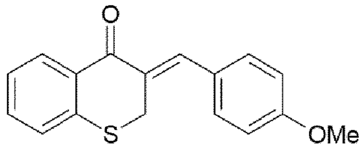

- les composés du type benzylidène quinuclidinone tels que ceux décrits dans la demande EP 0 576 974 comme la 4-méthoxy benzylidène-1-azabicyclo[2.2.2]octan-3-one :

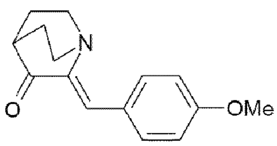

- les composés du type benzylidène cycloalcanone tels que ceux décrits dans la demande FR 2 395 023 comme les 2-(4-méthoxy-benzylidène)-cyclopentanone et 2-(4-méthoxy-benzyl-idène)-cyclohexanone :

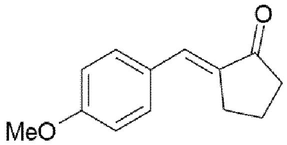

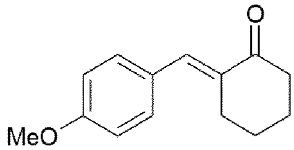

- les composés du type benzylidène hydantoïne tels que ceux décrits dans la demande JP 01 158 090 comme la 5-(3,4-diméthoxy-benzylidène)-imidazolidine-2,4-dione :

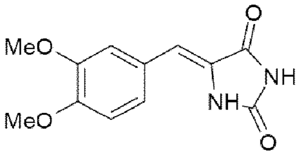

- les composés du type benzylidène indanone tels que ceux décrits dans la demande JP 04 134 043 comme la 2-(4-méthoxy-benzylidène)-indan-1-one :

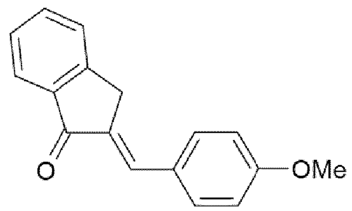

- les composés du type benzylidène tétralone tels que ceux décrits dans la demande JP 04 134 043 comme la 2-(4-méthoxy-benzylidène)-3,4-dihydro-2H-naphthalen-1-one :

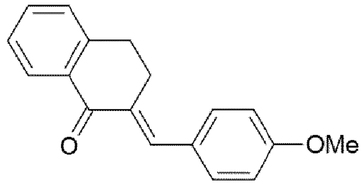

- les composés du type benzylidène furanone tels que ceux décrits dans la demande EP 0 390 683 comme la 4-(4-méthoxy-benzylidène)-2,2,5,5-tétraméthyl-dihydro-furan-3-one :

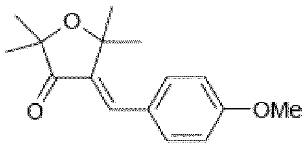

- les composés du type benzylidène benzofuranone tels que ceux décrits dans la demande JP 04 134 041 comme la 2-benzylidène-benzofuran-3-one :

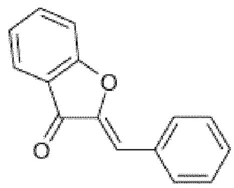

- les composés du type benzylidène indanedione telle que la 2-(3,5-di-tert-butyl-4-hydroxy-benzylidène)-indan-1,3-dione :

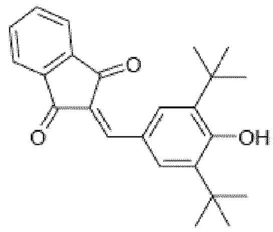

- les composés du type benzylidène benzothiofuranone comme ceux décrits dans la demande JP 04,134,043) comme la 2-benzylidène-benzo[b]thiophen-3-one :

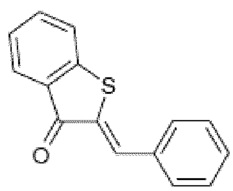

- les composés du type benzylidène barbiturique tels que la 5-(4-méthoxybenzylidène)-1,3-diméthyl-pyrimidine-2,4,6-trione :

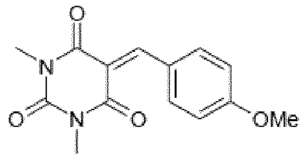

- les composés du type benzylidène pyrazolone comme la 4-(4-méthoxy benzylidène)-5-méthyl-2-phényl-2,4-dihydro-pyrazol-3-one :

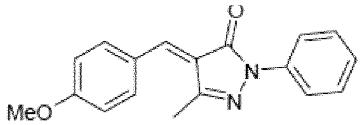

- les composés du type benzylidène imidazolone tels que la 5-(4-méthoxybenzylidène)-2-phényl-3,5-dihydro-imidazol-4-one :

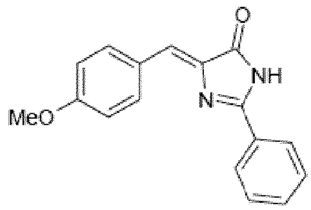

- les composés du type chalcone tels que la 1-(2-hydroxy-4-méthoxy-phényl)-3-phényl-propènone :

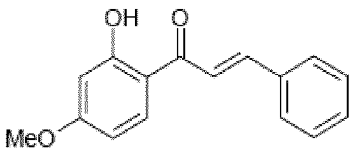

- les composés benzylidène one tels que décrits dans le document FR 2 506 156 comme la 3-hydroxy-1-(2-hydroxy-4-méthoxy-phényl)-3-phényl-propènone :

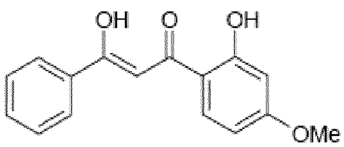

A titre d'exemples de composés de formule (X) dans laquelle n'=2 insolubles, filtrant le rayonnement UV, ayant une taille moyenne de particules comprise entre 10 nm et 5 μm, on peut mentionner les familles suivantes :

- les composés du type phénylène bis méthylidène-nor-camphre tels que décrits dans le document EP 0 693 471 comme la 1,4-phénylène-bis-{3-méthylidènebicyclo[2.2. 1]heptan-2-one} :

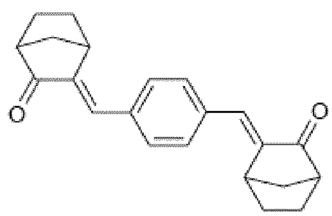

- les composés du type phénylène bis méthylidène camphre tels que décrits dans le document FR 2 528 420 comme la 1,4-phénylène-bis-{3-méthylidène-1,7,7-triméthyl-bicyclo [2.2.1]heptan-2-one} :

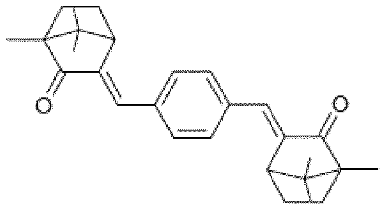

ou la 1,3-phénylène-bis-{3-méthylidène-1,7,7-triméthyl-bicyclo [2.2.1]heptan-2-one} :

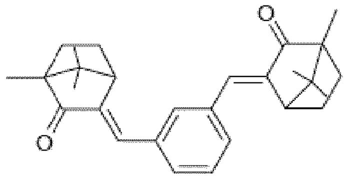

- les composés du type phénylène bis méthylidène camphre sulfonamide tels que ceux décrits dans le document FR2 529 887 comme le 1,4-phénylène-bis-3,3'-méthylidène camphre-10,10'-sulfonamide d'éthyle ou de 2-éthylhexyle :

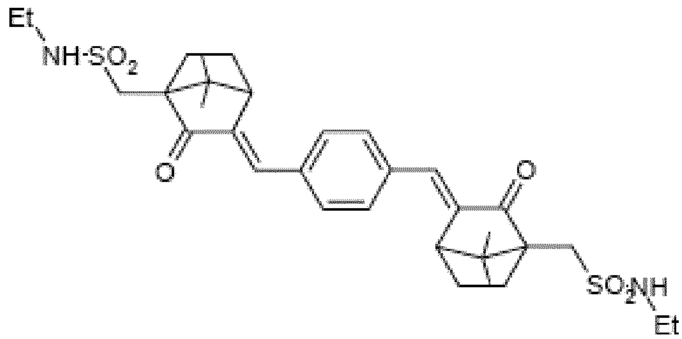

ou

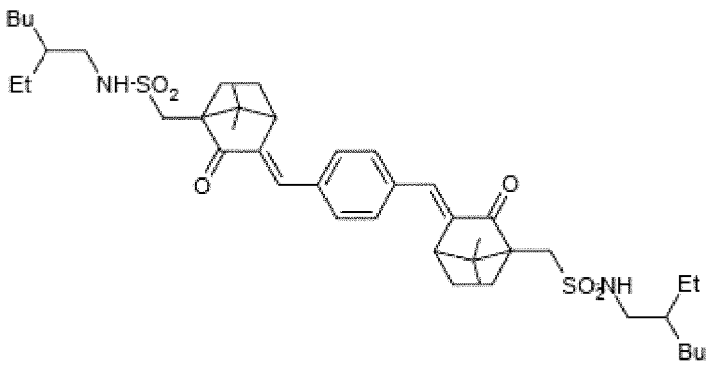

- les composés du type phénylène bis méthylidène cinéole tels que décrits dans l'article E. Mariani et al, 16th IFSCC Congress, New York (1990) comme la 1,4-phénylène-bis-{5-méthylidène-3,3-diméthyl-2-oxa-bicyclo[2.2.2]octan-6-one} :

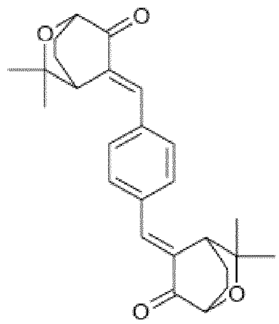

- les composés du type phénylène bis méthylidène cétotricyclodécane tels que décrits dans la demande EP 0 694 521 commel la 1,4-phénylène-bis-(octahydro-4,7-méthano-6-indèn-5-one) :

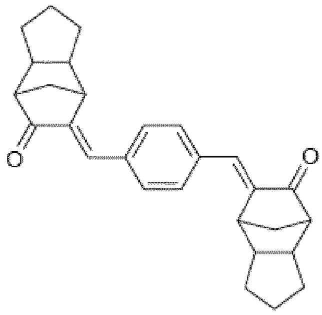

- les composés du type phénylène bis alkylène cétone tels que ceux décrits dans la demande JP 04 134 041 comme la 1,4-phénylène-bis-(4,4-diméthyl-pent-1-èn-3-one) :

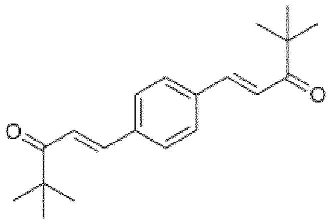

- les composés du type phénylène bis méthylidène furanone tels que décrits dans la demande FR 2 638 354 comme la 1,4-phénylène-bis-(4-méthylidène-2,2,5,5-tétraméthyl-dihydrofuran-3-one) :

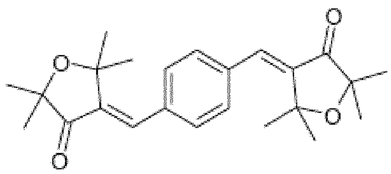

- les composés du type phénylène bis méthylidène quinuclidinone tels que ceux décrits dans la demande EP 0 714 880 comme la 1,4-phénylène-bis-{2-méthylidène-1-aza-bicyclo [2.2.2]octan-3-one} :

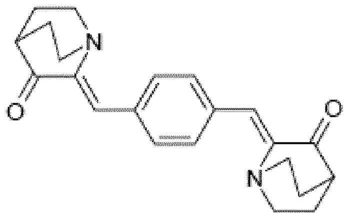

A titre de composés de formule (XI), on peut mentionner les familles suivantes

- les composés du type bis benzylidène cycloalcanone tels que la 2,5-dibenzylidène-cyclopentanone :

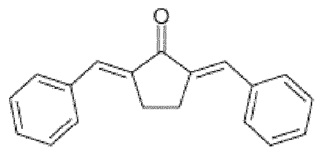

- les composés du type gamma pyrone tels que décrits dans le document JP 04 290 882 comme la 2,6-bis-(3,4-diméthoxy-phényl)-pyran-4-one :

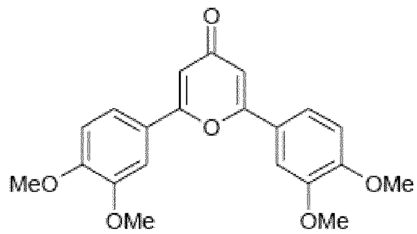

Parmi ces composés organiques insolubles filtrant le rayonnement UV du type aryl vinylène cétone, on préfère tout particulièrement les composés de formule (X) dans laquelle n'=2.

H/ Phénylène bis-benzoxazinones

Parmi les filtres organiques insolubles du type phénylène bis-benzoxazinone, on peut citer ceux répondant à la formule (XII) suivante :

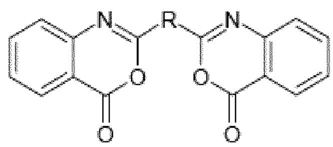

(XII)

avec R représentant un reste aromatique divalent choisi parmi les formules (e) à (h) suivantes :

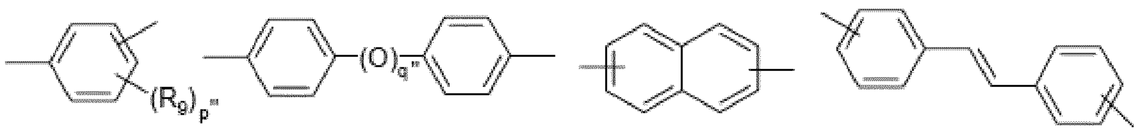

(e") (f") (g") (h")

dans lesquelles :

chacun des symboles $R_9$ représente indépendamment un groupe OH, un atome d'halogène, un groupe alkyle en $C_{1-6}$, linéaire ou ramifié et contenant éventuellement un atome de silicium, un groupe alcoxy en $C_{1-6}$, linéaire ou ramifié et contenant éventuellement un atome de silicium, un groupe alcoxycarbonyle en $C_{1-5}$, linéaire ou ramifié, ou un groupe alkylsulfonamide en $C_{1-6}$, linéaire ou ramifié et contenant éventuellement un atome de silicium ou une fonction aminoacide,
p" représente un nombre entier compris entre 0 et 4 inclus,
q" représente 0 ou 1,

A titre d'exemples de composés de formule (XII), insolubles, filtrant le rayonnement UV, ayant une taille moyenne de particules comprise entre 10 nm et 5 $\mu$m, on peut mentionner les dérivés suivants :

- le 2,2'-p-phénylène bis(3,1-benzoxazin-4-one), vendu notamment sous le nom commercial de CYASORB UV-3638® par la société CYTEC,
- le 2,2'-(4,4'-biphénylène) bis (3,1-benzoxazin-4-one),

- le 2,2'-(2,6-naphthylène) bis (3,1-benzoxazin-4-one).

I/ Dérivés amide, sulfonamide ou carbamate d'acrylonitrile

Parmi les filtres organiques insolubles du type dérivé amide, sulfonamide ou carbamate d'acrylonitrile, on peut citer ceux répondant à la formule (XIII) suivante :

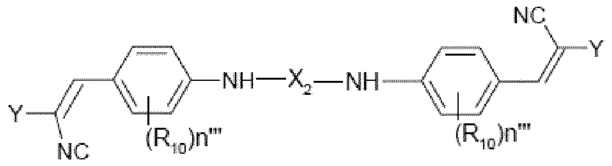

XIII)

dans laquelle :

$R_{10}$ représente un groupe alkyle en $C_1$-$C_8$, linéaire ou ramifié,
n''' vaut 0, 1 ou 2,
$X_2$ représente un radical divalent de formule -(C=O)-$R_{11}$-(C=O)-, -$SO_2$-$R_{11}$-$SO_2$- ou -(C=O)-O-$R_{11}$-O-(C=O)-,
Y représente un radical -(C=O)-$R_{12}$ ou -$SO_2R_{13}$,
$R_{11}$ représente une simple liaison ou un radical divalent alkylène en $C_1$-$C_{30}$ ou alcénylène en $C_3$-$C_{30}$, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium,
$R_{12}$ représente un radical -$OR_{14}$ ou -$NHR_{14}$,
$R_{13}$ représente un radical alkyle en $C_1$-$C_{30}$, linéaire ou ramifié, ou un noyau phényle non substitué ou substitué par des radicaux alkyle ou alcoxy en $C_1$-$C_4$,
$R_{14}$ représente un radical alkyle en $C_1$-$C_{30}$ ou alcényle en $C_3$-$C_{30}$, linéaire ou ramifié, pouvant porter un ou plusieurs substituants hydroxyle et pouvant contenir, dans la chaîne carbonée, un ou plusieurs hétéroatomes choisis parmi les atomes d'oxygène, d'azote et de silicium.

Bien que dans la formule (XIII) ci-dessus, seuls soient représentés les isomères dans lesquelles le substituant cyano est en position *cis* par rapport au substituant para-amino-phényle, cette formule doit être comprise comme englobant également les isomères *trans* correspondants; pour chacune des deux double liaisons et de façon indépendante, les substituants cyano et para-amino-phényle peuvent être en configuration *cis* ou *trans* l'un par rapport à l'autre.

A titre d'exemple, on peut citer le dimère de 2-cyano-3-[4-(acétylamino)phényl]-acrylate de 2-éthylhexyle de formule :

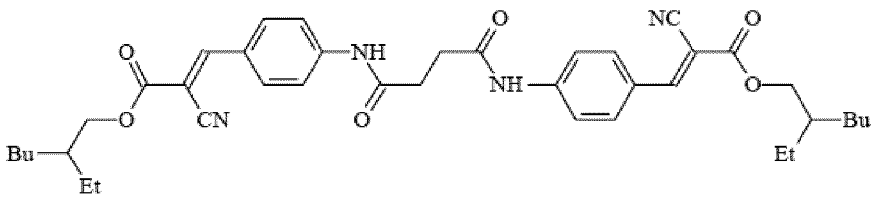

J/ Métaux polyvalents

Une autre famille particulière de filtres organiques insolubles conformes à l'invention sont les sels de métaux polyvalents (par exemple $Ca^{2+}$, $Zn^{2+}$, $Mg^{2+}$, $Ba^{2+}$, $Al^{3+}$ ou $Zr^{4+}$ ) de filtres organiques sulfoniques ou carboxyliques tels que les sels de métaux polyvalents de dérivés sulfonés de benzylidène camphre tels que ceux décrits dans la demande FR-A 2 639 347 ; les sels de métaux polyvalents de dérivés sulfonés de benzimidazole tels que ceux décrits dans la demande EP-A-893119 ; les sels de métaux 5 polyvalents de dérivés d'acide cinnamique tels que ceux décrits dans la demande JP-87 166 517.

On peut également citer les complexes de métaux ou d'ammonium ou d'ammonium substitué de filtres organiques UV-A et/ou UV-B tels que décrits dans les demandes de brevet WO93/10753, WO93/11095 et WO95/05150.

Parmi les filtres UV organiques insolubles, on peut citer également le composé 1,1'-(1,4-piperazinediyl)bis[1-[2-[4-(diethylamino) -2-hydroxybenzoyl]phenyl] -methanone (CAS 919803-06-8) de structure suivante :

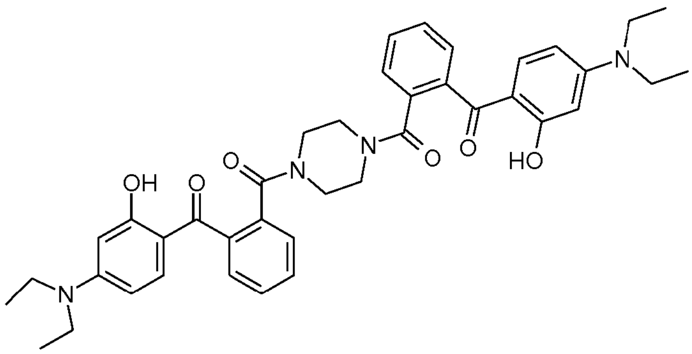

tel que décrit dans la demande WO2007/071584 ; ce composé étant avantageusement utilisé sous forme micronisée (taille moyenne de 0,02 à 2 μm) pouvant être obtenue par exemple selon le procédé de micronisation décrit dans les demandes GB-A-2 303 549 et EP-A-893119 et notamment sous forme de dispersion aqueuse.

Selon une forme particulièrement préférée de l'invention, on utilisera les filtres UV organiques insolubles choisis parmi

(i) les filtres triazines symétriques substituées par des groupes naphthalenyles ou des groupes polyphényles décrits dans le brevet US6,225,467, la demande WO2004/085412 (voir composés 6 et 9) ou le document « Symetrical Triazine Derivatives » IP.COM IPCOM000031257 Journal , INC WEST HENRIETTA, NY, US (20 septembre 2004) notamment la 2,4,6-tris(di-phenyl)-triazine et la 2,4,6-tris(ter-phenyl)-triazine qui est repris dans les demandes de brevet WO06/035000, WO06/034982, WO06/034991, WO06/035007, WO2006/034992, WO2006/034985, ces composés étant utilisés avantageusement sous forme micronisée (taille moyenne de particule de 0,02 à 3 μm) pouvant être obtenue par exemple selon le procédé de micronisation décrit dans les demandes GB-A-2 303 549 et EP-A-893119 et notamment sous forme de dispersion aqueuse;

(ii) les composés de méthylène bis-(hydroxyphényl benzotriazole) de formule (IV) suivante :

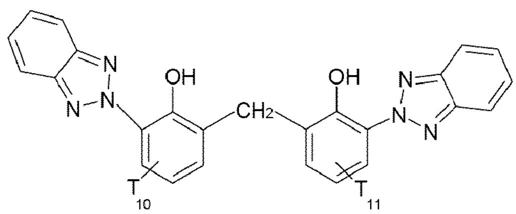

(IV)

dans laquelle les radicaux $T_{10}$ et Tu, identiques ou différents, désignent un radical alkyle en $C_1$-$C_{18}$ pouvant être substitué par un ou plusieurs radicaux choisis parmi alkyle en $C_1$-$C_4$, cycloalkyle en $C_5$-$C_{12}$ ou un reste aryle ;

(iii) et leurs mélanges.

Selon une particulièrement préférée de l'invention, les composés méthylène bis-(hydroxyphényl benzotriazole) de formule (IV) sont sous forme de dispersion aqueuse de particules ayant une taille moyenne des particules qui varie de 0,01 à 5μm et plus préférentiellement de 0,01 à 2 μm et plus particulièrement de 0,020 à 2 μm avec au moins un tensioactif de structure $C_nH_{2n+1}$ $O(C_6H_{10}O_5)_xH$ dans laquelle n est un entier de 8 à 16 et x est le degré moyen de polymérisation de l'unité ($C_6H_{10}O_5$) et varie de 1,4 à 1,6 tel que défini précédemment. Ledit tensio-actif est utilisé de préférence à une concentration de allant de 1 à 50 % en poids et plus préférentiellement de 5 à 40 % en poids par rapport au filtre benzotriazole et la quantité de filtre benzotriazole de formule (I) dans la dispersion aqueuse varie de préférence de 10 à 50 % en poids et plus préférentiellement de 30 à 50 % en poids par rapport au poids total de la dispersion.

Le diamètre moyen des particules étant mesuré par un analyseur de distribution de taille de particules du type Culter N4 PLUS® fabriqué par Bechman Coulter INC.

Selon une forme particulièrement préférée de l'invention, les composés méthylène bis-(hydroxyphényl benzotriazole) de formule (IV) peuvent être sous forme de dispersion aqueuse de particules ayant une taille moyenne des particules qui varie de 0,02 à 2μm et plus préférentiellement de 0,01 à 1,5 μm et plus particulièrement de 0,02 à 1 μm en présence d'au moins un mono-($C_8$-$C_{20}$)alkyl-ester de polyglycérol ayant un dégré de polymérisation de glycérol d'au moins 5 telles que les dispersions aqueuses décrites dans la demande WO2009/063392.

Comme exemple de tensioactifs mono-($C_8$-$C_{20}$)alkylesters de polyglycérol, on peut citer le caprate de décaglycéryle , le laurate de décaglycéryle, le myristate de décaglycéryle, l'oléate de décaglycéryle, le stéarate de décaglycéryle, l'isostéarate de décaglycéryle, le caprate d'hexaglycéryle, le laurate d'hexaglycéryle, le myristate d'hexaglycéryle, l'oléate d'hexaglycéryle, le stéarate d'hexaglycéryle, l'isostéarate d'hexaglycéryle, le caprate de pentaglycéryle, le laurate de pentaglycéryle, le myristate de pentaglycéryle, l'oléate de pentaglycéryle, le stéarate de pentaglycéryle, l'isostéarate de pentaglycéryle.

- On utilisera plus en particulier
- le caprate de décaglycéryle tel que les produits vendus sous les noms commerciaux suivants SUNSOFT Q10Y®, SUNSOFT Q10S®, SUNSOFT Q12Y®, SUNSOFT Q12S®, SUNSOFT M12J® par la société Taiyo Kagaku Co. Ltd., NIKKOL Decaglyn 1-L par la société Nikko Chemicals Co. Ltd, RYOTO-Polyglycerylester L-10D® et L-7D® par la société Mitsubishi-Kagaku Co. Ltd.,
- le laurate de de décaglycéryle tel que les produits vendus sous les noms commerciaux suivants SUNSOFT Q14Y®, SUNSOFT Q14S®, SUNSOFT Q12Y®, SUNSOFT Q12S®, SUNSOFT M12J® par la société Taiyo Kagaku Co. Ltd., NIKKOL Decaglyn 1-M® par la société Nikko Chemicals Co. Ltd, RYOTO-Polyglycerylester M-10D et M-7D par la société Mitsubishi-Kagaku Co. Ltd.,
- le stéarate de décaglycéryle tel que les produits vendus sous les noms commerciaux suivants SUNSOFT Q18Y®, SUNSOFT Q18S®, SUNSOFT Q12Y®, SUNSOFT Q12S®, SUNSOFT M12J® par la société Taiyo Kagaku Co. Ltd., NIKKOL Decaglyn 1-SV par la société Nikko Chemicals Co. Ltd, RYOTO-Polyglycerylester S-15D® par la société Mitsubishi-Kagaku Co. Ltd.,
- le caprate d'hexagléryle tel que les produits vendus sous les noms commerciaux suivants NIKKOL Hexaglyn 1-L® par la société Nikko Chemicals Co. Ltd, GLYSURF 6ML par la société Aoki Oil Industrial Co. Ltd., UNIGLY GL-106® par la société Nippon Oil & Fats Co. Ltd.,
- le myristate d'hexaglycéryle tel que les produits vendus sous les noms commerciaux suivants NIKKOL Hexaglyn 1-M®, NIKKOL Hexaglyn 1-OV® par la société Nikko Chemicals Co. Ltd, GLYSURF 6ML® par la société Aoki Oil Industrial Co. Ltd., UNIGLY GL-106 par la société Nippon Oil & Fats Co. Ltd.,
- le stéarate d'hexaglycéryle tel que les produits vendus sous les noms commerciaux suivants NIKKOL Hexaglyn 1-M®, NIKKOL Hexaglyn 1-SV® par la société Nikko Chemicals Co. Ltd, EMALEXMSG-6K® par la société Nihon-Emulsion Co. Ltd., UNIGLY GL-106 par la société Nippon Oil & Fats Co. Ltd.,
- l'isostéarate d'hexaglycéryle tel que les produits vendus sous les noms commerciaux suivants MATSUMATE MI-610® par la société Matsumoto Fine Chemical Co. Ltd,
- le caprate de pentaglycéryle tel que les produits vendus sous les noms commerciaux suivants SUNSOFT A10E®, par la société Taiyo Kagaku Co. Ltd.,
- le laurate de pentaglycéryle tel que les produits vendus sous les noms commerciaux suivants SUNSOFT A12E®, SUNSOFT A121E®, par la société Taiyo Kagaku Co. Ltd.,
- le myristate de pentaglycéryle tel que les produits vendus sous les noms commerciaux suivants SUNSOFT A14E®, SUNSOFT A141E®, par la société Taiyo Kagaku Co. Ltd.,
- l'oléate de pentaglycéryle tel que les produits vendus sous les noms commerciaux suivants SUNSOFT A17E®, SUNSOFT A171E®, par la société Taiyo Kagaku Co. Ltd.,
- le stéarate de pentaglycéryle tel que les produits vendus sous les noms commerciaux suivants SUNSOFT A18E®, SUNSOFT A181E®, par la société Taiyo Kagaku Co. Ltd.,

Parmi ces tensioactifs, on préfère utiliser ceux ayant un HLB supérieur ou égal à 14,5 et plus préférentiellement supérieur ou égal à 15. Comme exemples de tensioactifs mono-($C_8$-$C_{20}$)alkylesters de polyglycérol ayant un degré de polymérisation ayant un dégré de polymérisation de glycérol d'au moins 5 ayant un HLB supérieur ou égal à 14,5, on peut citer le caprate de décaglycéryle , le laurate de décaglycéryle, le myristate de décaglycéryle, l'oléate de décaglycéryle, le stéarate de décaglycéryle, l'isostéarate de décaglycéryle, le laurate d'hexaglycéryle, le caprate de pentaglycéryle, le laurate de pentaglycéryle, le myristate de pentaglycéryle, l'oléate de pentaglycéryle, le stéarate de pentaglycéryle. Comme exemples de tensioactifs mono-($C_8$-$C_{20}$)alkylesters de polyglycérol ayant un degré de polymérisation ayant un dégré de polymérisation de glycérol d'au moins 5 ayant un HLB supérieur ou égal à 15, on peut citer le caprate de décaglycéryle , le laurate de décaglycéryle.

La quantité de composé méthylène bis-(hydroxyphényl benzotriazole) de formule (IV) dans la dispersion aqueuse varie de préférence de 10 à 50 % en poids et plus préférentiellement de 30 à 50 % en poids par rapport au poids total de la dispersion.

De façon préférentielle, le rapport en poids composé méthylène bis-(hydroxyphényl benzotriazole) / mono-($C_8$-$C_{20}$)alkylester de polyglycérol varie de 0,05 à 0,5 et plus préférentiellement de 0,1 à 0,3.

On utilisera plus préférentiellement dans ces dispersions aqueuses, comme composé méthylène bis-(hydroxyphényl benzotriazole) de formule (IV), le composé 2,2'-méthylène-bis-[6-(2H-benzotriazol-2-yl)-4-(1,1,3,3-tetraméthylbutyl)phénol] de structure :

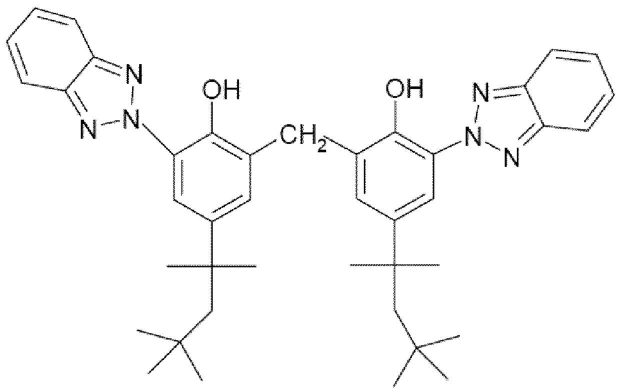

composé (a)

tel que le produit commercial vendu sous le nom TINOSORB M® par BASF qui est une dispersion aqueuse comprenant du decylglucoside, de la gomme de xanthane et du propylèneglycol (Nom INCI : Methylene Bis-Benzotriazolyl Tetramethylbutylphenol (and) Aqua (and) Decyl Glucoside (and) Propylene Glycol (and) Xanthan Gum).

Le ou les filtres UV organiques insolubles de l'invention sont présents à une concentration en matière active allant de préférence de 0,1 et 30 % en poids environ, et plus particulièrement de 0,5 à 20 % en poids par rapport au poids total de la composition.

## B/ FILTRES UV INORGANIQUES INSOLUBLES

Les filtres UV inorganiques utilisés conformément à la présente invention sont des pigments d'oxyde métallique. Plus préférentiellement, les filtres UV inorganiques de l'invention sont des particules d'oxyde métallique ayant une taille moyenne de particule élémentaire inférieure ou égale à 0,50 µm, plus préférentiellement comprise entre 0,005 et 0,50 µm et encore plus préférentiellement comprise entre 0,01 et 0,2 µm, encore mieux entre 0,01 et 0,1 µm, et plus particulièrement préférentiellement entre 0,015 et 0,05 µm.

Par « taille élémentaire », on entend la taille de particules non agrégées.

Ils peuvent être notamment choisis parmi les oxydes de titane, de zinc, de fer, de zirconium, de cérium ou leurs mélanges.

De tels pigments d'oxydes métalliques, enrobés ou non enrobés sont en particulier décrits dans la demande de brevet EP-A- 0 518 773. A titre de pigments commerciaux on peut mentionner notamment les produits vendus les sociétés SACHTLEBEN PIGMENTS, TAYCA, MERCK ET DEGUSSA.

Les pigments d'oxydes métalliques peuvent être enrobés ou non enrobés.

Les pigments enrobés sont des pigments qui ont subi un ou plusieurs traitements de surface de nature chimique, électronique, mécanochimique et/ou mécanique avec des composés tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les pigments enrobés sont plus particulièrement des oxydes de titane enrobés :

- de silice tels que le produit « SUNVEIL » de la société IKEDA,
- de silice et d'oxyde de fer tels que le produit « SUNVEIL F » de la société IKEDA,
- de silice et d'alumine tels que les produits « MICROTITANIUM DIOXIDE MT 500 SA » et « MICROTITANIUM DIOXIDE MT 100 SA » de la société TAYCA, « TIOVEIL » de la société TIOXIDE,
- d'alumine tels que les produits « TIPAQUE TTO-55 (B) » et « TIPAQUE TTO-55 (A) » de la société ISHIHARA, et « UVT 14/4 » de la société SACHTLEBEN PIGMENTS,
- d'alumine et de stéarate d'aluminium tels que les produits «MICROTITANIUM DIOXIDE MT 100 T, MT 100 TX, MT 100 Z, MT-01 » de la société TAYCA, les produits « Solaveil CT-10 W » et « Solaveil CT 100 » de la société UNIQEMA et le produit « Eusolex T-AVO » de la société MERCK,
- de silice, d'alumine et d'acide alginique tel que le produit « MT-100 AQ » de la société TAYCA,
- d'alumine et de laurate d'aluminium tel que le produit « MICROTITANIUM DIOXIDE MT 100 S » de la société TAYCA,
- d'oxyde de fer et de stéarate de fer tels que le produit « MICROTITANIUM DIOXIDE MT 100 F » de la société TAYCA,
- d'oxyde de zinc et de stéarate de zinc tels que le produit « BR 351 » de la société TAYCA,
- de silice et d'alumine et traités par une silicone tels que les produits « MICROTITANIUM DIOXIDE MT 600 SAS », « MICROTITANIUM DIOXIDE MT 500 SAS » ou «MICROTITANIUM DIOXIDE MT 100 SAS » de la société TAYCA,

- de silice, d'alumine, de stéarate d'aluminium et traités par une silicone tels que le produit « STT-30-DS » de la société TITAN KOGYO,
- de silice et traité par une silicone tel que le produit « UV-TITAN X 195 » de la société SACHTLEBEN PIGMENTS,
- d'alumine et traités par une silicone tels que les produits « TIPAQUE TTO-55 (S) » de la société ISHIHARA, ou « UV TITAN M 262 » de la société SACHTLEBEN PIGMENTS,
- de triéthanolamine tels que le produit « STT-65-S » de la société TITAN KOGYO,
- d'acide stéarique tels que le produit « TIPAQUE TTO-55 (C) » de la société ISHIHARA, d'hexamétaphosphate de sodium tels que le produit « MICROTITANIUM DIOXIDE MT 150 W » de la société TAYCA.
- le $TiO_2$ traité par l'octyl triméthyl silane vendu notamment sous la dénomination commerciale « T 805 » par la société DEGUSSA SILICES,
- le $TiO_2$ traité par un polydiméthylsiloxane vendu notamment sous la dénomination commerciale « 70250 Cardre UF TiO2SI3 » par la société CARDRE,
- le $TiO_2$ anatase/rutile traité par un polydiméthylhydrogénosiloxane vendu notamment sous la dénomination commerciale « MICRO TITANIUM DIOXYDE USP GRADE HYDROPHOBIC » par la société COLOR TECHNIQUES.

On peut également citer les pigments de $TiO_2$ dopés par au moins un métal de transition tel que le fer, le zinc, le manganèse et plus particulièrement le manganèse. De préférence lesdits pigments dopés sont sous forme de dispersion huileuse. L'huile présente dans la dispersion huileuse est de préférence choisie parmi les triglycérides dont ceux des acides capriques/capryliques. La dispersion huileuse de particules d'oxyde de titane peut comporter en plus un ou plusieurs agents dispersants comme par exemple un ester de sorbitan comme l'isostéarate de sorbitan, un ester d'acide gras et de glycérol polyoxyalkyléné comme le TRI-PPG3 MYRISTYLETHER CITRATE et le POLYGLYCERYL-3 POLYRICINOLEATE. De préférence, la dispersion huileuse de particules d'oxyde de titane comporte au moins un agent dispersant choisi parmi les esters d'acide gras et de glycérol polyoxyalkyléné. On peut citer plus particulièrement la dispersion huileuse de particules de $TiO_2$ dopées au manganèse dans le triglycéride d'acide caprique/caprylique en présence de TRI-PPG-3 MYRISTYLETHER CITRATE et le POLYGLYCERYL-3-POLYRICINOLEATE et SORBITAN ISOSTERATE de nom INCI : TITANIUM DIOXIDE (and) TRI-PPG-3 MYRISTYLETHER CITRATE (and) POLYGLYCERYL-3 RICINOLEATE (and) SORBITAN ISOSTEARATE comme le produit vendu notamment sous le nom commercial OPTISOL TD50 par la société CRODA.

Les pigments d'oxyde de titane non enrobés sont par exemple vendus par la société TAYCA sous les dénominations commerciales « MICROTITANIUM DIOXIDE MT 500 B » ou « MICROTITANIUM DIOXIDE MT600 B », par la société DEGUSSA sous la dénomination « P 25 », par la société WACKHER sous la dénomination « Oxyde de titane transparent PW », par la société MIYOSHI KASEI sous la dénomination « UFTR », par la société TOMEN sous la dénomination « ITS » et par la société TIOXIDE sous la dénomination « TIOVEIL AQ ».

Les pigments d'oxyde de zinc non enrobés, sont par exemple :

- ceux commercialisés notamment sous la dénomination « Z-cote » par la société Sunsmart ;
- ceux commercialisés notamment sous la dénomination « Nanox » par la société Elementis ;
- ceux commercialisés notamment sous la dénomination « Nanogard WCD 2025 » par la société Nanophase Technologies ;

Les pigments d'oxyde de zinc enrobés sont par exemple :

- ceux commercialisés notamment sous la dénomination « Oxide zinc CS-5 » par la société Toshibi (ZnO enrobé par polymethylhydrogenesiloxane) ;
- ceux commercialisés notamment sous la dénomination « Nanogard Zinc Oxide FN » par la société Nanophase Technologies (en dispersion à 40 % dans le Finsolv TN, benzoate d'alcools en $C_{12}$-$C_{15}$) ;
- ceux commercialisés notamment sous la dénomination « DAITOPERSION Zn-30 » et « DAITOPERSION Zn-50 » par la société Daito (dispersions dans cyclopolyméthylsiloxane /polydiméthylsiloxane oxyéthyléné, contenant 30 % ou 50 % d'oxydes de zinc enrobés par la silice et le polyméthylhydrogènesiloxane) ;
- ceux commercialisés notamment sous la dénomination « NFD Ultrafine ZnO » par la société Daikin (ZnO enrobé par phosphate de perfluoroalkyle et copolymère à base de perfluoroalkyléthyle en dispersion dans du cyclopentasiloxane) ;
- ceux commercialisés notamment sous la dénomination « SPD-Z1 » par la société Shin-Etsu (ZnO enrobé par polymère acrylique greffé silicone, dispersé dans cyclodiméthylsiloxane) ;
- ceux commercialisés notamment sous la dénomination « Escalol Z100 » par la société ISP (ZnO traité alumine et dispersé dans le mélange methoxycinnamate d'ethylhexyle / copolymère PVP-hexadecene / methicone) ;
- ceux commercialisés notamment sous la dénomination « Fuji ZnO-SMS-10 » par la société Fuji Pigment (ZnO enrobé silice et polymethylsilsesquioxane) ;

- ceux commercialisés notamment sous la dénomination « Nanox Gel TN » par la société Elementis (ZnO dispersé à 55 % dans du benzoate d'alcools en $C_{12}$-$C_{15}$ avec polycondensat d'acide hydroxystéarique).

Les pigments d'oxyde de cérium non enrobés peuvent être par exemple ceux vendus sous la dénomination « COLLOÏDAL CERIUM OXIDE » par la société RHONE POULENC.

Les pigments d'oxyde de fer non enrobés sont par exemple vendus par la société ARNAUD sous les dénominations « NANOGARD WCD 2002 (FE 45B) », « NANOGARD IRON FE 45 BL AQ », « NANOGARD FE 45R AQ », « NANOGARD WCD 2006 (FE 45R) », ou par la société MITSUBISHI sous la dénomination « TY-220 ».

Les pigments d'oxyde de fer enrobés sont par exemple vendus par la société ARNAUD sous les dénominations «NANOGARD WCD 2008 (FE 45B FN) », « NANOGARD WCD 2009 (FE 45B 556) », « NANOGARD FE 45 BL 345 », « NANOGARD FE 45 BL », ou par la société BASF sous la dénomination « OXYDE DE FER TRANSPARENT ».

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination «SUNVEIL A», ainsi que le mélange de dioxyde de titane et de dioxyde de zinc enrobé d'alumine, de silice et de silicone tel que le produit «M 261» vendu par la société SACHTLEBEN PIGMENTS ou enrobé d'alumine, de silice et de glycérine tel que le produit « M 211 » vendu par la société SACHTLEBEN PIGMENTS.

Selon l'invention, les pigments d'oxyde de titane, enrobés ou non enrobés, sont particulièrement préférés.

Les filtres UV insolubles inorganiques de l'invention sont de préférence présents dans les compositions selon l'invention à une teneur allant de 0,1 % à 50 % en poids, plus particulièrement de 0,1 à 40 % en poids, et en particulier de 0,5 à 30 % en poids par rapport au poids total de la composition.

Comme exposé précédemment, selon un mode de réalisation particulièrement préféré, le ou les filtres UV sont choisis parmi les filtres UV organiques hydrosolubles, les filtres UV organiques liposolubles, et leurs mélanges.

Selon une forme particulière de l'invention, la composition comprend

- au moins une phase aqueuse gélifiée par au moins un agent gélifiant hydrophile non amylacé choisi parmi les copolymères d'acide 2-acrylamido 2-méthylpropane sulfonique (AMPS®), réticulés et ou neutralisés et plus particulièrement un copolymère d'AMPS® et d'hydroxyéthyl acrylate
- au moins une phase huileuse gélifiée par au moins un agent gélifiant lipophile choisi parmi les hectorites modifiées par un chlorure d'ammonium en C10 à C22, et plus particulièrement une hectorite modifiée par du chlorure de di-stéaryl diméthyl ammonium.

lesdites phases y formant un mélange macroscopiquement homogène ;
ladite composition comprenant en outre au moins un filtre UV choisi parmi les filtres UV organiques hydrosolubles, les filtres UV organiques liposolubles, ainsi que leurs mélanges et de préférence les filtres UV organiques liposolubles.

## Phase aqueuse

La phase aqueuse d'une composition selon l'invention comprend de l'eau et éventuellement un solvant hydrosoluble.

Par « solvant hydrosoluble », on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans la composition de l'invention peuvent en outre être volatils.

Parmi les solvants hydrosolubles pouvant être utilisés dans la composition conforme à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$ et $C_4$ et les aldéhydes en $C_2$-$C_4$.

La phase aqueuse (eau et éventuellement le solvant miscible à l'eau) peut être présente dans la composition en une teneur allant de 5 % à 95 %, mieux de 30 % à 80 % en poids, de préférence de 40 % à 75 % en poids, par rapport au poids total de ladite composition.

Selon une autre variante de réalisation, la phase aqueuse d'une composition selon l'invention peut comprendre au moins un polyol en $C_2$-$C_{32}$.

Par « *polyol* », il faut comprendre, au sens de la présente invention, toute molécule organique comportant au moins deux groupements hydroxyle libres.

De préférence, un polyol conforme à la présente invention est présent sous forme liquide à température ambiante.

Un polyol convenant à l'invention peut être un composé de type alkyle, linéaire, ramifié ou cyclique, saturé ou insaturé, portant sur la chaîne alkyle au moins deux fonctions -OH, en particulier au moins trois fonctions -OH, et plus particulièrement au moins quatre fonctions -OH.

Les polyols convenant avantageusement pour la formulation d'une composition selon la présente invention sont ceux présentant notamment de 2 à 32 atomes de carbone, de préférence 3 à 16 atomes de carbone.

Avantageusement, le polyol peut être par exemple choisi parmi l'éthylèneglycol, le pentaérythritol, le triméthylolpropane, le propylène glycol, le 1,3 propanediol, le butylène glycol, l'isoprène glycol, le pentylène glycol, l'héxylène glycol, le glycérol, les polyglycérols, tels que les oligomères du glycérol comme le diglycérol, les polyéthylènes glycols, et leurs mélanges.

Selon un mode de réalisation préféré de l'invention, ledit polyol est choisi parmi l'éthylèneglycol, le pentaérythritol, le triméthylolpropane, le propylène glycol, le glycérol, les polyglycérols, les polyéthylènes glycols, et leurs mélanges.

Selon un mode particulier, la composition de l'invention peut comprendre au moins du propylène glycol.

Selon un autre mode particulier, la composition de l'invention peut comprendre au moins du glycérol.

**Phase huileuse**

Au sens de l'invention, une phase huileuse comprend au moins une huile.

On entend par « *huile* », tout corps gras sous forme liquide à température ambiante à pression atmosphérique.

Une phase huileuse convenant à la préparation des compositions cosmétiques selon l'invention peut comprendre des huiles hydrocarbonées, siliconées, fluorées ou non, ou leurs mélanges.

Les huiles pourront être volatiles ou non volatiles.

Elles peuvent être d'origine animale, végétale, minérale ou synthétique. Selon une variante de réalisation, les huiles D'origine siliconées sont préférées.

Au sens de la présente invention, on entend par « huile non volatile », une huile ayant une pression de vapeur inférieure à 0,13 Pa.

Au sens de la présente invention, on entend par « huile siliconée », une huile comprenant au moins un atome de silicium, et notamment au moins un groupe Si-O.

On entend par « *huile fluorée* », une huile comprenant au moins un atome de fluor.

On entend par « *huile hydrocarbonée* », une huile contenant principalement des atomes d'hydrogène et de carbone.

Les huiles peuvent éventuellement comprendre des atomes d'oxygène, d'azote, de soufre et/ou de phosphore, par exemple, sous la forme de radicaux hydroxyles ou acides.

Par « *huile volatile* », on entend, au sens de l'invention, toute huile susceptible de s'évaporer au contact de la peau en moins d'une heure, à température ambiante et pression atmosphérique. L'huile volatile est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1 300 Pa (0,01 à 10 mm de Hg).

**Huiles volatiles**

Les huiles volatiles peuvent être hydrocarbonées, ou siliconées.

On peut notamment citer parmi les huiles volatiles hydrocarbonées ayant de 8 à 16 atomes de carbone les alcanes ramifiés en $C_8$-$C_{16}$ comme les iso-alcanes (appelées aussi isoparaffines) en $C_8$-$C_{16}$, l'isododécane, l'isodécane, l'isohexadécane et par exemple les huiles vendues sous les noms commerciaux d'Isopars ou de Permetyls, les esters ramifiés en $C_8$-$C_{16}$ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isohexadécane.

On peut également citer les alcanes linéaires volatils comprenant de 8 à 16 atomes de carbone, en particulier de 10 à 15 atomes de carbone, et plus particulièrement de 11 à 13 atomes de carbone, par exemple tels que le n-dodécane ($C_{12}$) et le n-tétradécane ($C_{14}$) vendus par Sasol respectivement sous les références PARAFOL 12-97 et PARAFOL 14-97, ainsi que leurs mélanges, le mélange undécane-tridécane, les mélanges de n-undécane ($C_{11}$) et de n-tridécane ($C_{13}$) obtenus aux exemples 1 et 2 de la demande WO 2008/155059 de la Société Cognis, et leurs mélanges.

Comme huiles volatiles siliconées, on peut citer les huiles volatiles siliconées linéaires telles que l'hexamethyldisiloxane, l'octamethyltrisiloxane, le decamethyltetrasiloxane, le tetradecamethylhexasiloxane, l'hexadecamethylheptasiloxane et le dodecaméthylpentasiloxane.

Comme huiles volatiles siliconées cycliques, on peut citer l'hexamethylcyclotrisiloxane, l'octamethylcylotetrasiloxane, le decamethylcyclopentasiloxane et le dodecamethylcyclohexasiloxane.

### Huiles non volatiles

Les huiles non volatiles peuvent, notamment, être choisies parmi les huiles hydrocarbonées, fluorées et/ou les huiles siliconées non volatiles.

Comme huile hydrocarbonée non volatile, on peut notamment citer :

- les huiles hydrocarbonées d'origine animale,
- les huiles hydrocarbonées d'origine végétale, les éthers de synthèse ayant de 10 à 40 atomes de carbone, comme le dicapryl ether,
- les esters de synthèse, comme les huiles de formule $R_1COOR_2$, dans laquelle $R_1$ représente un reste d'un acide gras linéaire ou ramifié comportant de 1 à 40 atomes de carbone et $R_2$ représente une chaîne hydrocarbonée, notamment, ramifiée contenant de 1 à 40 atomes de carbone à condition que $R_1 + R_2$ soit $\geq$ 10. Les esters peuvent être, notamment, choisis parmi les esters d'alcool et d'acide gras, comme par exemple, l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate d'isopropyle, le stéarate d'octyle, les esters hydroxylés, comme le lactate d'isostéaryle, l'hydroxystéarate d'octyle, les ricinoléates d'alcools ou de polyalcools, le laurate d'hexyle, les esters de l'acide néopentanoïque, comme le néopentanoate d'isodécyle, le néopentanoate d'isotridécyle, les esters de l'acide isononanoïque, comme l'isononanoate d'isononyle, l'isononanoate d'isotridécyle,
- les esters de polyols et les esters du pentaérythritol, comme le tétrahydroxystéarate/tétraisostéarate de dipentaérythritol,
- les alcools gras liquides à température ambiante à chaîne carbonée ramifiée et/ou insaturée ayant de 12 à 26 atomes de carbone, comme le 2-octyldodécanol, l'alcool isostéarylique, l'alcool oléique,
- les acides gras supérieurs en $C_{12}$-$C_{22}$, tels que l'acide oléique, l'acide linoléique, l'acide linolénique, et leurs mélanges,
- les huiles siliconés non phénylées, comme par exemple la caprylyl méthycone, et
- les huiles siliconés phénylées, comme par exemple les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, la triméthylpentaphényltrisiloxane, et leurs mélanges ; ainsi que les mélanges de ces différentes huiles.

De préférence, une composition selon l'invention comprend des huiles siliconées volatiles et/ou non volatiles. De telles huiles siliconées sont particulièrement appréciées lorsque l'agent gélifiant lipophile est un élastomère d'organopolysiloxane.

Une composition selon l'invention peut comprendre de 1 % à 95 % en poids, mieux de 5 % à 40 % en poids en poids d'huile(s) par rapport au poids total de ladite composition.

Comme précisé ci-dessus la phase huileuse gélifiée selon l'invention peut posséder une contrainte seuil supérieure à 1,5 Pa et de préférence supérieure à 10 Pa.

La phase huileuse gélifiée selon l'invention peut posséder une contrainte seuil inférieure à 10 000 Pa de préférence inférieure à 5 000Pa.

Cette valeur de contrainte seuil traduit une texture de type gel de cette phase huileuse.

### Matières colorantes

Une composition selon l'invention peut comprendre en outre au moins une matière colorante particulaire ou non, hydrosoluble ou non, et de préférence à raison d'au moins 0,01 % en poids par rapport au poids total de la composition.

Pour des raisons évidentes, cette quantité est susceptible de varier significativement au regard de l'intensité de l'effet coloriel recherchée et de l'intensité coloriel procuré par les matières colorantes considérées et son ajustement relève clairement des compétences de l'homme de l'art.

Une composition selon l'invention peut comprendre de 0,01 % à 25 % en poids, notamment de 0,1 % à 25 % en poids, en particulier de 1 % à 20 % en poids et de préférence de 5 % à 18 % en poids de matières colorantes, par rapport au poids total de ladite composition.

Comme précisé ci-dessus, les matières colorantes convenant à l'invention peuvent être hydrosolubles mais également liposolubles.

Par « *matière colorante hydrosoluble* », au sens de l'invention, on entend tout composé généralement organique, naturel ou synthétique, soluble dans une phase aqueuse ou les solvants miscibles à l'eau et apte à colorer.

A titre de colorants hydrosolubles convenant à l'invention peuvent notamment être cités les colorants hydrosolubles synthétiques ou naturels tels que par exemple le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5, le FDC

Blue 1, la bétanine (betterave), le carmin, la chlorophylline cuivrée, le bleu de méthylène, les anthocyanines (enocianine, carotte noire, hibiscus, sureau), le caramel, la riboflavine.

Les colorants hydrosolubles sont, par exemple, le jus de betterave et le caramel.

Par « *matière colorante liposoluble* », au sens de l'invention, on entend tout composé généralement organique, naturel ou synthétique, soluble dans une phase huileuse ou les solvants miscibles à un corps gras et apte à colorer.

A titre de colorants liposolubles convenant à l'invention peuvent notamment être cités les colorants liposolubles, synthétiques ou naturels tels que par exemple, le DC Red 17, le DC Red 21, le DC Red 27, le DC Green 6, le DC Yellow 11, le DC Violet 2, le DC Orange 5, le rouge Soudan, les carotènes (le β-carotène, le lycopène), les xanthophylles (capsanthine, capsorubine, lutéine), l'huile de palme, le brun Soudan, le jaune quinoléine, le rocou, le curcumin.

Les matériaux particulaires colorants peuvent être présents à raison de 0,01 % à 25 % en poids, notamment de 0,1 % à 25 % en poids, en particulier de 1 % à 20 % en poids et de préférence de 5 % à 18 % en poids de matériaux particulaires, par rapport au poids total de la composition les contenant.

Il peut notamment s'agir de pigments, de nacres et/ou de particules à reflets métalliques.

Par « *pigments* », il faut comprendre des particules blanches ou colorées, minérales ou organiques, insolubles dans une solution aqueuse, destinées à colorer et/ou opacifier la composition les contenant.

Une composition selon l'invention peut comprendre de 0,01 % à 25 % en poids, notamment de 0,1 % à 25 % en poids, en particulier de 1 % à 25 % en poids et de préférence de 5 % à 18 % en poids de pigments, par rapport au poids total de ladite composition.

De préférence, lorsque la composition selon l'invention est une composition de maquillage, elle peut comprendre au moins 5 %, et préférentiellement au moins 3 % en poids de pigments, par rapport au poids total de ladite composition.

Les pigments peuvent être blancs ou colorés, minéraux et/ou organique.

Comme pigments minéraux utilisables dans l'invention, on peut citer les oxydes ou dioxydes de titane, de zirconium ou de cérium, ainsi que les oxydes de zinc, de fer ou de chrome, le bleu ferrique, le violet de manganèse, le bleu outremer et l'hydrate de chrome, et leurs mélanges.

Il peut également s'agir d'un pigment ayant une structure qui peut être par exemple de type séricite/oxyde de fer brun/dioxyde de titane/silice. Un tel pigment est commercialisé par exemple sous la référence Coverleaf NS ou JS par la société Chemicals And Catalysts et présente un rapport de contraste voisin de 30.

Il peut encore s'agir de pigments ayant une structure qui peut être, par exemple, de type microsphères de silice contenant de l'oxyde de fer. Un exemple de pigment présentant cette structure est celui commercialisé par la société Miyoshi sous la référence PC Ball PC-LL-100 P, ce pigment étant constitué de microsphères de silice contenant de l'oxyde de fer jaune.

De manière avantageuse, les pigments conformes à l'invention sont les oxydes de fer et/ou les dioxydes de titane.

Par « *nacres* », il faut comprendre des particules colorées de toute forme, irisées ou non, notamment produites par certains mollusques dans leur coquille ou bien synthétisées, et qui présentent un effet de couleur par interférence optique.

Une composition selon l'invention peut comprendre de 0 % à 15 % en poids de nacres, par rapport au poids total de ladite composition.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres Timica, Flamenco et Duochrome (sur base de mica) commercialisées par la société ENGELHARD, les nacres Timiron commercialisées par la société Merck, les nacres sur base de mica Prestige commercialisées par la société Eckart et les nacres sur base de mica synthétique Sunshine commercialisées par la société Sun Chemical.

Les nacres peuvent plus particulièrement posséder une couleur ou un reflet jaune, rose, rouge, bronze, orangé, brun, or et/ou cuivré.

De manière avantageuse, les nacres conformes à l'invention sont les micas recouverts de dioxyde de titane ou d'oxyde de fer ainsi que l'oxychlorure de bismuth.

Par « *particules à reflet métallique* », au sens de la présente invention, on entend tout composé dont la nature, la taille, la structure et l'état de surface lui permet de réfléchir la lumière incidente notamment de façon non iridescente.

Les particules à reflet métallique utilisables dans l'invention sont en particulier choisies parmi :

- les particules d'au moins un métal et/ou d'au moins un dérivé métallique ;

- les particules comportant un substrat, organique ou minéral, monomatière ou multimatériaux, recouvert au moins partiellement par au moins une couche à reflet métallique comprenant au moins un métal et/ou au moins un dérivé métallique ; et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « *dérivés métalliques* », on désigne des composés dérivés de métaux notamment des oxydes, des fluorures, des chlorures et des sulfures.

A titre illustratif de ces particules, on peut citer des particules d'aluminium, telles que celles commercialisées sous les dénominations Starbrite 1200 EAC® par la société Siberline et Metalure® par la société Eckart et des particules de verre recouvertes d'une couche métallique notamment celles décrites dans les documents JP-A-09188830, JP-A-10158450, JP-A-10158541, JP-A-07258460 et JP-A-05017710.

Traitement hydrophobe des matières colorantes

Les matières colorantes pulvérulentes telles que décrites précédemment peuvent être traitées en surface, totalement ou partiellement, avec un agent hydrophobe, pour les rendre plus compatibles avec la phase huileuse de la composition de l'invention, notamment pour qu'ils aient une bonne mouillabilité avec les huiles. Ainsi, ces pigments traités sont bien dispersés dans la phase huileuse.

Des pigments traités hydrophobes sont notamment décrits dans le document EP- A-1 086683.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné ; les perfluoroalkyl phosphates ; les polyoxydes d'hexafluoropropylène ; les perfluoropolyéthers ; les acides aminés ; les acides aminés N-acylés ou leurs sels ; la lécithine, le trisostéaryle titanate d'isopropyle, le sébaçate d'isostéaryle, et leurs mélanges.

Le terme alkyle mentionné dans les composés cités précédemment désigne notamment un groupe alkyle ayant de 1 à 30 atomes de carbone, de préférence ayant de 5 à 16 atomes de carbone.

Avantageusement, une composition selon l'invention peut comprendre en outre une ou plusieurs charge(s) classiquement utilisés dans les compositions de soin et/ou de maquillage.

Ces charges sont des particules incolores ou blanches, solides de toutes formes, qui se présentent sous une forme insoluble et dispersée dans le milieu de la composition.

De nature minérale ou organique, naturelle ou synthétique, elles permettent de conférer à la composition les contenant de la douceur, de la matité et de l'uniformité au maquillage. En outre, ces charges permettent avantageusement de lutter contre différentes agressions telles que le sébum ou la sueur.

A titre illustratif de ces charges, peuvent être cités le talc, le mica, la silice, le kaolin, les poudres de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, le sulfate de barium, les oxydes d'aluminium, les poudres de polyuréthane, les charges composites, les microsphères de silice creuses, et les microcapsules de verre ou de céramique. On peut également utiliser des particules, qui ont la forme de portions de sphères creuses, telles que décrites dans les demandes de brevet JP-2003 128 788 et JP-2000 191 789.

En particulier, de telles charges peuvent être présentes dans une composition selon l'invention dans une teneur comprise entre 0,01 % et 30 % en poids, notamment comprise entre 0,1 % et 25 % en poids, en particulier comprise entre 1 % et 20 %, poids, par rapport au poids total de la composition.

Selon un mode de réalisation de l'invention, une composition peut comprendre au moins des particules solides telles que des pigments et/ou des charges.

**Agent dispersant**

Avantageusement, une composition selon l'invention peut comprendre en outre un agent dispersant.

Un tel agent dispersant peut être un tensioactif, un oligomère, un polymère ou un mélange de plusieurs d'entre eux.

Selon un mode de réalisation particulier, un agent dispersant conforme à l'invention est un tensioactif.

Selon une variante particulière, une composition selon l'invention comprend au moins 1 % en poids de tensioactif

par rapport au poids total de la composition voire est dénuée de tensioactif.

## Actif

Pour une application en particulier de soin, une composition selon l'invention peut comprendre au moins un agent hydratant (également appelé agent humectant).

De préférence, l'agent hydratant est de la glycérine.

Le ou les agents hydratants pourront être présents dans la composition en une teneur allant de 0,1 % à 30 % en poids, notamment de 0,5 % à 15 % en poids, voire de 1 % à 10 % en poids, par rapport au poids total de ladite composition.

Comme autres actifs utilisables dans la composition de l'invention, on peut citer par exemple les vitamines.

De préférence, une composition selon l'invention comprend au moins un actif.

Il relève des opérations de routine de l'homme de l'art d'ajuster la nature et la quantité des additifs présents dans les compositions conformes à l'invention, de telle sorte que les propriétés cosmétiques désirées de celles-ci n'en soient pas affectées.

Selon un mode de réalisation, une composition de l'invention peut avantageusement se présenter sous la forme d'une composition photoprotectrice de soin de la peau et/ou des fibres kératiniques notamment les cheveux, en particulier du corps, du visage.

Selon un autre mode de réalisation, une composition de l'invention peut avantageusement se présenter sous la forme d'une composition de base de maquillage pour le maquillage.

Selon un autre mode de réalisation, une composition de l'invention peut avantageusement se présenter sous la forme d'un fond de teint.

Selon un mode de réalisation, une composition de l'invention peut avantageusement se présenter sous la forme d'une composition de maquillage de la peau et notamment du visage. Il peut ainsi s'agir d'un fard à paupières ou d'un fard à joues. Selon un autre mode de réalisation, une composition de l'invention peut avantageusement se présenter sous la forme d'un produit pour lèvres, notamment un rouge à lèvres.

De telles compositions sont notamment préparées selon les connaissances générales de l'homme de l'art.

Dans toute la description, y compris les revendications, l'expression « *comportant un* » doit être comprise comme étant synonyme de « *comportant au moins un* », sauf si le contraire est spécifié.

Les expressions « *compris entre ... et ...* » et « *allant de ... à ...* » doivent se comprendre bornes incluses, sauf si le contraire est spécifié.

L'invention est illustrée plus en détail par les exemples présentés ci-après. Sauf indication contraire, les quantités indiquées sont exprimées en pourcentage massique.

## Méthodologie pour les mesures de rhéologie dynamique en oscillation

Ce sont des mesures rhéologiques en régime harmonique qui assurent la mesure du module élastique.

Les mesures sont réalisées à l'aide d'un rhéomètre type Haake RS600 sur un produit au repos, à 25 °C avec un mobile plan plan Ø 60 mm et un entrefer de 2 mm.

Les mesures en régime harmonique permettent de caractériser les propriétés viscoélastiques des produits. La technique consiste à soumettre un matériau à une contrainte qui varie sinusoïdalement au cours du temps et à mesurer la réponse du matériau à cette sollicitation. Dans un domaine où le comportement est viscoélastique linéaire (zone où la déformation est proportionnelle à la contrainte), la contrainte ($\tau$) et la déformation (y) sont deux fonctions sinusoïdales du temps qui s'écrivent de la façon suivante :

$$\tau(t) = \tau_0 \sin(\omega t)$$

$$\gamma(t) = \gamma_0 \sin(\omega t + \delta)$$

où :

- $\tau_0$ représente l'amplitude maximale de la contrainte (Pa) ;
- yo représente l'amplitude maximale de la déformation (-) ;
- $\omega = 2\Pi N$ représente la pulsation ($rad.s^{-1}$) avec N représentant la fréquence (Hz) ; et
- $\delta$ représente le déphasage de la contrainte par rapport à la déformation (rad).

Ainsi, les deux fonctions ont la même fréquence angulaire mais elles sont déphasées d'un angle $\delta$. Selon le décalage de phases $\delta$ entre $\tau$(t) et y(t), le comportement du système peut être appréhendé :

- Si $\delta = 0$, le matériau est purement élastique ;
- Si $\delta = \Pi/2$, le matériau est purement visqueux (fluide newtonien) ; et
- Si $0 < \delta < \Pi/2$, le matériau est viscoélastique.

En général, la contrainte et la déformation s'écrivent sous forme complexe :

$$\tau^*(t) = \tau_0 \, e^{i\omega t}$$

$$\gamma^*(t) = \gamma_0 \, e^{(i\omega t + \delta)}$$

Un module de rigidité complexe, représentant la résistance globale du matériau à la déformation qu'elle soit d'origine élastique ou visqueuse, est alors défini par :

$$G^* = \tau^*/\gamma^* = G' + iG''$$

Où:

G' est le module de conservation ou module élastique qui caractérise l'énergie emmagasinée et totalement restituée au cours d'un cycle, $G' = (\tau_0/\gamma_0) \cos \delta$ ; et
G" est le module de perte ou module visqueux qui caractérise l'énergie dissipée par frottement interne au cours d'un cycle, $G'' = (\tau_0/\gamma_0) \sin \delta$.
Le paramètre retenu est le module de rigidité moyen G* relevé au plateau mesuré à une fréquence de 1 Hz.

## EXEMPLES

Dans les tableaux suivants, la quantité de chaque composé est donnée en % de matière première en poids par rapport au poids total de la composition.
Les formules suivantes sont préparées de sorte à ce que reste constant :

- le pourcentage massique de la phase huileuse,
- le pourcentage massique de la phase aqueuse,
- le pourcentage massique de gélifiant huileux,
- le pourcentage massique de gélifiant aqueux,
- le pourcentage massique de filtres UV,

Tous les autres constituants des formules sont présents dans le même pourcentage massique.

### A Première série d'exemples

### Préparation des compositions

#### Préparation des phases lipophiles L

La phase grasse est gélifiée par au moins un agent gélifiant huileux avec ou sans filtres organiques ou particulaires.
Dans les compositions selon l'invention, la phase grasse est gélifiée par la Disteardimonium Hectorite (bentone 38 VCG) seule (exemples 1-2 et 9).
Les exemples comparatifs sont eux gélifiés soit par l'Ethylcellulose (exemples 3, 4, 10, 11 et 12), soit par une cire hydrocarbonée apolaires à haut point de fusion, Microcristalline Wax (exemples 5 et 6) ou encore un polyamide siliconé (exemples 7 et 8).
Mode Opératoire : Dans un premier temps, la totalité des filtres liposolubles et des matières premières solubles à chaud est pesée dans un bécher et solubilisée sous agitation mécanique à 80 °C.
Dès que la solution de filtres est macroscopiquement limpide, sont rajoutés les gélifiants huileux sous agition mécanique à l'aide d'une « défloculeuse ». A l'obtention d'une phase gélifiée homogène, vient se rajouter les solvants sous la même agitation mécanique. Le gel obtenu constitue un gel homogène.

**Préparation des gels hydrophiles H**

Les composants de la phase aqueuse sont pesés dans un bécher et placés sous agitation.

La phase aqueuse est gélifiée par au moins un agent gélifiant aqueux avec ou sans filtres organiques ou particulaires.

Dans les compositions selon l'invention, la phase aqueuse est gélifiée par le Hydroxyethyl acrylate/sodium acryloydimethyl taurate copolymer - Sepinov EMT 10 (exemples let 2) ou par un Carbomer (exemple 9).

Les exemples comparatifs sont gélifiés soit par le Hydroxyethyl acrylate/sodium acryloydimethyl taurate copolymer - Sepinov EMT 10 (exemples comparatifs 3 à 8) soit par un Carbomer (exemple hors invention 10) soit par un poloxamer (exemple hors invention 11) ou la sodium CMC (exemple hors invention 12).

Mode Opératoire : Le gélifiant aqueux est introduit dans les solvants aqueux sous agitation à l'aide d'une « défloculeuse » à température ambiante. Le gel obtenu constitue un gel homogène.

**Mode opératoire du gel/gel**

Les phase lipophiles et hydrophiles étant homogènes, le gel/gel est préparé en mélangeant les deux phases dans un mélangeur du type « Pétrin » muni d'une cuve et d'une pale axiale sous agitation modérée pendant 4 minutes.

Le gel final se caractérise par une dispersion bi-continue macroscopiquement homogène.

**Propriétés testées**

L'observation de l'aspect macroscopique du gel huileux, du gel aqueux, et du gel/gel sont observés à :

t0 c'est-à-dire, en fin de formulation, en sortie de cuve ;

t1 c'est-à-dire après 2h00 de repos à température ambiante.

**Compositions**

| | Composés Nom INCI | Exemple 1 conforme | Exemple 2 conforme | Exemple 3 comparatif | Exemple 4 comparatif |
|---|---|---|---|---|---|
| PHASE B LIPOPHILE | Ethylhexyl salicylate commercialisé sous le nom Néo Héliopan OS par Svmrise | 5 | 5 | 5 | 5 |
| | Octocrylene commercialisé sous le nom Uvinul N539 T par BASF | 7 | 7 | 7 | 7 |
| | Butyl methoxydibenzoylmethane commercialisé sous le nom Avobenzone par MFCI | 3 | 3 | 3 | 3 |
| | Disteardimonium Hectorite commercialisé sous le nom Bentone 38 VCG par Elementis | 5 | 5 | - | - |
| | Ethylcellulose commercialisé sous le nom Ethocel par Dow Chemicals | - | - | 5 | 5 |
| | Propylene carbonate commercialisé sous le nom Arconate propylene carbonate par Lvondell | 1,5 | 1,5 | 1,5 | 1,5 |
| | Octyldodecanol commercialisé sous le nom Eutanol G par Cognis | 27,7 | 27,7 | 27,7 | 27,7 |
| | Caprylyl glycol commercialisé sous le nom Dermasoft Octiol par Dr Straetmans | 0,1 | 0,1 | 0,1 | 0,1 |
| | Phenoxyethanol commercialisé sous le nom | 0,5 | 0,5 | 0,5 | 0,5 |
| PHASE A HYDROPHILE | Sepicide LD par Seppic | | | | |
| | Disodium EDTA commercialisé sous le nom EDETA BD par BASF | 0,2 | 0,2 | 0,2 | 0,2 |
| | Terephthalylidene Dicamphor Sulfonic Acid (à 33 %MA) commercialisé sous le nom Mexoryl SX par Chimex | - | 18 | - | 18 |
| | Phenylbenzimidazole sulfonic acide commercialisé sous le nom Eusolex 232 par Merck | - | 6 | - | 6 |
| | Triethanolamine commercialisé sous le nom Triethanolamine par BASF | - | 6,75 | - | 6,75 |
| | Water -eau désionisée | 39,9 | 9,15 | 39,9 | 9,15 |
| | Caprylyl glycol commercialisé sous le nom commercialisé sous le nom Dermasoft Octiol par Dr Straetmans | 0,1 | 0,1 | 0,1 | 0,1 |
| | Phenoxyethanol commercialisé sous le nom Sepicide LD par Seppic | 0,5 | 0,5 | 0,5 | 0,5 |
| | Glycerin commercialisé sous le nom Glycerine USP par VVF | 7 | 7 | 7 | 7 |
| | Hydroxyethyl acrylate/sodium acryloydimethyl taurate copolymer commercialisé sous le nomSepinov EMT 10 par Seppic | 2,5 | 2,5 | 2,5 | 2,5 |

Résultats :

Pour les compositions conformes 1 et 2, le gel huileux, le gel aqueux, et le gel/gel final sont toujours homogènes à t0 et à t1.

Par contre,

- concernant la composition comparative 3, l'aspect macroscopique de la composition à t1 apparait déphasé et

- et concernant la composition comparative 4, on observe un relargage du gel huileux à t1 ainsi qu'un déphasage de la composition à t0.

| | **Composés Nom INCI** | **Exemple 5 comparatif** | **Exemple 6 comparatif** | **Exemple 7 comparatif** | **Exemple 8 comparatif** |
|---|---|---|---|---|---|
| PHASE B LIPOPHILE | Ethylhexyl salicylate commercialisé sous le nom Néo Héliopan OS par Symrise | 5 | 5 | 5 | 5 |
| | Octocrylene commercialisé sous le nom Uvinul N539 T par BASF | 7 | 7 | 7 | 7 |
| | Butyl methoxydibenzoylmethane commercialisé sous le nom Avobenzone par MFCI | 3 | 3 | 3 | 3 |
| | Microcristalline Wax commercialisé sous le nom Microwax HW par Paramelt | 5 | 5 | - | - |
| | Nylon-611/Dimethicone copolymer commercialisé sous le nom Dow Corning 2-8179 gellant par Dow Corning | - | - | 5 | 5 |
| | Propylene carbonate commercialisé sous le nom Arconate propylene carbonate par Lvondell | 1,5 | 1,5 | 1,5 | 1,5 |
| | Octyldodecanol commercialisé sous le nom Eutanol G par Cognis | 27,7 | 27,7 | 27,7 | 27,7 |
| | Caprylyl glycol commercialisé sous le nom Dermasoft Octiol par Dr Straetmans | 0,1 | 0,1 | 0,1 | 0,1 |
| | Phenoxyethanol commercialisé sous le nom Sepicide LD par Seppic | 0,5 | 0,5 | 0,5 | 0,5 |
| | Disodium EDTA commercialisé sous le nom EDETA BD par BASF | 0,2 | 0,2 | 0,2 | 0,2 |

(suite)

| | Composés Nom INCI | Exemple 5 comparatif | Exemple 6 comparatif | Exemple 7 comparatif | Exemple 8 comparatif |
|---|---|---|---|---|---|
| PHASE A HYDROPHILE | Terephthalylidene Dicamphor Sulfonic Acid commercialisé sous le nom Mexoryl SX par Chimex | - | 18 | - | 18 |
| | Phenylbenzimidazole sulfonic acide commercialisé sous le nom Eusolex 232 par Merck | - | 6 | - | 6 |
| | Triethanolamine commercialisé sous le nom Triethanolamine par BASF | - | 6,75 | - | 6,75 |
| | Water -eau désionisée | 39,9 | 9,15 | 39,9 | 9,15 |
| | Caprylyl glycol commercialisé sous le nom commercialisé sous le nom Dermasoft Octiol par Dr Straetmans | 0,1 | 0,1 | 0,1 | 0,1 |
| | Phenoxyethanol commercialisé sous le nom Sepicide LD par Seppic | 0,5 | 0,5 | 0,5 | 0,5 |
| | Glycerin commercialisé sous le nom Glycerine USP par VVF | 7 | 7 | 7 | 7 |
| | Hydroxyethyl acrylate/sodium acryloydimethyl taurate copolymer commercialisé sous le nomSepinov EMT 10 par Seppic | 2,5 | 2,5 | 2,5 | 2,5 |

Résultats :

Pour toutes les compositions comparatives 5, 6, 7 et 8, lors de l'étude de l'aspect macroscopique du gel huileux, on observe un amas dudit gel huileux dès t0, accompagné pour les compositions 7 et 8 de présence d'huile en surface.

En outre on observe un déphasage de toutes ces compositions comparatives dés t0.

| | Composés Nom INCI | Exemple 9 conforme | Exemple 10 comparatif | Exemple 11 comparatif | Exemple 12 comparatif |
|---|---|---|---|---|---|
| PHASE B LIPOPHILE | Ethylhexyl Methoxycinnamate commercialisé sous le nom Parsol MCX par BASF | 7 | 7 | 7 | 7 |
| | Disteardimonium Hectorite commercialisé sous le nom Bentone 38 VCG par Elementis | 5 | - | - | - |
| | Ethylcellulose commercialisé sous le nom Ethocel par Dow Chemicals | - | 5 | 5 | 5 |
| | Propylene carbonate commercialisé sous le nom Arconate propylene carbonate par Lyondell | 1,5 | - | - | - |

(suite)

| | Composés Nom INCI | Exemple 9 conforme | Exemple 10 comparatif | Exemple 11 comparatif | Exemple 12 comparatif |
|---|---|---|---|---|---|
| | Caprylic/Capric Triglycéride commercialisé sous le nom Triglycéride C8/C10 par Stearineries Dubois | 35,9 | 37,4 | 37,4 | 37,4 |
| | Caprylyl glycol commercialisé sous le nom Dermasoft Octiol par Dr Straetmans | 0,1 | 0,1 | 0,1 | 0,1 |
| | Phenoxyethanol commercialisé sous le nom Sepicide LD par Seppic | 0,5 | 0,5 | 0,5 | 0,5 |
| PHASE A HYDROPHILE | Water -eau désionisée | 46,9 | 46,9 | 46,9 | 46,9 |
| | Caprylyl glycol commercialisé sous le nom Dermasoft Octiol par Dr Straetmans | 0,1 | 0,1 | 0,1 | 0,1 |
| | Phenoxyethanol commercialisé sous le nom Sepicide LD par Seppic | 0,5 | 0,5 | 0,5 | 0,5 |
| | Carbomer commercialisé sous le nom Carbopol 980 Polymer par Ashland | 1,25 | 1,25 | - | - |
| | Triethanolamine commercialisé sous le nom Triethanolamine par BASF | 1,25 | 1,25 | - | - |
| | Synperonic PE/L 64-LQ-(CQ) commercialisé sous le nom Ploxamer par Croda | - | - | 2,5 | - |
| | Sodium CarboxyMéthylCellulose commercialisé sous le nom Blanose par Ashland | - | - | - | 2,5 |

Résultats

Pour la composition conforme 9, le gel huileux, le gel aqueux, et le gel/gel final sont toujours homogènes à t1.

Par contre, pour les compositions comparatives 10, 11 et 12, l'aspect macroscopique de la composition à t1 apparait déphasé.

Seuls les exemples 1 , 2 et 9 conformes à l'invention conduisent à des compositions gel/gelstables et homogènes présentant en outre un sensoriel agréable.

**B Deuxième série d'exemples (comprenant un élastomère de silicone et un aérogel de silice)**

**Compositions**

| | Composés | Exemple 1 Conforme (Composition gel/gel) | Exemple 2 comparatif (émulsion directe) |
|---|---|---|---|
| PHASE B LIPOPHILE | Homosalate Commercialisé sous le nom Néo Héliopan HMS PBF par Symrise | 4 | 4 |
| | Ethylhexyl salicylate commercialisé sous le nom Néo Héliopan OS par Symrise | 2 | 2 |
| | Octocrylene commercialisé sous le nom Uvinul N539 T par BASF | 5 | 5 |
| | Butyl methoxydibenzoylmethane commercialisé sous le nom Avobenzone par MFCI | 4 | 4 |
| | Bis-ethylhexyloxyphenol methoxyphenyl triazine commercialisée sous le nom Tinosorb S par BASF | 2 | 2 |
| | Ethvlhexvl triazone commercialisée sous le nom Uvinul T150 par BASF | 1 | 1 |
| | Disteardimonium Hectorite commercialisé sous le nom Bentone 38 VCG par Elementis | 2,69 | 2,69 |
| | Silice Silylée commercialisé sous le nom DC V %-2270 Aerogel fine particles par Dow Corning | 1 | 1 |
| | Dimethicone commercialisé sous le nom DC Toray SH200 C Fluid 5cs par Dow Corning | 7,1 | 7,1 |
| | Dimethicone and Dimethicone crosspolymer commercialisé sous le nom DC 9041 Silicone Elastomer Blend par Dow Corning | 15,4 (soit 2,4% de Dimethicone crosspolymer) | 15,4 (soit 2,4% de Dimethicone crosspolymer) |
| | acide Stearique commercialisé sous le nom Radiacid 0461 par Oleon | - | 1,5 |
| | Glyceryl stearate and PEG-100 stearate commercialisé sous le nom Simulsol 165 par Seppic | - | 1,5 |

(suite)

| | Composés | Exemple 1 Conforme (Composition gel/gel) | Exemple 2 comparatif (émulsion directe) |
|---|---|---|---|
| PHASE A HYDROPHILE | Alcool denat commercialisé sous le nom Ethanol SDA 40B 200 Proof par Sasol | 5,31 | 5,31 |
| | Water -eau désionisée | 38,35 | 38,35- |
| | Disodium EDTA commercialisé sous le nom EDETA BD par BASF | 0,2 | 0,2 |
| | Caprylyl glycol commercialisé sous le nom commercialisé sous le nom Dermasoft Octiol par Dr Straetmans | 0,7 | 0,7 |
| | Phenoxyethanol commercialisé sous le nom Sepicide LD par Seppic | 0,2 | 0,2 |
| | Glycerine commercialisée sous le nom Glycerine USP par VVF | 6,6 | 6,6 |
| | Triéthanolamine commercialisé sous le nom Triethanolamine 99 % Dow Chemical | - | 0,45 |
| | Potassium cetyl phosphate commercialisé sous le nom Amphisol K par DSM Nutritional Products | - | 1 |
| | Hydroxyethyl acrylate/sodium acryloydimethyl taurate copolymer commercialisé sous le nomSepinov EMT 10 par Seppic | 4,45 | - |

## Préparation des compositions

### Préparation des phases lipophiles L

La phase grasse est gélifiée par au moins l'aérogel, agent gélifiant huileux avec ou sans filtres organiques ou particulaires.

Dans l'exemple 1, la phase grasse est gélifiée par de l'aérogel associé à la bentone 38 VCG. L'exemple hors invention 2 est également gélifié par les mêmes gélifiants, mais elle contient en plus des tensio-actifs pour former l'émulsion.

Dans un premier temps, la totalité des filtres lipophiles et des matières premières solubles à chaud est pesée dans un bécher et solubilisée sous agitation mécanique à 80 °C.

Dès que la solution de filtres est macroscopiquement limpide, sont rajoutés les gélifiants huileux sous agitation mécanique à l'aide d'une « défloculeuse ». A l'obtention d'une phase gélifiée homogène, on ajoute les solvants sous la même agitation mécanique. Le gel obtenu est homogène.

### Préparation des phases hydrophiles H

Les composants de la phase aqueuse sont pesés dans un bécher et placés sous agitation.

La phase aqueuse est gélifiée par au moins un agent gélifiant aqueux avec ou sans filtres organiques ou particulaires.

Dans l'exemple 1, la phase aqueuse est gélifiée par le Sepinov EMT 10.

Le gélifiant aqueux est introduit dans les solvants aqueux sous agitation à l'aide d'une « défloculeuse » à température ambiante. Le gel obtenu est homogène

La phase aqueuse de l'exemple hors invention 2 n'est pas gélifiée par le Sépinov EMT 10, mais contient un pourcentage massique équivalent de tensio-actifs et neutralisant associé.

**Mode opératoire du gel/gel- exemple conforme 1**

Le gel/gel est préparé en mélangeant les deux phases dans un mélangeur du type « Pétrin » muni d'une cuve et d'une pale axiale sous agitation modérée pendant 4 minutes.

Le gel final se caractérise par une dispersion bi-continue macroscopiquement homogène.

**Mode de préparation de l'émulsion - exemple comparatif 2**

L'émulsion est préparée par introduction de la phase L au sein de la phase H sous agitation à l'aide d'un homogénéisateur de rotor-stator sous une vitesse d'agitation de 4500 RPM pendant 20 minutes. L'émulsion est refroidie à température ambiante.

L'émulsion finale se caractérise par des gouttes de taille comprise entre 1 $\mu$m et 20$\mu$m.

**Mesures**

Le SPF, le PPD et l'analyse sensorielle de ces compositions 1 et 2 ont été mesurés.

Protocole d'évaluation ***in vitro*** **de l'efficacité filtrante (SPF)**

Le facteur de protection solaire est déterminé selon la méthode « *in vitro* » décrite par B. L. Diffey dans J. Soc. Cosmet. Chem. 40, 127-133, (1989). Les mesures ont été réalisées à l'aide d'un spectrophotomètre UV-1000S de la société Labsphère. Chaque composition est appliquée sur une plaque rugueuse de PMMA, sous la forme d'un dépôt homogène et régulier à raison de 1 mg/cm$^2$.

**Protocole de mesure de PPD** *in vitro*

Les mesures d'indice de PPD *in vitro* ont été effectuées dans les mêmes conditions au moyen d'un spectrophotomètre UV-1000S de la société Labsphère. Chaque composition est appliquée sur une plaque rugueuse de PMMA, sous la forme d'un dépôt homogène et régulier à raison de 1 mg/cm$^2$.

On extrait la valeur « Indice UV-A ppd :Spectre d'action « Persistent Pigment Darkening ».

Le facteur de protection solaire UVA PPD (FP UVAppd) s'exprime mathématiquement par le rapport de la dose de rayonnement UV-A nécessaire pour atteindre le seuil de pigmentation avec le filtre UV (MPPDp) sur la dose de rayonnement UV-A nécessaire pour atteindre le seuil de pigmentation sans filtre UV (MPPDnp).

$$\mathrm{FP\,UVA_{PPD}} = \frac{\mathrm{MPPDp}}{\mathrm{MPPDnp}}$$

**Protocole d'évaluation des propriétés sensorielles des formules sur la peau**

Les propriétés sensorielles des formules sur la peau sont évaluées par application de la formule sur un avant bras à raison de 2 mg/cm2 et observation d'un temps de séchage égal à 2 minutes. La fraîcheur est évaluée pendant l'application alors que l'aspect gras et doux sont appréciés après application, entre les doigts et la surface de l'avant bras.

**Résultats**

Résultats

| Propriété testée | **Exemple 1 conforme** | **Exemple 2 comparatif** |
|---|---|---|
| SPF in vitro | 43,9 +/- 4,8 | 21,3+/-3,9 |
| PPD in vitro | 21,8+/-2,1 | 10,4+/-1,1 |
| Analyse sensorielle | Frais à l'application, doux | Toucher plus gras |

Ces résultats montrent que la composition 1 de l'invention permet d'obtenir un niveau d'efficacité filtrante plus

important que la composition 2 (émulsion directe), tout en montrant une sensorialité améliorée.

## C Troisième série d'exemples (comprenant un élastomère de silicone)

### Compositions non teintées

**Préparation des compositions**

**Préparation des phases lipophiles L**

La phase grasse est gélifiée par l'agent gélifiant huileux.

Mode opératoire

Dans un premier temps, la totalité des filtres lipophiles et des matières premières solubles à chaud est pesée dans un bécher et solubilisée sous agitation mécanique à 80 °C.

Dès que la solution de filtres est macroscopiquement limpide, sont rajoutés le(s) gélifiant(s) huileux et les solvants sous agitation mécanique à l'aide d'une *« défloculeuse »*.. Le gel obtenu est homogène.

**Préparation des phases hydrophiles H**

Les composants de la phase aqueuse sont pesés dans un bécher et placés sous agitation.

La phase aqueuse est gélifiée par au moins un agent gélifiant hydrophile.

Mode opératoire

Le gélifiant hydrophile est introduit dans les solvants aqueux sous agitation à l'aide d'une « *défloculeuse* » à température ambiante. Le gel obtenu est homogène.

**Mode opératoire du gel/gel-**

Le gel/gel est préparé en mélangeant les deux phases dans un mélangeur du type « Pétrin » muni d'une cuve et d'une pale axiale sous agitation modérée pendant 4 minutes.

Le gel final se caractérise par une dispersion bi-continue macroscopiquement homogène.

### Mesures

Les protocole d'évaluation *in vitro* de l'efficacité filtrante (SPF), protocole de mesure de PPD *in vitro* et protocole_d'évaluation des propriétés sensorielles des formules sur la peau correspondent à ceux décrits pour la série B ci-dessus.

### Evaluation de l'effet flouteur des formules en film mince

Les compositions 1 et 2 ont été étalées avec une épaisseur de 50 microns sur un film transparent et l'effet flouteur de chaque composition a été évalué par une mesure de « Haze ».

Le « Haze » correspond au pourcentage de lumière diffusée par rapport à la transmittance totale selon la norme ASTM D 1003 (Standard Test Method for Haze and Luminous Transmittance of Transparent Plastics).

### Protocole d'évaluation de l'homogénéité du film

La formule est étalée sous forme de film de de 30μm sur plaque de verre au moyen d'un tire-film manuel. L'homogénété du film de formule est ensuite évaluée par observation des dépôts à l'œil.

On note par :

« - » un dépôt non homogène, qui se caractérise par la présence de trous visibles à l'œil nu important ;

« + » un dépôt homogène ;

« ++ » un dépôt très homogène, qui se caractérise pas un nombre de trous visibles très faible.

| | Composés | Exemple 1 conforme | Exemple 2 conforme |
|---|---|---|---|
| PHASE B LIPOPHILE | Homosalate Commercialisé sous le nom Néo Héliopan HMS PBF par Symrise | **4** | **4** |
| | Ethylhexyl salicylate commercialisé sous le nom Néo Héliopan OS par Symrise | 2 | 2 |
| | Octocrylene commercialisé sous le nom Uvinul N539 T par BASF | 7 | 7 |
| | Butyl methoxydibenzoylmethane commercialisé sous le nom Avobenzone par MFCI | 3 | 3 |
| | Silice Silylée commercialisé sous le nom DC V%-2270 Aerogel fine particles par Dow Corning | 1 | 1,13 |
| | Disteardimonium Hectorite commercialisé sous le nom Bentone 38 VCG par Elementis | 2,69 | - |
| | Propylene carbonate commercialisé sous le nom Arconate propylene carbonate par Lvondell | 0,81 | - |
| | Dimethicone commercialisé sous le nom DC Toray SH200 C Fluid 5cs par Dow Corning | 8,8 | 1 |
| | Dimethicone and Dimethicone crosspolymer commercialisé sous le nom DC 9041 Silicone Elastomer Blend par Dow Corning | 15,4 (soit 2,4% de Dimethicone crosspolymer) | - |
| | Dimethicone and Dimethicone crosspolymer commercialisé sous le nom EL-9240 Silicone Elastomer Blend par Dow Corning | - | 15,4 (soit 2,0% de Dimethicone crosspolymer) |
| | Alcool denat commercialisé sous le nom Ethanol SDA 40B 200 Proof par Sasol | 4,5 | - |
| PHASE A HYDROPHILE | Water -eau désionisée | Qsp 100 | Qsp 100 |
| | Conservateur | 0,98 | 0,9 |
| | Glycerine commercialisé sous le nom Glycerine USP par VVF | 6,6 | 6,6 |
| | Hydroxyethyl acrylate/sodium acryloydimethyl taurate copolymer commercialisé sous le nom-Sepinov EMT 10 par Seppic | 2,25 | 2,25 |

**Résultats**

| Propriété testée | **Exemple 1 conforme** |
|---|---|
| SPF in vitro | 46±8 |
| PPD in vitro | 20±3 |
| Haze sur films de 50μm | 87,7 |

La composition 1 présente de bonnes propriétés photoprotectrices (niveau d'efficacité filtrante) ainsi qu'un floutage important.

En outre la composition 1 présente des propriétés de fraicheur et de velouté ainsi que des propriétés sensorielles et de matité très satisfaisantes.

Pour la composition 2, seule la propriété d'homogénéité du film a été testée.

| Propriété testée | **Exemple 2** |
|---|---|
| Homogénéité du film (30μm), couvrance | ++ |

La composition 2 présente une très bonne homogénéité qui se traduit par un aspect lisse, de la composition déposée, visible à l'œil nu.

**<u>Compositions teintées (exemples 3 à 9)</u>**

Les compositions de fond de teint sont préparées selon le protocole ci-dessous conformément au décrit protocole d'évaluation de l'homogénéité du film ci-dessus.

**Préparation des compositions**

**Préparation de la phase hydrophile H**

Les composants de la phase aqueuse sont pesés dans un bécher et placés sous agitation au Rayneri à température ambiante.
Le gélifiant aqueux est ajouté sous agitation à température ambiante. L'agitation est ajustée afin de ne pas incorporer d'air au mélange. Le mélange est laissé sous agitation modérée pendant environ 10 minutes à température ambiante.
On obtient un gel aqueux homogène.

**Préparation de la phase lipophile L**

Les pigments sont broyés avec les solvants de la phase lipophile B à l'aide d'une tricylindre. Le broyat est ensuite introduit dans un bécher et mis sous agitation au rayneri à température ambiante. Le gélifiant est ajouté sous forte agitation à température ambiante. Le gel s'épaissit peu à peu. Le mélange est laissé sous forte agitation pendant 10 minutesphase grasse est gélifiée par l'agent gélifiant huileux.
On obtient un gel huileux homogène.

**Préparation de la formulation de fond de teint**

La formulation est obtenue par mélange des phases dédiées à former le fond de teint conforme à l'invention.
Les gels aqueux et huileux sont pesés puis mélangés au rayneri sous agitation moyenne. La formule est préparée à partir des proportions pondérales décrites dans les formules.
Pour les compositions teintées, seule la propriété d'homogénéité du film a été testée.

| | Composés | Exemple 3 conforme | Exemple 4 conforme | Exemple 5 conforme |
|---|---|---|---|---|
| PHASE B LIPOPHILE | Homosalate Commercialisé sous le nom Néo Héliopan HMS PBF par Symrise | 4 | 4 | 4 |
| | Ethylhexyl salicylate commercialisé sous le nom Néo Héliopan OS par Symrise | 2 | 2 | 2 |
| | Octocrylene commercialisé sous le nom Uvinul N539 T par BASF | 7 | 7 | 7 |
| | OXYDES DE FER ENROBES DE STEAROYL GLUTAMATE D'ALUMINIUM (NAI-C33-9001-10 commercialisé par la société MIYOSHI KASEI | 2,54 | 2,54 | 2,54 |
| | DIOXYDE DE TITANE ENROBE DE STEAROYL GLUTAMATE D'ALUMINIUM (NAI-TAO-77891 commercialisé par la société MIYOSHI KASEI | 8,46 | 8,46 | 8,46 |
| | Disteardimonium Hectorite commercialisé sous le nom Bentone 38 VCG par Elementis | - | 3 | - |
| | Silice Silylée commercialisé sous le nom DC V%-2270 Aerogel fine particles par Dow Corning | - | - | 0,96 |
| | Dimethicone and Dimethicone crosspol ymer commercialisé sous le nom EL-9240 Silicone Elastomer Blend par Dow Corning | 7 (soit 0,9% de Dimethicone crosspolymer) | 7 (soit 0,9% de Dimethicone crosspolymer) | 7 (soit 0,9% de Dimethicone crosspolymer) |
| PHASE A HYDROPHILE | Water -eau désionisée | Qsp 100 | Qsp 100 | Qsp 100 |
| | Conservateur | 0,7 | 0,7 | 0,7 |
| | Glycerin commercialisé sous le | 6 | 6 | 6 |
| | nom Glycerine USP par VVF | | | |
| | Hydroxyethyl acrylate/sodium acryloydimethyl taurate copolymer commercialisé sous le nom-Sepinov EMT 10 par Seppic | 2,4 | 2,4 | 2,4 |

Résultats

| | Exemple 3 conforme | Exemple 4 conforme | Exemple 5 conforme |
|---|---|---|---|
| Homogénéité du film (30μm), couvrance | + | ++ | ++ |

| | Composés | Exemple 6 conforme | Exemple 7 conforme | Exemple 8 conforme | Exemple 9 conforme |
|---|---|---|---|---|---|
| PHASE B LIPOPHILE | Homosalate Commercialisé sous le nom Néo Héliopan HMS PBF par Symrise | 4 | 4 | 4 | 4 |
| | Ethylhexyl salicylate commercialisé sous le nom Néo Héliopan OS par Symrise | 2 | 2 | 2 | 2 |
| | Octocrylene commercialisé sous le nom Uvinul N539 T par BASF | 7 | 7 | 7 | 7 |
| | OXYDES DE FER ENROBES DE STEAROYL GLUTAMATE D'ALUMINIUM (NAI-C33-9001-10 commercialisé par la société MIYOSHI KASEI | 2,54 | 2,54 | 2,54 | 2,54 |
| | DIOXYDE DE TITANE ENROBE DE STEAROYL GLUTAMATE D'ALUMINIUM (NAI-TAO-77891 commercialisé par la société MIYOSHI KASEI | 8,46 | 8,46 | 8,46 | 8,46 |
| | Dimethicone and Dimethicone crosspolymer commercialisé sous le nom DC 9041 Silicone Elastomer Blend par Dow Corning | 7 (soit 1,1 % de Dimethicone crosspolymer) | - | - | - |
| | DIPHENYLSILOXY PHENYL TRIMETHICONE 84 % DIMETHICONE/PHENY L VINYL DIMETHICONE CROSSPOLYMER 16% | - | 7 | - | - |
| | (KSG 18A commercialisé par la société SHIN ETSU) | | | | |
| | VINYL DIMETHICONE / METHICONE SILSESQUIOANE CROSSPOLYMER (KSP 100 commercialisé par la société SHIN ETSU) | - | - | 7 | - |
| | DIPHENYL DIMETHICONE/VINYL DIPHENYL DIMETHICONE/SILSES QUIOANE CROSSPOLYMER (KSP 300 commercialisé par la société SHIN ETSU) | - | - | - | 7 |
| PHASE A HYDROPHILE | Water -eau désionisée | Qsp 100 | Qsp 100 | Qsp 100 | Qsp 100 |
| | Conservateur | 0,7 | 0,7 | 0,7 | 0,7 |
| | Glycerine commercialisé sous le nom Glycerine USP par VVF | 6 | 6 | 6 | 6 |
| | Hydroxyethyl acrylate/sodium acryloydimethyl taurate copolymer commercialisé sous le nomSepinov EMT 10 par Seppic | 2,4 | 2,4 | 2,4 | 2,4 |

Résultats

| | Exemple 6 conforme | Exemple 7 conforme | Exemple 8 conforme | Exemple 9 conforme |
|---|---|---|---|---|
| Homogénéité du film (30μm), couvrance | ++ | ++ | ++ | ++ |

Les compositions 3 à 9 présentent une très bonne homogénéité qui se traduit par un aspect lisse, de la composition déposée, cette propriété étant visible à l'œil nu.

## Revendications

1. Composition, différente d'une émulsion, en particulier cosmétique, de maquillage et/ou de soin des matières kératiniques comprenant :

   - au moins une phase aqueuse gélifiée par au moins un agent gélifiant hydrophile non amylacé,
   - au moins une phase huileuse gélifiée par au moins un agent gélifiant lipophile choisi parmi les élastomères d'organopolysiloxane, les polymères semi-cristallins, les esters de dextrine, les polyamides hydrocarbonés, les gélifiants particulaires choisis parmi les cires polaires, les cires apolaires hydrocarbonées de point de fusion inférieur ou égal à 75,0 °C, les cires siliconées, les argiles modifiées, les silices, ainsi que leurs mélanges ;

   lesdites phases y formant un mélange macroscopiquement homogène ;
   ladite composition comprenant en outre au moins un filtre UV choisi parmi les filtres organiques hydrosolubles, les filtres organiques liposolubles et les filtres organiques insolubles.

2. Composition selon la revendication précédente, dans laquelle ledit agent gélifiant hydrophile non amylacé est choisi parmi les gélifiants polymériques synthétiques, les silicates mixtes et les silices pyrogénées, les gélifiants polymériques naturels ou d'origine naturelle non amylacés, et leurs mélanges, de préférence est un gélifiant polymérique synthétique, de préférence choisi parmi les polymères et copolymères d'acide 2-acrylamido 2-méthylpropane sulfonique, réticulés et/ou neutralisés et les polymères carboxyvinyliques modifiés ou non, encore plus préférentiellement est choisi parmi les polymères réticulés d'acrylamido-2-methyl propane sulfonate d'ammonium, les copolymères d'AMPS® et d'hydroxyéthyl acrylate, et les homopolymères d'acide (méth)acrylique réticulés, de préférence les copolymères d'AMPS® et d'hydroxyéthyl acrylate.

3. Composition selon l'une des revendications précédentes, dans laquelle le gélifiant lipophile est choisi parmi les homo- ou co-polymères semi-cristallins portant au moins une chaîne latérale cristallisable et les homo- ou co-polymères semi-cristallins portant dans le squelette au moins une séquence cristallisable, les polyamides hydrocarbonés, les aérogels de silice hydrophobes, les hectorites modifiées par un sel d'ammonium en $C_{10}$ à $C_{22}$, en particulier les hectorites modifiées par un chlorure d'ammonium en $C_{10}$ à $C_{22}$.

4. Composition selon l'une quelconque des revendications précédentes, dans laquelle le gélifiant lipophile est choisi parmi les hectorites modifiées par un sel d'ammonium en $C_{10}$ à $C_{22}$ de préférence les hectorites modifiées par un chlorure d'ammonium en $C_{10}$ à $C_{22}$.

5. Compositionselon l'une quelconque des revendications 1 à 2, dans laquelle le gélifiant lipophile choisi parmi les élastomères d'organopolysiloxane suivants : Dimethicone Crosspolymer, Vinyl Dimethicone Crosspolymer, Dimethicone/Vinyl Dimethicone Crosspolymer, Dimethicone Crosspolymer-3, VINYL DIMETHICONE/METHICONE SILSESQUIOANE CROSSPOLYMER, PHENYL VINYL DIMETHICONE CROSSPOLYMER et en particulier Dimethicone Crosspolymer.

6. Composition selon l'une quelconque des revendications 1 à 4, comprenant :

   - au moins un gélifiant hydrophile non amylacé choisi parmi les copolymères d'acide 2-acrylamido 2-méthylpropane sulfonique (AMPS®), réticulés et ou neutralisés, et plus particulièrement les copolymères d'AMPS® et d'hydroxyéthyl acrylate et
   - au moins un gélifiant lipophile non cellulosique choisis parmi les hectorites modifiées par un sel, de préférence un chlorure, d'ammonium en $C_{10}$ à $C_{22}$, et particulièrement les hectorites modifiées par du chlorure de di-stéaryl di-méthyl ammonium,

- au moins un filtre UV choisi parmi les filtres organiques hydrosolubles, les filtres organiques liposolubles et les filtres organiques insolubles.

**7.** Composition selon l'une des revendications précédentes, dans laquelle le ou les filtres UV sont choisis parmi les filtres UV organiques hydrosolubles, les filtres UV organiques liposolubles et leurs mélanges, et plus préférentiellement les filtres UV organiques liposolubles.

**8.** Composition selon l'une quelconque des revendications précédentes, dans laquelle le ou les filtres UV sont présents en tout ou en partie, et de préférence uniquement, dans la phase aqueuse gélifiée ou sont présents en tout ou en partie, et de préférence uniquement, dans la phase huileuse gélifiée.

**9.** Composition selon l'une quelconque des revendications précédentes, contenant les phases aqueuse et huileuse gélifiées dans un rapport pondéral phase aqueuse/phase huileuse 95/5 à 5/95, de préférence de 20/80 à 80/20 et encore plus préférentiellement variant de 30/70 à 70/30.

**10.** Composition selon l'une quelconque des revendications précédentes, comprenant en outre au moins des particules solides telles que des pigments et/ou des charges.

**11.** Composition selon l'une quelconque des revendications précédentes, comprenant en outre des huiles siliconées volatiles et/ou non volatiles.

**12.** Composition selon l'une quelconque des revendications précédentes, comprenant en outre au moins un agent hydratant, de préférence de la glycérine.

**13.** Procédé de préparation d'une composition, différente d'une émulsion, en particulier cosmétique de maquillage et/ou de soin des matières kératiniques, comprenant au moins une étape de mélange :

- d'une phase aqueuse gélifiée par au moins un agent gélifiant hydrophile non amylacé ; et
- d'au moins une phase huileuse gélifiée par au moins un agent gélifiant lipophile choisi parmi les élastomères d'organopolysiloxane, les polymères semi-cristallins, les esters de dextrine, les polyamides hydrocarbonés, les gélifiants particulaires choisis parmi les cires polaires, les cires apolaires hydrocarbonées de point de fusion inférieur ou égal à 75,0 °C, les cires siliconées, les argiles modifiées, les silices, ainsi que leurs mélanges ;

lesdites phases y formant un mélange macroscopiquement homogène ;
ladite composition comprenant en outre au moins un filtre UV choisi parmi les filtres organiques hydrosolubles, les filtres organiques liposolubles et les filtres organiques insolubles.

**14.** Procédé selon la revendication précédente, comprenant une étape de mélange d'au moins deux phases gélifiées.

**15.** Procédé selon l'une quelconque des revendications 13 ou 14, dans lequel le mélange est réalisé à température ambiante.

**16.** Procédé cosmétique et non-thérapeutique de maquillage et/ou de soin de matières kératiniques, en particulier de la peau du corps et/ou du visage, et/ou des fibres kératiniques, notamment les cheveux comprenant au moins une étape consistant à appliquer sur ladite matière kératinique une composition telle que définie selon l'une quelconque des revendications 1 à 12.

**17.** Procédé cosmétique selon la revendication 16 de maquillage et/ou de soin de matières kératiniques, en particulier de la peau du corps et/ou du visage, et/ou des fibres kératiniques, notamment les cheveux, **caractérisé en ce que** la composition macroscopiquement homogène est obtenue par mélange extemporané, avant application ou au moment de l'application sur ladite matière kératinique,

- d'une phase aqueuse gélifiée par au moins un agent gélifiant hydrophile non amylacé ; et
- d'au moins une phase huileuse gélifiée par au moins un agent gélifiant lipophile choisi parmi les élastomères d'organopolysiloxane, les polymères semi-cristallins, les esters de dextrine, les polyamides hydrocarbonés, les gélifiants particulaires choisis parmi les cires polaires, les cires apolaires hydrocarbonées de point de fusion inférieur ou égal à 75,0 °C, les cires siliconées, les argiles modifiées, les silices, ainsi que leurs mélanges ; et

ladite composition comprenant en outre au moins un filtre UV choisi parmi les filtres organiques hydrosolubles, les

filtres organiques liposolubles et les filtres organiques insolubles.

**18.** Procédé cosmétique selon la revendication 16 pour limiter l'assombrissement de la peau, et/ou améliorer la couleur et/ou l'homogénéité du teint.

**19.** Procédé cosmétique selon la revendication 16 pour prévenir et/ou traiter les signes du vieillissement d'une matière kératinique.

## Patentansprüche

**1.** Zusammensetzung, verschieden von einer Emulsion, insbesondere kosmetisch, zum Schminken und/oder Pflegen von Keratinmaterialien, umfassend:

- mindestens eine wässrige Phase, die durch mindestens ein hydrophiles nicht-stärkehaltiges Geliermittel geliert ist,
- mindestens eine ölige Phase, die durch mindestens ein lipophiles Geliermittel geliert ist, ausgewählt aus den Organopolysiloxan-Elastomeren, den halbkristallinen Polymeren, den Dextrin-Estern, den Kohlenwasserstoff-Polyamiden, den besonderen Geliermitteln, ausgewählt den Polarkohlenwasserstoffe, den polaren Wachsen, den unpolaren Kohlenwasserstoffwachsen mit einem Schmelzpunkt kleiner als oder gleich wie 75,0 °C, den Silikonwachsen, dem modifizierten Ton, den Kieselsäuren und deren Gemischen;

wobei die Phasen ein makroskopisch homogenes Gemisch bilden;

die Zusammensetzung ferner umfassend mindestens einen UV-Filter, ausgewählt aus den organischen wasserlöslichen Filtern, den organischen fettlöslichen Filtern und den organischen unlöslichen Filtern.

**2.** Zusammensetzung nach dem vorherigen Anspruch, wobei das nichtstärkehaltige hydrophile Geliermittel ausgewählt ist aus den synthetischen polymeren Geliermitteln, den gemischten Silikaten und den pyrogenen Kieselsäuren, den nichtstärkehaltigen polymeren Geliermitteln natürlichen Ursprungs und deren Gemischen, es vorzugsweise ein synthetisches polymeres Geliermittel ist, das vorzugsweise ausgewählt ist aus den vernetzten und/oder neutralisierten Polymeren und den Copolymeren von 2-Acrylamido-2-methylpropansulfonsäure, vernetzten und/oder neutralisierten und modifizierten oder unmodifizierten Carboxyvinylpolymeren, noch bevorzugter ausgewählt aus vernetzten Polymeren von Ammoniumacrylamido-2-methylpropansulfonat, Copolymeren von AMPS® und Hydroxyethylacrylat, und vernetzten Homopolymeren von (Meth)acrylsäure, vorzugsweise Copolymeren von AMPS® und Hydroxyethylacrylat.

**3.** Zusammensetzung nach einem der vorherigen Ansprüche, wobei das lipophile Geliermittel ausgewählt ist aus homo- oder co-polymeren Halbkristallen, die mindestens eine kristallisierbare Seitenkette aufweisen, und den homo- oder co-polymere Halbkristalle, die in dem Gerüst mindestens eine kristallisierbare Sequenz aufweisen, den Kohlenwasserstoff-Polyamiden, den hydrophoben Kieselsäure-Aerogelen, den durch ein $C_{10}$- bis $C_{22}$-Ammoniumsalz modifizierten Hectoriten, insbesondere den durch ein $C_{10}$- bis $C_{22}$-Ammoniumchlorid modifizierten Hectoriten.

**4.** Zusammensetzung nach einem der vorherigen Ansprüche, wobei das lipophile Geliermittel ausgewählt ist aus den durch ein $C_{10}$- bis $C_{22}$-Ammoniumsalz modifizierten Hectoriten, insbesondere den durch ein $C_{10}$- bis C22-Ammoniumchlorid modifizierten Hectoriten.

**5.** Zusammensetzung nach einem der Ansprüche 1 bis 2, wobei das lipophile Gel ausgewählt ist aus den folgenden Organopolysiloxan-Elastomeren: Dimethicon Crosspolymer, Vinyldimethicon-Crosspolymer, Dimethicon/Vinyldimethicon-Crosspolymer, Dimethicon-Crosspolymer-3, VINYLDIMETHICON/METHICON SILSESQUIOXAN-CROSSPOLYMER, PHENYLVINYLDIMETHICON-CROSSPOLYMER und insbesondere Dimethicon-Crosspolymer.

**6.** Zusammensetzung nach einem der Ansprüche 1 bis 4, umfassend:

- mindestens ein hydrophiles, nicht-stärkehaltiges Gel, ausgewählt aus den vernetzten und/oder neutralisierten Copolymeren von 2-Acrylamido-2-methylpropansulfonsäure (AMPS®), insbesondere den Copolymeren von AMPS® und Hydroxyethylacrylat, und
- mindestens ein lipophiles, nicht-zellulosehaltiges Geliermittel, ausgewählt aus den durch ein $C_{10}$- bis $C_{22}$-Ammoniumsalz, vorzugsweise -chlorid, modifizierten Hectoriten, und insbesondere den durch das Di-Stearyl-Di-Methylammoniumchlorid modifizierten Hectoriten,
- mindestens ein UV-Filter, ausgewählt aus den organischen wasserlöslichen Filtern, den organischen fettlöslichen Filtern und den organischen unlöslichen Filtern.

**7.** Zusammensetzung nach einem der vorherigen Ansprüche, wobei der oder die UV-Filter ausgewählt sind aus den wasserlöslichen organischen UV-Filtern, den fettlöslichen organischen UV-Filtern und ihren Gemischen, am meisten bevorzugt den fettlöslichen organischen UV-Filtern.

**8.** Zusammensetzung nach einem der vorherigen Ansprüche, wobei der oder die UV-Filter ganz oder teilweise, vorzugsweise ausschließlich, in der wässrigen, gelierten Phase vorhanden sind, oder ganz oder teilweise, vorzugsweise ausschließlich, in der gelierten öligen Phase vorhanden sind.

**9.** Zusammensetzung gemäß einer der vorherigen Ansprüche, die die gelierte wässrige und ölige Phase in einem gewichteten Verhältnis von wässrige Phase/ölige Phase von 95/5 bis 5/95, vorzugsweise von 20/80 bis 80/20 und noch bevorzugter in einer Variante von 30/70 bis 70/30 enthält.

**10.** Zusammensetzung nach einem der vorherigen Ansprüche, ferner umfassend mindestens Feststoffteilchen, wie beispielsweise Pigmente und/oder Füllstoffe.

**11.** Zusammensetzung nach einem der vorherigen Ansprüche, ferner umfassend flüchtige und/oder nicht-flüchtige Silikonöle.

**12.** Zusammensetzung nach einem der vorherigen Ansprüche, ferner umfassend mindestens ein Hydratationsmittel, vorzugsweise Glyzerin.

**13.** Verfahren zur Herstellung einer Zusammensetzung, verschieden von einer Emulsion, insbesondere kosmetisch, zum Schminken und/oder Pflegen von Keratinmaterialien, umfassend mindestens eine Mischstufe:

- mindestens einer wässrigen Phase, die durch mindestens ein hydrophiles nicht-stärkehaltiges Geliermittel geliert ist; und
- mindestens einer öligen Phase, die durch mindestens ein lipophiles Geliermittel geliert ist, ausgewählt aus den Organopolysiloxan-Elastomeren, den halbkristallinen Polymeren, den Dextrin-Estern, den Kohlenwasserstoff-Polyamiden, den besonderen Geliermitteln, ausgewählt den Polarkohlenwasserstoffe, den polaren Wachsen, den unpolaren Kohlenwasserstoffwachsen mit einem Schmelzpunkt kleiner als oder gleich wie 75,0 °C, den Silikonwachsen, dem modifizierten Ton, den Kieselsäuren und deren Gemischen;

wobei die Phasen ein makroskopisch homogenes Gemisch bilden;
die Zusammensetzung ferner umfassend mindestens einen UV-Filter, ausgewählt aus den organischen wasserlöslichen Filtern, den organischen fettlöslichen Filtern und den organischen unlöslichen Filtern.

**14.** Verfahren nach dem vorherigen Anspruch, umfassend einen Mischschritt von mindestens zwei gelierten Phasen.

**15.** Verfahren nach einem der Ansprüche 13 oder 14, wobei das Mischen bei Raumtemperatur ausgeführt wird.

**16.** Kosmetisches und nicht-therapeutisches Schmink- und/oder Pflegeverfahren von Keratinmaterialien, insbesondere der Haut des Körpers und/oder des Gesichts, und/oder von Keratinfasern, insbesondere des Haars, umfassend mindestens einen Schritt, der darin besteht, auf das Keratinmaterial eine Zusammensetzung aufzutragen, wie sie in einem der Ansprüche 1 bis 12 definiert ist.

**17.** Kosmisches Verfahren nach Anspruch 16 zum Schminken und/oder Pflegen von Keratinmaterialien, insbesondere der Haut des Körpers und/oder des Gesichts, und/oder von Keratinfasern, insbesondere des Haars, **dadurch gekennzeichnet, dass** die makroskopisch homogene Zusammensetzung durch Mischen unmittelbar vor oder im Moment der Anwendung auf das Keratinmaterial erlangt wird,

- mindestens einer wässrigen Phase, die durch mindestens ein hydrophiles nicht-stärkehaltiges Geliermittel geliert ist; und
- mindestens einer öligen Phase, die durch mindestens ein lipophiles Geliermittel geliert ist, ausgewählt aus den Organopolysiloxan-Elastomeren, den halbkristallinen Polymeren, den Dextrin-Estern, den Kohlenwasserstoff-Polyamiden, den besonderen Geliermitteln, ausgewählt den Polarkohlenwasserstoffe, den polaren Wachsen,

den unpolaren Kohlenwasserstoffwachsen mit einem Schmelzpunkt kleiner als oder gleich wie 75,0 °C, den Silikonwachsen, dem modifizierten Ton, den Kieselsäuren und deren Gemischen; und

die Zusammensetzung ferner umfassend mindestens einen UV-Filter, ausgewählt aus den organischen wasserlöslichen Filtern, den organischen fettlöslichen Filtern und den organischen unlöslichen Filtern.

**18.** Kosmetisches Verfahren nach Anspruch 16 zur Begrenzung von Hautverdunkelung und/oder zur Verbesserung der Farbe und/oder Gleichförmigkeit des Teints.

**19.** Kosmetisches Verfahren nach Anspruch 16 zur Vorbeugung und/oder Behandlung von Alterungserscheinungen eines Keratinmaterials.

## Claims

**1.** A composition, different from an emulsion, in particular a cosmetic composition, for making up and/or caring for keratin materials, comprising:

- at least one aqueous phase gelled with at least one non-starchy hydrophilic gelling agent,
- at least one oily phase gelled with at least one lipophilic gelling agent chosen from among organopolysiloxane elastomers, semi-crystalline polymers, dextrin esters, hydrocarbon polyamides, particulate gelling agents chosen from among polar waxes, hydrocarbon apolar waxes having a melting point lower than or equal to 75.0 °C, silicone waxes, modified clays, silicas and mixtures thereof;

said phases forming therein a macroscopically homogeneous mixture;
said composition also comprising at least one UV-screening agent chosen from among water-soluble organic screening agents, liposoluble organic screening agents and insoluble organic screening agents.

**2.** The composition according to the preceding claim, wherein said non-starchy hydrophilic gelling agent is chosen from among synthetic polymeric gelling agents, mixed silicates and fumed silicas, non-starchy polymeric gelling agents that are natural or of natural origin, and mixtures thereof, preferably a synthetic polymeric gelling agent, preferably chosen from among crosslinked and/or neutralized 2-acrylamido-2-methylpropanesulfonic acid polymers and copolymers, and modified or unmodified carboxyvinyl polymers, more preferably chosen from among crosslinked polymers of ammonium acrylamido-2-methylpropanesulfonate, copolymers of AMPS® and of hydroxyethyl acrylate, and crosslinked (meth)acrylic acid homopolymers, preferably copolymers of AMPS® and of hydroxyethyl acrylate.

**3.** The composition according to one of the preceding claims, wherein the lipophilic gelling agent is chosen from among semi-crystalline homo- or co-polymers carrying at least one crystallizable side chain, and semi-crystalline homo-or co-polymers carrying at least one crystallizable block in the backbone thereof, hydrocarbon polyamides, hydrophobic silica aerogels, hectorites modified by a $C_{10}$ to $C_{22}$ ammonium salt, in particular hectorites modified by a $C_{10}$ to $C_{22}$ ammonium chloride.

**4.** The composition according to any of the preceding claims, wherein the lipophilic gelling agent is chosen from among hectorites modified by a $C_{10}$ to $C_{22}$ ammonium salt, preferably hectorites modified by a $C_{10}$ to $C_{22}$ ammonium chloride.

**5.** The composition according to any of claims 1 to 2, wherein the lipophilic gelling is chosen from among the following organopolysiloxane elastomers: Dimethicone crosspolymer, Vinyl Dimethicone Crosspolymer, Dimethicone/Vinyl Dimethicone Crosspolymer, Dimethicone Crosspolymer-3, VINYL DIMETHICONE/METHICONE SILSESQUIOXANE CROSSPOLYMER, PHENYL VINYL DIMETHICONE CROSSPOLYMER, and in particular Dimethicone Crosspolymer.

**6.** The composition according to any one of claims 1 to 4, comprising:

- at least one non-starchy hydrophilic gelling agent chosen from among crosslinked and/or neutralized 2-acrylamido-2-methylpropanesulfonic acid (AMPS®) copolymers, and more particularly copolymers of AMPS® and of hydroxyethyl acrylate, and
- at least one non-cellulose-based lipophilic gelling agent chosen from among hectorites modified by a salt, preferably a $C_{10}$ to $C_{22}$ ammonium chloride, and particularly hectorites modified by distearyldimethylammonium chloride,

- at least one UV-screening agent chosen from among water-soluble organic screening agents, liposoluble organic screening agents and insoluble organic screening agents.

**7.** The composition according to one of the preceding claims, wherein the UV-screening agent(s) are chosen from among water-soluble organic UV-screening agents, liposoluble organic UV-screening agents, and mixtures thereof, and more preferentially liposoluble organic UV-screening agents.

**8.** The composition according to any of the preceding claims, wherein the UV-screening agent(s) are totally or partly, and preferably solely, present in the gelled aqueous phase, or are totally or partly, and preferably solely, present in the gelled oily phase.

**9.** The composition according to any of the preceding claims, containing the gelled aqueous and oily phases in an aqueous phase/oily phase weight ratio of from 95:5 to 5:95, preferably from 20:80 to 80:20 and even more preferentially ranging from 30:70 to 70:30.

**10.** The composition according to any of the preceding claims, also comprising at least solid particles such as pigments and/or fillers.

**11.** The composition according to any of the preceding claims, also comprising volatile and/or nonvolatile silicone oils.

**12.** The composition according to any of the preceding claims, also comprising at least one moisturizer, preferably glycerol.

**13.** A process for preparing a composition, different from an emulsion, in particular a cosmetic composition for making up and/or caring for keratin materials, comprising at least one step of mixing:

- an aqueous phase gelled with at least one non-starchy hydrophilic gelling agent; and
- at least one oily phase gelled with at least one lipophilic gelling agent chosen from among organopolysiloxane elastomers, semi-crystalline polymers, dextrin esters, hydrocarbon polyamides, particulate gelling agents chosen from among polar waxes, hydrocarbon apolar waxes having a melting point lower than or equal to 75.0 °C, silicone waxes, modified clays, silicas and mixtures thereof;

said phases forming therein a macroscopically homogeneous mixture;
said composition also comprising at least one UV-screening agent chosen from among water-soluble organic screening agents, liposoluble organic screening agents and insoluble organic screening agents.

**14.** The process according to the preceding claim, comprising a step of mixing at least two gelled phases.

**15.** The process according to either of claims 13 or 14, wherein the mixing is performed at room temperature.

**16.** A non-therapeutic cosmetic process for making up and/or caring for keratin materials, in particular body and/or facial skin, and/or keratin fibers, especially the hair, comprising at least one step consisting of applying to said keratin material a composition such as defined according to any one of Claims 1 to 12.

**17.** The cosmetic process according to claim 16 for making up and/or caring for keratin materials, in particular body and/or facial skin, and/or keratin fibers, especially the hair, **characterized in that** the macroscopically homogeneous composition is obtained by extemporaneous mixing before application or at the time of application to said keratin material, of:

- an aqueous phase gelled with at least one non-starchy hydrophilic gelling agent; and
- at least one oily phase gelled with at least one lipophilic gelling agent chosen from among organopolysiloxane elastomers, semi-crystalline polymers, dextrin esters, hydrocarbon polyamides, particulate gelling agents chosen from among polar waxes, hydrocarbon apolar waxes having a melting point lower than or equal to 75.0 °C, silicone waxes, modified clays, silicas and mixtures thereof; and

said composition also comprising at least one UV-screening agent chosen from among water-soluble organic screening agents, liposoluble organic screening agents and insoluble organic screening agents.

**18.** The cosmetic process according to claim 16 for limiting darkening of the skin and/or improving the color and/or

uniformity of the complexion.

**19.** The cosmetic process according to claim 16 for preventing and/or treating the signs of aging of a keratin material.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9965455 A
- WO 04057589 A
- WO 9962497 A
- JP 2005112834 A
- WO 2008081175 A
- WO 2013093869 A
- US 3301848 A
- US 3589578 A
- US 4031307 A
- EP 0815928 B1
- US 5455340 A
- US 4017460 A
- US 7470725 B
- EP 295886 A
- EP 242219 A
- EP 285886 A
- EP 765656 A
- JP 61194009 A
- US 5538793 A
- EP 0951897 A
- US 5156911 A
- WO 0119333 A
- US 2002005562 A
- US 5221534 A
- FR 2528420 A
- FR 2639347 A
- EP 0669323 A
- US 2463264 A
- US 5624663 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- US 4195999 A
- WO 2004006878 A
- WO 2008090066 A
- WO 2011113718 A
- WO 2009027258 A
- EP 0841341 A
- GB 2303549 A
- EP 893119 A
- WO 9522959 A
- WO 9703642 A
- GB 2286774 A
- EP 743309 A
- WO 9822447 A
- GB 2319523 A
- EP 0790243 A
- WO 9825922 A
- US 6225467 B
- WO 2004085412 A
- WO 06035000 A
- WO 06034982 A
- WO 06034991 A
- WO 06035007 A
- WO 2006034992 A
- WO 2006034985 A
- US 5687521 A
- US 5373037 A
- US 5362881 A
- US 5237071 A
- US 5166355 A
- DE 19726184
- US 5888481 A
- DE 676103
- CH 350763
- US 5501850 A
- US 5961960 A
- US 5518713 A
- EP 0921126 A
- EP 712855 A
- JP 04134042 B
- JP 04134043 B
- EP 0576974 A
- FR 2395023
- JP 01158090 A
- EP 0390683 A
- JP 04134041 B
- FR 2506156
- EP 0693471 A
- FR 2528420
- FR 2529887
- EP 0694521 A
- FR 2638354
- EP 0714880 A
- JP 04290882 B
- JP 87166517 B
- WO 9310753 A
- WO 9311095 A
- WO 9505150 A

- WO 2007071584 A
- WO 2009063392 A
- EP 0518773 A
- WO 2008155059 A
- JP 09188830 A
- JP 10158450 A
- JP 10158541 A
- JP 07258460 A
- JP 05017710 A
- EP 1086683 A
- JP 2003128788 B
- JP 2000191789 B

**Littérature non-brevet citée dans la description**

- **ALMEIDA et al.** *Pharmaceutical Development and Technology*, 2008, vol. 13, 487
- **C. M. HANSEN**. The three dimensionnal solubility parameters. *J. Paint Technol.*, 1967, vol. 39, 105
- Remington: The Science and Practice of Pharmacy. 1995, 282
- **KIRK-OTHMER**. Encyclopedia of Chemical Technology. 1982, vol. 3, 896-900
- **KIRK-OTHMER**. ENCYCLOPEDIA OF CHEMICAL TECHNOLOGY, vol. 15, 439-458
- **E. A. MC GREGOR** ; **C. T. GREENWOOD**. Polymers in Nature. John Wiley & Sons, 1980, 240-328
- **ROBERT L. DAVIDSON**. Handbook of Water soluble gums and resins. Mc Graw Hill Book Company, 1980
- **GUMS**. Polysaccharides and their Derivatives. Academic Press Inc
- KIRK-OTHMER ENCYCLOPEDIA OF CHEMICAL TECHNOLOGY. Wiley Interscience, 1983, vol. 21, 492-507
- Silica silylate. CTFA, 2000
- Silica diméthyl silylate. CTFA, 2000
- **BRINKER CJ.** ; **SCHERER G.W.** Sol-Gel Science. Academic Press, 1990
- *The journal of the American Chemical Society*, February 1938, vol. 60, 309
- **VAN DE HULST, H.C.** Light Scattering by Small Particles. Wiley, 1957
- Intelimer® polymers. *Landec IP22*
- *IP COM JOURNAL*, 23 February 2009 (000179675D)
- *IP COM JOURNAL*, 29 April 2009 (000182396D)
- *IP COM JOURNAL*, 12 November 2009 (000189542D)
- *IP COM Journal*, 04 March 2004 (000011179D)
- Symetrical Triazine Derivatives. IP.COM Journal. IP.COM INC, 20 September 2004
- *J. Am. Chem. Soc.*, 1957, vol. 79, 5706-5708
- *J. Am. Chem. Soc.*, 1960, vol. 82, 609-5 611
- *J. Am. Chem. Soc.*, 1960, vol. 82, 609
- *J. Am. Chem. Soc.*, 1957, vol. 79, 5706-5708
- *J. Chim. Phys.*, 1967, vol. 64, 1602
- **E. MARIANI et al.** *16th IFSCC Congress*, 1990
- *CHEMICAL ABSTRACTS*, 919803-06-8
- Symetrical Triazine Derivatives. IP.COM IPCOM000031257 Journal. INC WEST HENRIETTA, 20 September 2004
- **B. L. DIFFEY**. *J. Soc. Cosmet. Chem.*, 1989, vol. 40, 127-133